# EUROPEAN PATENT APPLICATION

(11) **EP 4 368 640 A1**
(43) Date of publication of application: **15.05.2024**
(21) Application number: 22837042.5
(22) Date of filing: 08.07.2022
(51) Int. Cl.: C07K 16/28, C12N 15/13, C12N 15/62, A61K 39/395, A61P 35/00, A61P 35/02, A61P 37/02

(54) **CD19 ANTIBODY AND APPLICATION THEREOF**

(30) Priority: 09.07.2021 CN 202110780438
(71) Applicant: Hainan Simcere Zaiming Pharmaceutical Co., Ltd., Hainan 570311 (CN)
(72) Inventor: XIN, Lian, Shanghai 201318 (CN); WANG, Qiong, Shanghai 201318 (CN); YANG, Cuiqing, Shanghai 201318 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: KATZAROV S.A.
(86) International application number: PCT/CN2022/104564
(87) International publication number: WO 2023/280297

(57) **Abstract**

A CD19 antibody and an application thereof, an antibody or antigen binding fragment specifically binding to human CD19, a multi-characteristic antigen binding molecule, a chimeric antigen receptor, an immune effector cell, a nucleic acid molecule, a vector, a cell, a preparation method, a pharmaceutical composition, a pharmaceutical use, and a disease treatment method. The present invention has great significance for the development of drugs for treating CD19-related diseases.

## Description

### Technical Field

The present disclosure relates to the field of antibodies, and specifically to a CD19 antibody and an application thereof.

### Background

CD19 antigen belongs to the immunoglobulin superfamily and is a 95 kDa type I transmembrane glycoprotein, with a single transmembrane domain, a cytoplasmic C-terminus and an extracellular N-terminus. The extracellular domain consists of two C2-type Ig-like domains, and the highly conserved cytoplasmic domain consists of 242 amino acids and 9 tyrosine residues near the C-terminus. CD19 is critically involved in establishing intrinsic B cell signaling thresholds through modulating both B cell receptor-dependent and independent signaling. CD19 interacts with other B cell receptors or surface molecules to directly or indirectly recruit and bind to various downstream protein kinases, including Src family (Lyn, Fyn), Ras family, Abl, Btk, adapter molecules (Vav, Grb2) and PI3K protein kinases. CD19, alongside complement receptor CD21, tetraspanin CD81 (TAPA-1) and CD225, functions as the dominant signaling component of a multi-molecular complex on the surface of mature B cells.

CD19 is expressed specifically in normal B cells and follicular dendritic cells, but not in pluripotent blood stem cells. During B cell lymphopoiesis, the surface expression of CD19 first takes place around the time of immunoglobulin gene rearrangement. The surface density of CD19 is highly regulated throughout B cell development and maturation, until the loss of expression during terminal plasma cell differentiation. CD19 is also expressed in most B cell lymphoma, mantle cell lymphoma, acute lymphocytic leukemia, chronic lymphocytic leukemia, hairy cell leukemia and some acute myelogenous leukemia.

B cells provide humoral immune components of the adaptive immune system by secreting antibodies, interacting with T cells and producing chemokines and cytokines, thus playing a key role in host defense. B cell subsets, particularly plasma cells and plasmablasts, have long been considered as the main source in antibody production. B cells are involved in the pathogenesis of many autoimmune diseases, including central nervous system (CNS) diseases, multiple sclerosis (MS) and neuromyelitis optica spectrum disorders (NMOSD). Deficiency of CD19 in experimental animals and humans can lead to impaired humoral responses, resulting in increased susceptibility to infection. However, overexpression of human CD19 can lead to autoimmune diseases in tight skin (TSK/+) mice (model of human systemic sclerosis (SSc)). There are currently some antibodies targeting CD19 in clinical trials on autoimmune diseases. The phase Ib/IIa clinical trial of XmAb5871 on moderate-to-severe rheumatoid arthritis has been completed, and XmAb5871 enters the phase II development for the treatment of IgG4-related diseases and systemic lupus erythematosus (SLE).

In summary, CD19 can be used as a landmark target for the treatment of B cell related leukemia and autoimmune diseases. Antibodies having good affinity to human CD19 are needed.

### Summary of the Invention

The present disclosure provides an antibody or antigen binding fragment specifically binding to human CD19, a multi-characteristic antigen binding molecule, a chimeric antigen receptor, an immune effector cell, a nucleic acid molecule, a vector, a cell, a preparation method, a pharmaceutical composition, a pharmaceutical use, and a disease treatment method, wherein the antibody or antigen binding fragment has good binding ability to human CD 19, improving human CD19-related detection, treatment or other applications related to human CD19 binding.

In a first aspect, the present disclosure discloses an antibody or antigen binding fragment specifically binding to human CD19, the antibody or antigen binding fragment comprises a heavy chain variable region and/or a light chain variable region, the heavy chain variable region comprises HCDR1-3, the light chain variable region comprises LCDR1-3,
the HCDR1 comprises a sequence as shown in any one of SEQ ID NOs: 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421 or 427, or a sequence having at least 70% identity or at most 3 mutations compared thereto;
the HCDR2 comprises a sequence as shown in any one of SEQ ID NOs: 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422 or 428, or a sequence having at least 70% identity or at most 3 mutations compared thereto;
the HCDR3 comprises a sequence as shown in any one of SEQ ID NOs: 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423 or 429, or a sequence having at least 70% identity or at most 3 mutations compared thereto;
the LCDR1 comprises a sequence as shown in any one of SEQ ID NOs: 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424 or 430, or a sequence having at least 70% identity or at most 3 mutations compared thereto;
the LCDR2 comprises a sequence as shown in any one of SEQ ID NOs: 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425 or 431, or a sequence having at least 70% identity or at most 3 mutations compared thereto;
and the LCDR3 comprises a sequence as shown in any one of SEQ ID NOs: 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426 or 432, or a sequence having at least 70% identity or at most 3 mutations compared thereto.

In some particular embodiments, the HCDR1-3 comprise sequences as shown in SEQ ID NOs: 115-117, 121-123, 127-129, 133-135, 139-141, 145-147, 151-153, 157-159, 163-165, 169-171, 175-177, 181-183, 187-189, 193-195, 199-201, 205-207, 211-213, 217-219, 223-225, 229-231, 235-237, 241-243, 247-249, 253-255, 259-261, 265-267, 271-273, 277-279, 283-285, 289-291, 295-297, 301-303, 307-309, 313-315, 319-321, 325-327, 331-333, 337-339, 343-345, 349-351, 355-357, 361-363, 367-369, 373-375, 379-381, 385-387, 391-393, 397-399, 403-405, 409-411, 415-417, 421-423 or 427-429, or sequences having at least 70% identity or at most 3 mutations compared thereto; and/or
the LCDR1-3 comprise sequences as shown in SEQ ID NOs: 118-120, 124-126, 130-132, 136-138, 142-144, 148-150, 154-156, 160-162, 166-168, 172-174, 178-180, 184-186, 190-192, 196-198, 202-204, 208-210, 214-216, 220-222, 226-228, 232-234, 238-240, 244-246, 250-252, 256-258, 262-264, 268-270, 274-276, 280-282, 286-288, 292-294, 298-300, 304-306, 310-312, 316-318, 322-324, 328-330, 334-336, 340-342, 346-348, 352-354, 358-360, 364-366, 370-372, 376-378, 382-384, 388-390, 394-396, 400-402, 406-408, 412-414, 418-420, 424-426 or 430-432, or sequences having at least 70% identity or at most 3 mutations compared thereto.

In some particular embodiments, the antibody or antigen binding fragment comprises the heavy chain variable region and the light chain variable region, and the HCDR1-3 and the LCDR1-3 comprise sequences selected from the group consisting of:
(1) sequences of SEQ ID NOs: 115-120, or sequences having at least 70% identity or at most 3 mutations compared thereto;
(2) sequences of SEQ ID NOs: 121-126, or sequences having at least 70% identity or at most 3 mutations compared thereto;
(3) sequences of SEQ ID NOs: 127-132, or sequences having at least 70% identity or at most 3 mutations compared thereto;
(4) sequences of SEQ ID NOs: 133-138, or sequences having at least 70% identity or at most 3 mutations compared thereto;
(5) sequences of SEQ ID NOs: 139-144, or sequences having at least 70% identity or at most 3 mutations compared thereto;
(6) sequences of SEQ ID NOs: 145-150, or sequences having at least 70% identity or at most 3 mutations compared thereto;
(7) sequences of SEQ ID NOs: 151-156, or sequences having at least 70% identity or at most 3 mutations compared thereto;
(8) sequences of SEQ ID NOs: 157-162, or sequences having at least 70% identity or at most 3 mutations compared thereto;
(9) sequences of SEQ ID NOs: 163-168, or sequences having at least 70% identity or at most 3 mutations compared thereto;
(10) sequences of SEQ ID NOs: 169-174, or sequences having at least 70% identity or at most 3 mutations compared thereto;
(11) sequences of SEQ ID NOs: 175-180, or sequences having at least 70% identity or at most 3 mutations compared thereto;
(12) sequences of SEQ ID NOs: 181-186, or sequences having at least 70% identity or at most 3 mutations compared thereto;
(13) sequences of SEQ ID NOs: 187-192, or sequences having at least 70% identity or at most 3 mutations compared thereto;
(14) sequences of SEQ ID NOs: 193-198, or sequences having at least 70% identity or at most 3 mutations compared thereto;
(15) sequences of SEQ ID NOs: 199-204, or sequences having at least 70% identity or at most 3 mutations compared thereto;
(16) sequences of SEQ ID NOs: 205-210, or sequences having at least 70% identity or at most 3 mutations compared thereto;
(17) sequences of SEQ ID NOs: 211-216, or sequences having at least 70% identity or at most 3 mutations compared thereto;
(18) sequences of SEQ ID NOs: 217-222, or sequences having at least 70% identity or at most 3 mutations compared thereto;
(19) sequences of SEQ ID NOs: 223-228, or sequences having at least 70% identity or at most 3 mutations compared thereto;
(20) sequences of SEQ ID NOs: 229-234, or sequences having at least 70% identity or at most 3 mutations compared thereto;
(21) sequences of SEQ ID NOs: 235-240, or sequences having at least 70% identity or at most 3 mutations compared thereto;
(22) sequences of SEQ ID NOs: 241-246, or sequences having at least 70% identity or at most 3 mutations compared thereto;
(23) sequences of SEQ ID NOs: 247-252, or sequences having at least 70% identity or at most 3 mutations compared thereto;
(24) sequences of SEQ ID NOs: 253-258, or sequences having at least 70% identity or at most 3 mutations compared thereto;
(25) sequences of SEQ ID NOs: 259-264, or sequences having at least 70% identity or at most 3 mutations compared thereto;
(26) sequences of SEQ ID NOs: 265-270, or sequences having at least 70% identity or at most 3 mutations compared thereto;
(27) sequences of SEQ ID NOs: 271-276, or sequences having at least 70% identity or at most 3 mutations compared thereto;
(28) sequences of SEQ ID NOs: 277-282, or sequences having at least 70% identity or at most 3 mutations compared thereto;
(29) sequences of SEQ ID NOs: 283-288, or sequences having at least 70% identity or at most 3 mutations compared thereto;
(30) sequences of SEQ ID NOs: 289-294, or sequences having at least 70% identity or at most 3 mutations compared thereto;
(31) sequences of SEQ ID NOs: 295-300, or sequences having at least 70% identity or at most 3 mutations compared thereto;
(32) sequences of SEQ ID NOs: 301-306, or sequences having at least 70% identity or at most 3 mutations compared thereto;
(33) sequences of SEQ ID NOs: 307-312, or sequences having at least 70% identity or at most 3 mutations compared thereto;
(34) sequences of SEQ ID NOs: 313-318, or sequences having at least 70% identity or at most 3 mutations compared thereto;
(35) sequences of SEQ ID NOs: 319-324, or sequences having at least 70% identity or at most 3 mutations compared thereto;
(36) sequences of SEQ ID NOs: 325-330, or sequences having at least 70% identity or at most 3 mutations compared thereto;
(37) sequences of SEQ ID NOs: 331-336, or sequences having at least 70% identity or at most 3 mutations compared thereto;
(38) sequences of SEQ ID NOs: 337-342, or sequences having at least 70% identity or at most 3 mutations compared thereto;
(39) sequences of SEQ ID NOs: 343-348, or sequences having at least 70% identity or at most 3 mutations compared thereto;
(40) sequences of SEQ ID NOs: 349-354, or sequences having at least 70% identity or at most 3 mutations compared thereto;
(41) sequences of SEQ ID NOs: 355-360, or sequences having at least 70% identity or at most 3 mutations compared thereto;
(42) sequences of SEQ ID NOs: 361-366, or sequences having at least 70% identity or at most 3 mutations compared thereto;
(43) sequences of SEQ ID NOs: 367-372, or sequences having at least 70% identity or at most 3 mutations compared thereto;
(44) sequences of SEQ ID NOs: 373-378, or sequences having at least 70% identity or at most 3 mutations compared thereto;
(45) sequences of SEQ ID NOs: 379-384, or sequences having at least 70% identity or at most 3 mutations compared thereto;
(46) sequences of SEQ ID NOs: 385-390, or sequences having at least 70% identity or at most 3 mutations compared thereto;
(47) sequences of SEQ ID NOs: 391-396, or sequences having at least 70% identity or at most 3 mutations compared thereto;
(48) sequences of SEQ ID NOs: 397-402, or sequences having at least 70% identity or at most 3 mutations compared thereto;
(49) sequences of SEQ ID NOs: 403-408, or sequences having at least 70% identity or at most 3 mutations compared thereto;
(50) sequences of SEQ ID NOs: 409-414, or sequences having at least 70% identity or at most 3 mutations compared thereto;
(51) sequences of SEQ ID NOs: 415-420, or sequences having at least 70% identity or at most 3 mutations compared thereto;
(52) sequences of SEQ ID NOs: 421-426, or sequences having at least 70% identity or at most 3 mutations compared thereto;
(53) sequences of SEQ ID NOs: 427-432, or sequences having at least 70% identity or at most 3 mutations compared thereto.

In some particular embodiments, the heavy chain variable region comprises a sequence as shown in any one of SEQ ID NOs: 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 439-442, or a sequence having at least 70% identity or at most 15 amino acid differences compared thereto;
and the light chain variable region comprises a sequence as shown in any one of SEQ ID NOs: 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 436, 437 or 438, or a sequence having at least 70% identity or at most 15 amino acid differences compared thereto.

In some particular embodiments, the at least 70% identity is preferably at least 75%, 80%, 85%, 90%, 91%, 92%, 93%, 94%, 95%, 96%, 97%, 98%, 99% or 100% identity; the at most 3 mutations are preferably at most 3, 2, 1 or 0 mutations; the at most 15 mutations are preferably at most 15, 14, 13, 12, 11, 10, 9, 8, 7, 6, 5, 4, 3, 2, 1 or 0 mutations; and preferably, the mutation is an insertion, a deletion or a substitution, the substitution is preferably a conservative amino acid substitution, and the mutation is preferably a back mutation or a hotspot mutation.

In some particular embodiments, the antibody or antigen binding fragment further comprises a heavy chain constant region and/or a light chain constant region;
preferably, the heavy chain constant region is selected from IgG, such as IgG1, IgG2, IgG3 or IgG4; the IgG can be selected from human IgG, such as human IgG1 or human IgG4; the light chain constant region is selected from a κ chain or a λ chain, preferably a κ chain;
preferably, the heavy chain constant region can be selected from SEQ ID NO: 433 or 448;
and preferably, the light chain constant region can be selected from SEQ ID NOs: 434-435 or 449.

In some particular embodiments, the antibody or antigen binding fragment is selected from a monoclonal antibody, a polyclonal antibody, a natural antibody, an engineered antibody, a mono-specific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, a fully human antibody, Fab, Fab', Fab'-SH, F(ab')2, Fd, Fv, scFv, a diabody or a single domain antibody.

In some specific embodiments, the antibody or antigen binding fragment further comprise a conjugate; and the conjugate can be selected from a therapeutic agent or a tracer, the therapeutic agent can be selected from a radioisotope, a chemotherapeutic drug or an immunomodulator, and the tracer can be selected from a radiological contrast agent, a paramagnetic ion, a metal, a fluorescent tag, a chemiluminescence tag, an ultrasonic contrast agent and a photosensitizer.

In some particular embodiments, the antibody or antigen binding fragment binds to human CD19 with a KD value of less than 1E-08M, 1E-09M, 1E-10M or 1E-11M; preferably, the antibody or antigen binding fragment also binds to monkey CD19, more preferably, the antibody or antigen binding fragment binds to monkey CD19 with a KD value of less than 1E-8M, 1E-9M, 1E-10M, 1E-11M or 1E-12M.

In a second aspect, the present disclosure further discloses a multi-specific antigen binding molecule, wherein the multi-specific antigen binding molecule comprises at least a first antigen binding module and a second antigen binding module, the first antigen binding module comprises the aforementioned antibody or antigen binding fragment, and the second antigen binding module binds to other targets different from the first antigen binding module or binds to different epitopes of the same target; preferably, the second antigen binding module is an antibody or an antigen binding fragment;
and preferably, the other targets are selected from the group consisting of: (1) a tumor specific antigen (TSA) or a tumor associated antigen (TAA); (2) an immune checkpoint; and (3) a target that recruits and/or activates immune cells.

In a third aspect, the present disclosure further discloses a chimeric antigen receptor (CAR), wherein the chimeric antigen receptor comprises at least an extracellular antigen binding domain, a transmembrane domain and an intracellular signaling domain, and the extracellular antigen binding domain comprises the aforementioned antibody or antigen binding fragment or the aforementioned multi-specific antigen binding molecule.

In a third aspect, the present disclosure further discloses an immune effector cell, wherein the immune effector cell expresses the aforementioned chimeric antigen receptor and/or comprises a nucleic acid molecule encoding the aforementioned chimeric antigen receptor;
preferably, the immune effector cell is selected from a T cell, a natural killer cell (a NK cell), a natural killer T cell (an NKT cell), a monocyte, a macrophage, a dendritic cell or a mast cell, more preferably the T cell is selected from a cytotoxic T cell, a regulatory T cell or a helper T cell;
and preferably, the immune effector cell is an autologous immune effector cell or an allogeneic immune effector cell.

In a fourth aspect, the present disclosure further discloses an isolated nucleic acid molecule, wherein the nucleic acid molecule encodes the aforementioned antibody or antigen binding fragment, the aforementioned multi-specific antigen binding molecule or the aforementioned chimeric antigen receptor.

In a fifth aspect, the present disclosure further discloses a vector, wherein the vector comprises the aforementioned nucleic acid molecule.

In a sixth aspect, the present disclosure further discloses a cell, wherein the cell comprises the aforementioned vector.

In a seventh aspect, the present disclosure further discloses a method for preparing the aforementioned antibody or antigen binding fragment or the aforementioned multi-specific antigen binding molecule, wherein the method comprises: (1) culturing the aforementioned cell, and/or (2) isolating the antibody or antigen binding fragment, or the multi-specific antigen binding molecule expressed by the cell.

In an eighth aspect, the present disclosure further discloses a method for preparing the aforementioned immune effector cell, wherein the method comprises: introducing a nucleic acid molecule encoding the aforementioned chimeric antigen receptor into the immune effector cell, and/or initiating the immune effector cell to express the chimeric antigen receptor.

In a ninth aspect, the present disclosure further discloses a pharmaceutical composition, wherein the pharmaceutical composition comprises the aforementioned antibody or antigen binding molecule, the aforementioned multi-specific antigen binding molecule, the aforementioned immune effector cell, the aforementioned nucleic acid molecule, the aforementioned vector, the aforementioned cell or a product prepared according to the aforementioned method; preferably, the composition further comprises a pharmaceutically acceptable carrier, diluent or auxiliary agent.

In a tenth aspect, the present disclosure further discloses the use of the aforementioned antibody or antigen binding fragment, the aforementioned multi-specific antigen binding molecule, the aforementioned immune effector cell, the aforementioned nucleic acid molecule, the aforementioned vector, the aforementioned cell, the aforementioned pharmaceutical composition, or a product prepared according to the aforementioned method in the preparation of a drug for treating a CD19-related disease; preferably, the CD19-related disease is selected from or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

In an eleventh aspect, the present disclosure further discloses a method for treating a CD19-related disease, wherein the method comprises administering to a subject an effective amount of a drug, and the drug comprises the aforementioned antibody or antigen binding fragment, the aforementioned multi-specific antigen binding molecule, the aforementioned immune effector cell, the aforementioned nucleic acid molecule, the aforementioned vector, the aforementioned cell, the aforementioned pharmaceutical composition, or a product prepared according to the aforementioned method, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

In a twelfth aspect, the present disclosure further discloses the aforementioned antibody or antigen binding fragment, the aforementioned multi-specific antigen binding molecule, the aforementioned immune effector cell, the aforementioned nucleic acid molecule, the aforementioned vector, the aforementioned cell, the aforementioned pharmaceutical composition, or a product prepared according to the aforementioned method, for use in a drug for treating a CD19-related disease, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

The CD 19 antibody and the antigen binding fragment thereof of the present disclosure have good binding ability to human CD19, wherein some of the antibodies can also cross-bind to monkey CD19, thereby providing great significance for the development of CD19-related antibody products or cell therapy products and has good application prospects.

### Definition and Description of Terminology

Unless defined otherwise in the present disclosure, scientific and technical terms related to the present disclosure shall have the meanings understood by one of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of'. Illustratively, "a composition comprising A and B" should be understood as the following technical solution: composition consisting of A and B, and composition containing other components in addition to A and B, fall within the scope of the aforementioned "a composition".

The term "and/or" as used herein includes the meanings of "and", "or" and "all or any other combination of elements linked by the term".

The term "specifically bind" herein means that an antigen binding molecule (e.g., an antibody) typically specifically binds to an antigen and substantially the same antigen with a high affinity, but does not bind to an unrelated antigen with a high affinity. Affinity is generally reflected by the equilibrium dissociation constant (KD), where lower KD indicates higher affinity. Taking an antibody as an example, a high affinity typically means having a KD of about 10⁻⁷ M or less, about 10⁻⁸ M or less, about 1 × 10⁻⁹ M or less, about 1 × 10⁻¹⁰ M or less, about 1 × 10⁻¹¹ M or less, or about 1 × 10⁻¹² M or less. KD is calculated as follows: KD = Kd/Ka, where Kd represents the dissociation rate and Ka represents the binding rate. The equilibrium dissociation constant KD can be measured using well-known methods in the art, such as surface plasmon resonance (e.g., Biacore) or equilibrium dialysis assay. Exemplarily, see the method for obtaining the KD value as shown in example 8 herein.

The term "antigen binding molecule" is used herein in the broadest sense and refers to a molecule specifically binding to an antigen. Exemplarily, the antigen binding molecule includes, but is not limited to, an antibody or an antibody mimetic. The "antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. The "antibody" herein encompasses various forms and various structures as long as they exhibit the desired antigen-binding activity. The "antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, for example, Korndorfer et al., 2003, Proteins: Structure, Function, and Bioinformatics, 53(1): 121-129 (2003); Roque et al., Biotechnol. Prog. 20: 639-654 (2004). Such scaffolds may also include non-antibody-derived scaffolds, for example, scaffold proteins known in the art which can be used for grafting CDRs, including but not limited to tenascin, fibronectin, a peptide aptamer, etc.

The "antibody" herein includes a typical "four-chain antibody", which belongs to an immunoglobulin consisting of two heavy chains (HC) and two light chains (LC); the heavy chain refers to a polypeptide chain that consists of, in a direction from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is an IgE isotype, it optionally also comprises a heavy chain constant region CH4 domain; the light chain is a polypeptide chain consisting of, in a direction from the N-terminus to the C-terminus, a light chain variable region (VL) and a light chain constant region (CL); the heavy chain is linked to the heavy chain and the heavy chain is linked to the light chain via disulfide bonds, forming a Y-shaped structure. Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

The "antibody" herein also includes an antibody that does not comprise light chains, for example, heavy-chain antibodies (HCAbs) produced from Camelus dromedarius, Camelus bactrianus, Lama glama, Lama guanicoe and Vicugna pacos, and Ig new antigen receptors (IgNARs) found in Chondrichthyes such as sharks.

The "antibody" herein may be derived from any animal, including but not limited to humans and non-human animals selected from primates, mammals, rodents and vertebrates, such as Camelidae, Lama glama, Lama guanicoe, Vicugna pacos, sheep, rabbits, mice, rats or Chondrichthyes (such as sharks).

The "antibody" herein includes but is not limited to a monoclonal antibody, a polyclonal antibody, a mono-specific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, or a fully human antibody.

The term "monoclonal antibody" herein refers to an antibody obtained from a population of substantially homogeneous antibodies, that is, individual antibodies comprising the population are identical and/or bind to the same epitope, except for possible variants (such as those containing naturally occurring mutations or produced during the production of a preparation, such variants generally being present in a small amount). In contrast to polyclonal antibody preparations that typically include different antibodies targeting different determinants (epitopes), each monoclonal antibody in monoclonal antibody preparations targets a single determinant on an antigen. The modifier "monoclonal" herein should not be construed as requiring the production of the antibody or antigen binding molecule by any particular method. For example, monoclonal antibodies can be prepared by a variety of techniques, including (but not limited to) hybridoma techniques, recombinant DNA methods, phage library display techniques, methods using transgenic animals containing all or part of human immunoglobulin loci, and other methods known in the art.

The term "natural antibody" herein refers to an antibody produced and paired by the immune system of a multicellular organism. The antibody of the term "engineered antibody" herein refers to a non-natural antibody obtained by techniques such as genetic engineering and antibody engineering. Exemplarily, the "engineered antibody" includes a humanized antibody, a small molecule antibody (such as scFv), a bispecific antibody, *etc.*

The term "monospecific" herein refers to having one or more binding sites, wherein each binding site binds to the same epitope of the same antigen.

The term "multi-specific antibody" herein refers to an antibody having at least two antigen binding sites, each of which binds to a different epitope of the same antigen or to a different epitope of a different antigen. Therefore, terms such as "bispecific", "trispecific" and "tetraspecific" refer to the number of different epitopes to which an antibody/antigen binding molecule can bind.

The term "valence" herein refers to the presence of a defined number of binding sites in an antibody/an antigen binding molecule. Therefore, the terms "monovalent", "bivalent", "tetravalent" and "hexavalent" indicate the presence of one binding site, two binding sites, four binding sites and six binding sites in an antibody/antigen binding molecule, respectively.

The "full-length antibody", "complete antibody" and "intact antibody" are used interchangeably herein and mean that they have a structure substantially similar to that of a natural antibody.

The "antigen binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. The "antigen binding fragment" or "antibody fragment" herein includes but is not limited to Fab, Fab', Fab'-SH, F(ab')₂, Fd, Fv, scFv, a diabody and a single domain antibody.

An intact antibody is digested with papain to produce two identical antigen binding fragments, referred to as "Fab" fragments, each containing a variable domain of heavy chain and a variable domain of light chain, and also a constant domain of the light chain and the first constant domain (CH1) of the heavy chain. Thus, the term "Fab fragment" herein refers to an antibody fragment comprising the VL domain and the constant domain (CL) of a light chain, and the VH domain and the first constant domain (CH1) of a heavy chain. The Fab' fragment differs from the Fab fragment by adding a small number of residues at the carboxyl terminus of the CH1 domain of the heavy chain, including one or more cysteines from the hinge region of the antibody. Fab'-SH is a Fab' fragment in which the cysteine residue of the constant domain carries a free thiol group. Treatment with pepsin produces a F(ab')₂ fragment having two antigen binding sites (two Fab fragments) and a portion of the Fc region.

The term "Fd" herein refers to an antibody consisting of VH and CH1 domains. The term "Fv" herein refers to an antibody fragment consisting of a single arm VL and a VH domain. The Fv fragment is generally considered to be the smallest antibody fragment that can form an intact antigen binding site. It is generally believed that the six CDRs confer antigen binding specificity to an antibody. However, even one variable region (such as a Fd fragment, which contains only three antigen-specific CDRs) is able to recognize and bind to an antigen, although it may have a lower affinity than an intact binding site.

The term "scFv" (single-chain variable fragment) herein refers to a single polypeptide chain comprising a VL domain and a VH domain, wherein the VL and the VH are linked via a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Roseburg and Moore eds., Springer-Verlag, New York, pages 269-315 (1994)). Such scFv molecules can have a general structure: NH2-VL-linker-VH-COOH or NH2-VH-linker-VL-COOH. Suitable linkers in the prior art consist of repeated GGGGS (SEQ ID NO.450) amino acid sequences or variants thereof. For example, linkers having an amino acid sequence (GGGGS)₄ (SEQ ID NO.451) can be used, but variants thereof can also be used (Holliger et al., (1993), Proc. Natl. Acad. Sci. USA 90: 6444-6448). Other linkers that can be used in the present disclosure are described by Alfthan et al., (1995), Protein Eng. 8: 725-731, Choi et al., (2001), Eur. J. Immunol. 31: 94-106, Hu et al., (1996), Cancer Res. 56: 3055-3061, Kipriyanov et al., (1999), J. Mol. Biol. 293: 41-56 and Roovers et al., (2001), Cancer Immunol. In some cases, a disulfide bond can also be present between VH and VL of scFv, forming a disulfide-linked Fv (dsFv).

The VH and VL domains of the term "diabody" herein are expressed on a single polypeptide chain, but a linker used is too short to allow for pairing between two domains of the same chain, thereby forcing the domain to pair with the complementary domain of the other chain and producing two antigen binding sites (see, for example, Holliger P. et al., Proc. Natl. Acad. Sci. USA 90: 6444-6448 (1993), and Poljak R.J. et al., Structure 2: 1121-1123 (1994)).

The terms "single domain antibody (sdAb)", "VHH" and "nanobody" herein have the same meaning and are used interchangeably, and refer to a single domain antibody consisting of only one heavy chain variable region constructed by cloning the heavy chain variable region of an antibody, which is the smallest antigen binding fragment with full functionality. Typically, a single domain antibody consisting of only one heavy chain variable region is constructed by obtaining an antibody that naturally lacks light chains and heavy chain constant region 1 (CH1), and then cloning the heavy chain variable region of the antibody. Single domain antibody can be derived from a Camelidae heavy chain antibody or Chondrichthyes fish IgNAR.

The term "naked antibody" herein refers to an antibody that is not conjugated with a therapeutic agent or a tracer; and the term "conjugated antibody" herein refers to an antibody that is conjugated with a therapeutic agent or a tracer.

The term "chimeric antibody" herein refers to an antibody in which a portion of the light chain or/and heavy chain thereof is derived from one antibody (which may be derived from a particular species or belong to a particular antibody class or subclass), and another portion of the light chain or/and the heavy chain is derived from another antibody (which may be derived from the same or a different species or belong to the same or a different antibody class or subclass), but which in any case retains the activity of binding to an antigen of interest (U.S.P 4,816,567, to Cabilly et al.; Morrison et al., Proc. Natl. Acad. Sci. USA, 81: 6851 6855 (1984)). For example, the term "chimeric antibody" may include an antibody (e.g., a human-murine chimeric antibody) in which the heavy and light chain variable regions of the antibody are derived from a first antibody (e.g., a murine-derived antibody) and the heavy and light chain constant regions of the antibody are derived from a second antibody (e.g., a human antibody).

The term "humanized antibody" herein refers to a genetically engineered non-human antibody whose amino acid sequence has been modified to increase sequence homology with a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody generally retains or partially retains the expected properties of a donor antibody, including but not limited to antigen specificity, affinity, reactivity, ability to improve immune cell activities, ability to enhance immune responses, etc.

The term "fully human antibody" herein refers to an antibody having variable regions in which both the FRs and CDRs are derived from human germline immunoglobulin sequences. Furthermore, if the antibody comprises a constant region, the constant region also is derived from human germline immunoglobulin sequences. The "fully human antibody" herein may include amino acid residues not encoded by human germline immunoglobulin sequences (for example, mutations introduced by random or site-specific mutagenesis in vitro or by somatic mutation in vivo). However, the "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains (VH and VL, respectively) of a natural antibody generally have similar structures, and each domain comprises four conserved framework regions (FRs) and three hypervariable regions (HVRs). See, for example, Kindt et al., Kuby Immunology, 6th ed., W.H. Freeman and Co., p.91 (2007). A single VH or VL domain may be sufficient to confer antigen binding specificity. The terms "complementarity determining region" and "CDR" are used interchangeably herein, and usually refer to the hypervariable region (HVR) of either the heavy chain variable region (VH) or the light chain variable region (VL), which is also referred to as the complementarity determining region because it can form precise complementarity with an antigenic epitope in the spatial structure, wherein the heavy chain variable region CDR can be abbreviated as HCDR and the light chain variable region CDR can be abbreviated as LCDR. The term "framework region" or "FR region" is interchangeable and refers to those amino acid residues other than CDRs in the heavy chain variable region or light chain variable region of an antibody. Usually, a typical antibody variable region consists of 4 FR regions and 3 CDR regions in the following order: FR1-CDR1-FR2-CDR2-FR3-CDR3-FR4.

For a further description of CDR, with reference to Kabat et al., J. Biol. Chem., 252: 6609-6616 (1977); Kabat et al., United States Department of Health and Human Services, "Sequences of proteins of immunological interest " (1991); Chothia et al., J. Mol. Biol. 196: 901-917 (1987); Al-Lazikani B. et al., J. Mol. Biol., 273: 927-948 (1997); MacCallum et al., J. Mol. Biol. 262: 732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309: 657-670 (2001). The "CDR" can be defined by the Kabat numbering system. Tool websites include abYsis website (www.abysis.org/abysis/sequence input/key_ annotation/key_annotation.cgi)

The term "Kabat numbering system" herein generally refers to the immunoglobulin alignment and numbering system proposed by Elvin A. Kabat (see, for example, Kabat et al., Sequences of Proteins of Immunological Interest, 5th Ed., Public Health Service, National Institutes of Health, Bethesda, Md., 1991).

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" comprises at least: a CH1 domain, a hinge region, a CH2 domain, a CH3 domain, or variants or fragments thereof. The "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" can be selected from CH1, Fc or CH3 domains.

The term "light chain constant region" herein refers to the carboxyl terminus portion of an antibody light chain, which is not directly involved in the binding of an antibody to an antigen, and the light chain constant region can be selected from a constant κ domain or a constant λ domain.

The term "Fc" herein refers to the carboxyl terminus portion of an intact antibody obtained by hydrolyzing the antibody with papain, which typically comprises CH3 and CH2 domains of an antibody. The Fc region includes, for example, an Fc region of a natural sequence, a recombinant Fc region and a variant Fc region. Although the boundary of the Fc region of an immunoglobulin heavy chain can be slightly changed, the Fc region of a human IgG heavy chain is usually defined as the region extending from the amino acid residue at position Cys226 or from Pro230 to its carboxyl terminus. The C-terminal lysine of the Fc region (residue 447 according to the Kabat numbering system) can be removed, for example, during the production or purification of an antibody, or by recombinant engineering of a nucleic acid encoding an antibody heavy chain, so the Fc region may or may not include Lys447.

The term "conservative amino acid" herein generally refers to amino acids that belong to the same class or have similar characteristics (such as charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:

Exemplarily, the following six groups are examples of amino acids that are considered to be conservative substitutions for one another:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method: to determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needlema and Wunsch algorithm ((1970) J. Mol. Biol. 48: 444-453) in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70, or 80 and a length weight of 1, 2, 3, 4, 5, or 6. A particularly preferred parameter set (and one parameter set that should be used unless otherwise stated) is the Blossum62 scoring matrix with a gap penalty of 12, a gap extension penalty of 4, and a gap frameshift penalty of 5.

The percent identity between two amino acid sequences or nucleotide sequences can also be determined using the PAM120 weighted remainder table, with a gap length penalty of 12 and a gap penalty of 4, and using the E. Meyers and W. Miller algorithm ((1989) CABIOS, 4: 11-17) that has been incorporated into the ALIGN program (version 2.0).

Additionally or alternatively, the nucleic acid and protein sequences of the present disclosure can further be used as "query sequences" to perform searches against public databases, e.g., to identify other family member sequences or related sequences. For example, the NBLAST and XBLAST programs (version 2.0) of Altschul et al., (1990) J. Mol. Biol. 215: 403-10 can be used to perform such searches. BLAST nucleotide searches can be performed with the NBLAST program, with score = 100 and word length = 12, to obtain the nucleotide sequences homologous to the nucleic acid molecule (SEQ ID NO: 1) of the present disclosure. BLAST protein searches can be performed with the XBLAST program, with score = 50 and word length = 3, to obtain the amino acid sequences homologous to the protein molecule of the present disclosure. To obtain gapped alignment results for comparison purposes, gapped BLAST can be used as described in Altschul et al., (1997) Nucleic Acids Res. 25: 3389-3402. When BLAST and gapped BLAST programs are used, the default parameters of the corresponding programs (e.g., XBLAST and NBLAST) can be used. See www.ncbi.nlm.nih.gov.

The term "chimeric antigen receptor (CAR)" herein refers to an artificial cell surface receptor engineered to express on immune effector cells and specifically bind to an antigen, comprising at least (1) an extracellular antigen binding domain, such as a variable heavy chain or light chain of an antibody, (2) a transmembrane domain anchoring the CAR into an immune effector cell, and (3) an intracellular signaling domain. The CAR can redirect T cells and other immune effector cells to selected targets, such as cancer cells, in a non-MHC-restricted way by using an extracellular antigen-binding domain.

The term "nucleic acid" herein includes any compound and/or substance comprising a polymer of nucleotides. Each nucleotide consists of a base, specifically a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the present disclosure *in vitro* and/or *in vivo,* for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody in vivo (see, for example, Stadler et al., Nature Medicine 2017, Published online June 12, 2017, doi: 10.1038/nm.4356 or EP 2 101 823 B1). The "isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of nucleic acid to which they are operably linked, and such vectors are referred to herein as "expression vectors".

The term "host cell" herein refers to a cell into which a foreign nucleic acid is introduced, including the progenies of such a cell. The host cell includes "transformant" and "transformed cell" which include the primary transformed cell and progeny derived therefrom, regardless of the number of passages. The progeny may not be identical to the parental cell in nucleic acid content, and may contain a mutation. The mutant progeny with the same function or biological activity as those screened or selected in the initially transformed cells are included herein.

As used herein, the term "pharmaceutical composition" refers to a preparation that is present in a form which allows the active ingredients contained therein to be biologically effective and does not contain additional ingredients that would be unacceptably toxic to the subject to which the pharmaceutical composition is administered. The pharmaceutical composition described herein can comprise a plurality of active ingredients. Exemplarily, in addition to the antibody or antigen binding fragment, the multi-specific antigen binding molecule, the immune effector cell, the nucleic acid molecule, the vector and the cell of the first to sixth aspects of the present disclosure or a product prepared according to the method of the seventh to eighth aspects of the present disclosure, the pharmaceutical composition can further comprise other active ingredients, such as a chemotherapeutic drug or an immune checkpoint inhibitor.

The term "treatment" herein refers to surgical or therapeutic treatment, the purpose of which is to prevent and slow down (reduce) an undesired physiological change or pathology, such as cancer progression, in a subject being treated. Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

The term "subject" refers to an organism receiving treatment for a particular disease or disorder as described in the present disclosure. Examples of subjects and patients include mammals, such as humans, primates (e.g., monkeys) or non-primate mammals, receiving treatment for a disease or a disorder.

The term "effective amount" herein refers to an amount of a therapeutic agent effective to prevent or relieve a disease or a disorder or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When the active ingredient is administered to an individual alone, a therapeutically effective dose refers to the ingredient alone. When a certain combination is applied, a therapeutically effective dose refers to the combined dosage of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Brief Description of the Figures

FIG. 1 shows the detection results of human CD19 exon1-3-his protein sample on reducing gel and non-reducing gel by SDS-PAGE, where lane M is protein marker, lane 1 is the sample under non-reducing conditions, and lane 2 is the sample under reducing conditions;
FIG. 2 shows the FACS results of detecting CD19 expression quantity in Raji cells by FMC63 antibody and 9G8 antibody, wherein A is the detection result of FMC63 antibody and B is the detection result of 9G8 antibody;
FIG. 3 shows the FACS screen and detection results of CHO-K1 cells transfected with human CD19;
FIG. 4 shows the FACS result of detecting HEK293T cells transfected with monkey CD19 protein by 9G8 antibody;
FIG. 5 shows the binding activity of CD19 chimeric antibody to human CD19-His protein detected by ELISA;
FIG. 6 shows the binding activity of CD19 chimeric antibody to Raji cells detected by FACS;
FIG. 7 shows the binding activity of CD19 chimeric antibody to CHO-K1-human CD19 detected by FACS;
FIG. 8A shows the binding activity of CD19 chimeric antibody to MOLT-4 cells and Raji cells detected by FACS;
FIG. 8B shows the binding activity of CD19 chimeric antibody to MOLT-4 cells and Raji cells detected by FACS;
FIG. 9A shows the binding activity of CD19 chimeric antibody to CHO-K1 cells and CHO-K1-human CD19 cells detected by FACS;
FIG. 9B shows the binding activity of CD19 chimeric antibody to CHO-K1 cells and CHO-K1-human CD19 cells detected by FACS;
FIG. 10 shows the binding activity of CD19 chimeric antibody to monkey CD19-His protein detected by ELISA;
FIG. 11 shows the binding activity of CD19 chimeric antibody to HEK293T-monkey CD19 cells detected by FACS;
FIG. 12A shows the binding activity of CD19 chimeric antibody to HEK293T cells and HEK293T-monkey CD19 cells detected by FACS;
FIG. 12B shows the binding activity of CD19 chimeric antibody to HEK293T cells and HEK293T-monkey CD19 cells detected by FACS;
FIG. 13 shows a scatter plot of peripheral blood mononuclear cells of cynomolgus monkeys stained with both CD20 antibody and 1 nM chimeric antibody detected by FACS, wherein CD20 is a B cell marker, the ratio as shown in the figure is the ratio of chimeric antibody-labeled positive cells to CD20 positive cells, anti-CD19 control antibody is 9G8, negative control is hIgG 1;
FIG. 14 shows the binding activity of CD19 chimeric antibody to human CD19 exon1-3-his protein detected by ELISA;
FIG. 15 shows the binding activity of humanized antibodies of F3.121.4 to human CD19 protein detected by ELISA;
FIG. 16A shows the binding activity of humanized antibodies of F3.121.4 to CHO-K1-human CD19 cells detected by FACS;
FIG. 16B shows the binding activity of humanized antibodies of F3.121.4 to CHO-K1 cells detected by FACS;
FIG. 16C shows the binding activity of humanized antibodies of F3.121.4 to Raji cells detected by FACS;
FIG. 16D shows the binding activity of humanized antibodies of F3.121.4 to MOLT-4 cells detected by FACS.

### Detailed Description of Embodiments

The present disclosure will be further described below in conjunction with specific examples, and the advantages and characteristics of the present disclosure will become clearer along with the description. If no specific conditions are indicated in the examples, conventional conditions or the conditions suggested by the manufacturer shall be followed. Any reagents or instruments used, unless the manufactures stated, are conventional products that are commercially available.

The examples of the present disclosure are only exemplary and do not limit the scope of the present disclosure in any way. Those skilled in the art should understand that the details and forms of the technical solutions of the present disclosure can be modified or replaced without departing from the spirit and scope of the present disclosure, but these modifications and replacements all fall within the scope of protection of the present disclosure.

### Example 1 Preparation of CD19 antigen and positive antibody

### 1.1 Preparation of human CD19 exonl-3-His tag protein

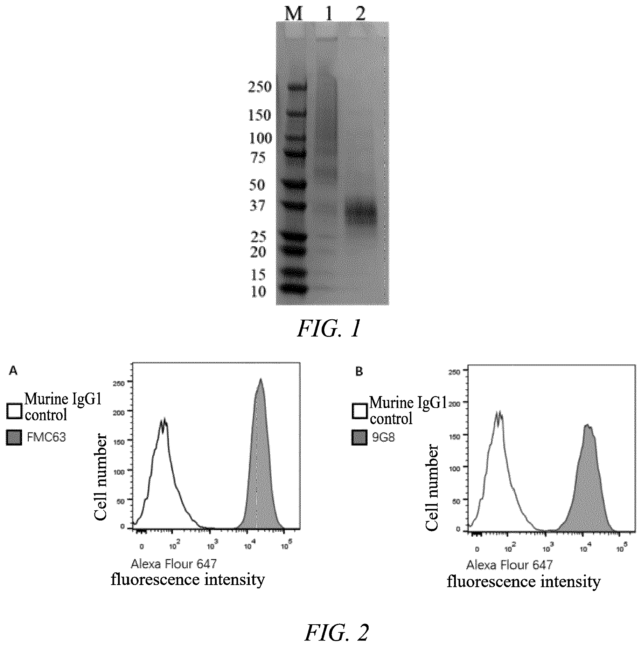

A His-tagged nucleotide sequence that contains a nucleotide sequence encoding the amino acid of human CD19 exon1-3 (from Pro at position 20 to Gin at position 186 of the sequence as shown in NP_001171569.1) was cloned into pTT5 vector by General Biosystems (Anhui) Co., Ltd., and the plasmid was prepared according to the established standard molecular biological method. For the specific method, see Sambrook, J., Fritsch, E. F., and Maniatis, T. (1989). Molecular Cloning: A Laboratory Manual, Second Edition (Plainview, New York: Cold Spring Harbor Laboratory Press). HEK293E cells (purchased from Suzhou Yiyan Biotechnology Co., Ltd.) were transiently transfected (PEI, Polysciences, catalog number: 24765-1) and FreeStyle TM 293 (Thermofisher scientific, catalog number: 12338018) was used for scale-up culture at 37°C. After 6 days, the cell culture fluid was collected, centrifuged to remove cell components, and the culture supernatant containing human CD19 exon1-3 was obtained. The culture supernatant was loaded onto a nickel ion affinity chromatography column HisTrap^{™} Excel (GE Healthcare, catalog number: GE17-3712-06), and the change in the ultraviolet absorbance (A280 nm) was monitored with an ultraviolet (UV) detector. After sample loading, the nickel ion affinity chromatography column was washed with 20 mM PB and 0.5 M NaCl (pH 7.4) until the ultraviolet absorbance returned to the baseline, and then gradient elutions (2%, 4%, 8%, 16%, 50% and 100%) were performed with buffer A: 20 mM PB and 0.5 M NaCl (pH 7.4), and buffer B: 20 mM PB, 0.5 M NaCl and 500 mM imidazole. The His-tagged human CD19 exon1-3 protein eluted from the nickel ion affinity chromatography column was collected and dialyzed with a PBS phosphate buffer (pH 7.4) overnight at 4°C. The dialyzed protein was aseptically filtered through a 0.22 µm filter membrane and then subpackaged for storage at -80°C to obtain a purified human CD19 exon1-3 protein. The bands of interest of samples detected by SDS-PAGE on reducing and non-reducing gels were as shown in FIG. 1.

### 1.2 Preparation of human CD19 control antibody

FMC63 and 9G8 clones are antibodies that recognize human CD19, and the antigen binding epitopes of both are located in the membrane-proximal end. The heavy chain variable region and light chain variable region sequences of FMC63 clone were obtained according to patent WO 2016033570 A1, and the heavy chain variable region and light chain variable region sequences of 9G8 clone were obtained according to patent WO 2018083535 A1. Biointron (Jiangsu) Biological Inc. was entrusted to clone the light chain variable region sequences of FMC63 and 9G8 clones into expression vector pcDNA3.4-B1BLK containing a signal peptide and the light chain constant regions of human and murine antibody IgG1, respectively, and clone the heavy chain variable region sequences into expression vector pcDNA3.4-B1HH1 containing a signal peptide and the heavy chain constant regions of human and murine antibody IgG1, respectively, and thus FMC63-hIgG1, FMC63-mIgG1, 9G8-hIgG1 and 9G8-mIgG1 were obtained. The corresponding amino acid sequence information is as shown in Table 1 below. The plasmids were prepared according to the established standard molecular biological method. For the specific method, see Sambrook, J., Fritsch, E. F., and Maniatis, T. (1989). Molecular Cloning: A Laboratory Manual, Second Edition (Plainview, New York: Cold Spring Harbor Laboratory Press). The expression vector was transiently transfected into HEK293E cells (purchased from Suzhou Yiyan Biotechnology Co., Ltd.) according to the instructions of PEI (purchased from Polysciences, catalog number: 24765-1), the transfected cells were continuously cultured at 37°C for 5 days using FreeStyle TM 293 (Thermofisher scientific, catalog number: 12338018), and the cell components were removed by centrifugation to obtain the culture supernatant containing the antibody. The culture supernatant was loaded onto a protein A chromatography column (the protein A packing AT Protein A Diamond and the chromatography column BXK16/26 were both purchased from Bestchrom (Shanghai) Biosciences Ltd., catalog numbers: AA0273 and B-1620), and the column was washed with a PBS phosphate buffer (pH 7.4), then washed with 20 mM PB and 1 M NaCl (pH 7.2), and finally eluted with a citric acid buffer (pH 3.4). The Fc-tagged antibody eluted from the protein A chromatography column was collected, neutralized with 1/10 volume of 1 M Tris (pH 8.0), and dialyzed with PBS at 4°C overnight, and the dialyzed protein was aseptically filtered through a 0.22 µm filter membrane and then subpackaged for storage at -80°C.

**Table 1 Control antibody sequence information**

| Designation of sequence | Amino acid sequence |
|---|---|
| FMC63-hIgG1 heavy chain | EVKLQESGPGLVAPSQSLSVTCTVSGVSLPDYGVSWIRQPPRKGLEWLGVIWGSETTYYNSALKS RLTIIKDNSKSQVFLKMNSLQTDDTAIYYCAKHYYYGGSYAMDYWGQGTSVTVSSASTKGPSVF PLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYTQKSLSLSPGK (SEQ ID NO: 1) |
| FMC63-mIgG1 heavy chain | EVKLQESGPGLVAPSQSLSVTCTVSGVSLPDYGVSWIRQPPRKGLEWLGVIWGSETTYYNSALKS RLTIIKDNSKSQVFLKMNSLQTDDTAIYYCAKHYYYGGSYAMDYWGQGTSVTVSSAKTTPPSVY PLAPGSAAQTNSMVTLGCLVKGYFPEPVTVTWNSGSLSSGVHTFPAVLQSDLYTLSSSVTVPSSTW PSETVTCNVAHPASSTKVDKKIVPRDCGCKPCICTVPEVSSVFIFPPKPKDVLTITLTPKVTCVVVDI SKDDPEVQFSWFVDDVEVHTAQTQPREEQFNSTFRSVSELPIMHQDWLNGKEFKCRVNSAAFPAP IEKTISKTKGRPKAPQVYTIPPPKEQMAKDKVSLTCMITDFFPEDITVEWQWNGQPAENYKNTQPI MDTDGSYFVYSKLNVQKSNWEAGNTFTCSVLHEGLHNHHTEKSLSHSPGK (SEQ ID NO: 2) |
| FMC63-hIgG1 light chain | DIQMTQTTSSLSASLGDRVTISCRASQDISKYLNWYQQKPDGTVKLLIYHTSRLHSGVPSRFSGSG SGTDYSLTISNLEQEDIATYFCQQGNTLPYTFGGGTKLEITRTVAAPSVFIFPPSDEQLKSGTASVVC LLNNFYPREAKVQWKVDNALQSGNSQESVTEQDSKDSTYSLSSTLTLSKADYEKHKVYACEVTH QGLSSPVTKSFNRGEC (SEQ ID NO: 3) |
| FMC63-mIgG1 light chain | DIQMTQTTSSLSASLGDRVTISCRASQDISKYLNWYQQKPDGTVKLLIYHTSRLHSGVPSRFSGSG SGTDYSLTISNLEQEDIATYFCQQGNTLPYTFGGGTKLEITRADAAPTVSIFPPSSEQLTSGGASVVC FLNNFYPKDINVKWKIDGSERQNGVLNSWTDQDSKDSTYSMSSTLTLTKDEYERHNSYTCEATH KTSTSPIVKSFNRNEC (SEQ ID NO: 4) |
| 9G8-hIgG1 heavy chain | EVQLVQSGAEVKKPGESLKISCKGSGYSFTSYWIGWVRQMPGKGLEWMGIIYPGDSDTRYSPSFQ GQVTISADKSISTAYLQWSSLKASDTAMYYCARGVSGIYNLHGFDIWGQGTLVTVSSASTKGPSV FPLAPSSKSTSGGTAALGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKGFYPSDIAVEWESNGQ |
| | PENNYKTTPPVLDSDGSFFLYSKLTVDKSRWQQGNVFSCSVMHEALHNHYTQKSLSLSPGK (SEQ ID NO: 5) |
| 9G8-mIgG1 heavy chain | EVQLVQSGAEVKKPGESLKISCKGSGYSFTSYWIGWVRQMPGKGLEWMGIIYPGDSDTRYSPSFQ GQVTISADKSISTAYLQWSSLKASDTAMYYCARGVSGIYNLHGFDIWGQGTLVTVSSAKTTPPSV YPLAPGSAAQTNSMVTLGCLVKGYFPEPVTVTWNSGSLSSGVHTFPAVLQSDLYTLSSSVTVPSST WPSETVTCNVAHPASSTKVDKKIVPRDCGCKPCICTVPEVSSVFIFPPKPKDVLTITLTPKVTCVVV DISKDDPEVQFSWFVDDVEVHTAQTQPREEQFNSTFRSVSELPIMHQDWLNGKEFKCRVNSAAFP APIEKTISKTKGRPKAPQVYTIPPPKEQMAKDKVSLTCMITDFFPEDITVEWQWNGQPAENYKNT QPIMDTDGSYFVYSKLNVQKSNWEAGNTFTCSVLHEGLHNHHTEKSLSHSPGK (SEQ ID NO: 6) |
| 9G8-hIgG1 light chain | DIQMTQSPSSLSASVGDRVTITCRASQSISSYLNWYQQKPGKAPKLLIYAASSLQSGVPSRFSGSGS GTDFTLTISSLQPEDFATYYCQQGRFGSPFTFGQGTKVEIKRTVAAPSVFIFPPSDEQLKSGTASVVC LLNNFYPREAKVQWKVDNALQSGNSQESVTEQDSKDSTYSLSSTLTLSKADYEKHKVYACEVTH QGLSSPVTKSFNRGEC (SEQ ID NO: 7) |
| 9G8-mIgG1 light chain | DIQMTQSPSSLSASVGDRVTITCRASQSISSYLNWYQQKPGKAPKLLIYAASSLQSGVPSRFSGSGS GTDFTLTISSLQPEDFATYYCQQGRFGSPFTFGQGTKVEIKRADAAPTVSIFPPSSEQLTSGGASVVC FLNNFYPKDINVKWKIDGSERQNGVLNSWTDQDSKDSTYSMSSTLTLTKDEYERHNSYTCEATH KTSTSPIVKSFNRNEC (SEQ ID NO: 8) |

### Example 2 Identification of endogenous expression cell line, and construction and identification of cell lines overexpressing human CD19 and monkey CD19

### 2.1 Identification of cell line expressing CD19 endogenously

Raji cells (purchased from China Center for Type Culture Collection, Wuhan University) were scale-up cultured in T-25 cell culture flasks to the logarithmic growth phase, the supernatant of the medium was discarded by centrifugation, and the cell pellet was washed 2 times with PBS. FACS (FACS CantoTM, purchased from BD company) was performed using FMC63-mIgG1 and 9G8-mIgG1 antibodies as primary antibody and Alexa Fluor 647-labeled secondary antibody (Jackson, catalog number: 115-605-003). The results are as shown in Table 2 and FIG. 2, indicating that Raji cells can bind to both FMC63-mIgG1 and 9G8-mIgG1.

**Table 2 Results of endogenous cell line Raji cells detected by FACS**

| No. | Designation of antibody | Mean fluorescence intensity of cells | |
|---|---|---|---|
| | | IgG subtype control | CD19 antibody |
| 1 | FMC63-mIgG1 | 72 | 23402 |
| 2 | 9G8-mIgG1 | 72 | 14600 |

### 2.2 Preparation of monoclonal cell line of CHO-K1 stably transfected with human CD19

A nucleotide sequence encoding the full-length amino acid sequence of human CD19 (NCBI: NP_001761.3) was cloned into vector pcDNA3.1 and a plasmid was prepared by General Biosystems (Anhui) Co., Ltd. Plasmid transfection (Lipofectamine^{®} 3000 Transfection Kit, purchased from Invitrogen, catalog number: L3000-015) was performed on CHO-K1 cell line (purchased from Shanghai Institutes for Biological Sciences, Chinese Academy of Sciences), and then the transfected cells were selectively incubated for 2 weeks in DMEM/F12 medium containing 10 µg/ml puromycin and 10% (v/v) fetal bovine serum. FMC63-mIgG1 antibody as primary antibody and Alexa Fluor 647-labeled secondary antibody (Jackson, catalog number: 115-605-003) were used to sort positive monoclonal cells into a 96-well plate on flow cytometer FACS AriaII (BD Biosciences), and the plate was placed in a cell incubator at 37°C and 5% (v/v) CO₂ for cell culture. After about 2 weeks, some wells containing monoclonal cells were selected for expansion. The expanded clones were screened by flow cytometry. The monoclonal cell line with better growth and higher fluorescence intensity was selected for further scale-up culture and then cryopreserved in liquid nitrogen.

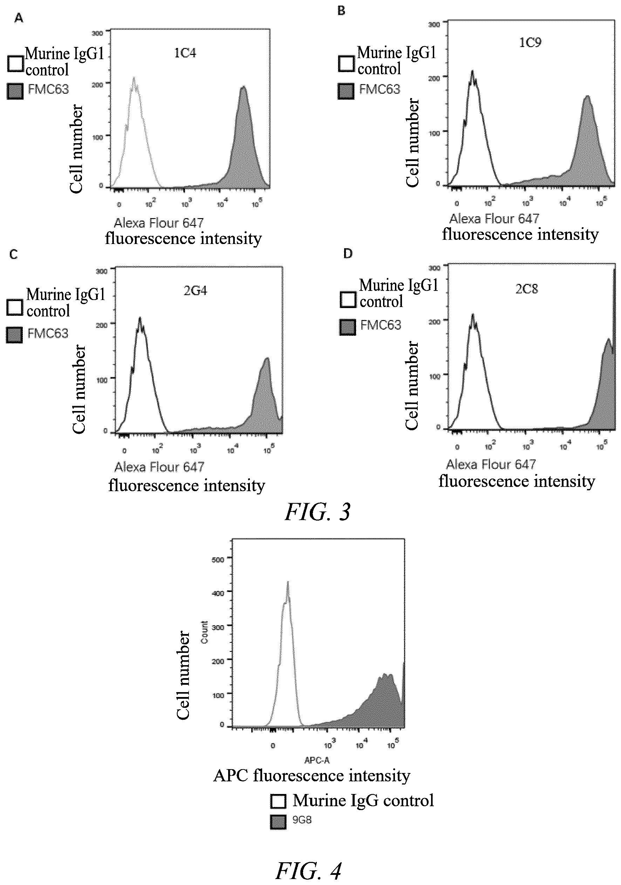

The specific results were as shown in Table 3 and FIG. 3, and the IgG subtype control was murine IgG1 control. The results show that CHO-K1 monoclonal cell lines with high level of human CD19 expression have been prepared: CHO-K1-human CD19-2C8, CHO-K1-human CD19-1C4, CHO-K1-human CD19-2G4 and CHO-K1-human CD19 1C9. The clone CHO-K1-human CD19-2C8 was selected for the subsequent experiment, and unless particularly stated, the CHO-K1-human CD19 cells used in the subsequent examples were all CHO-K1-human CD19-2C8.

**Table 3 Results of CHO-K1 cell line stably transfected with human CD19 protein detected by FACS**

| No. | Clone number of stably transfected cell line | Mean fluorescence intensity of cells | |
|---|---|---|---|
| | | IgG subtype control | CD19 antibody |
| 1 | CHO-K1-human CD19-2C8 | 50 | 131224 |
| 2 | CHO-K1-human CD19-1C4 | 50 | 45781 |
| 3 | CHO-K1-human CD19-2G4 | 50 | 44900 |
| 4 | CHO-K1-human CD19-1C9 | 50 | 31058 |

### 2.3 Preparation of cell line of HEK293T stably transfected with monkey CD19

A nucleotide sequence encoding the full-length amino acid sequence of monkey CD19 (NCBI: XM_005591542.1) was cloned into pcDNA3.1 vector (purchased from Thermofisher scientific) and a plasmid was prepared. After plasmid transfection of a HEK293T cell line with FuGENE^{®} HD (Promega, catalog number: #E2311), the transfected cells were selectively cultured in DMEM medium containing 10 µg/ml puromycin and 10% (v/v) fetal bovine serum for 2 weeks, subcloned in a 96-well culture plate by limited dilution method, and cultured in an incubator at 37°C and 5% (v/v) CO₂. After about 2 weeks, some wells containing polyclonal cells were selected and the cells were expanded into a 6-well plate. The expanded clones were screened by FACS with CD19 antibody 9G8-mIgG1 with monkey cross-activity as primary antibody and Alexa Fluor 647-labeled secondary antibody (Jackson, catalog number: 115-605-003), and the cell line with better growth and higher fluorescence intensity was selected for further scale-up culture and then cryopreserved in liquid nitrogen. The results are as shown in Table 4 and FIG. 4, indicating that the cell line that shows a positive cell peak overexpressing monkey CD19 has been screened and can be used to detect the cross-activity of antibodies.

**Table 4 Results of HEK293T cell line stably transfected with monkey CD19 protein detected by FACS**

| No. | Clone number of stably transfected cell line | Mean fluorescence intensity of cells | |
|---|---|---|---|
| | | IgG subtype control | CD19 antibody |
| 1 | HEK293T-monkey CD19 | 62 | 66503 |

### Example 3 Preparation of anti-human CD19 hybridoma monoclonal antibody

### 3.1 Animal immunization

An anti-human CD19 monoclonal antibody was produced by immunizing mice. BALB/c AnNCrl mice (purchased from Beijing Vital River Laboratory Animal Technology Co., Ltd.) or SJL/JorllcoCrl mice (purchased from Shanghai SLAC Co., Ltd.), female, aged 6-8 weeks, were used in the experiment. Breeding environment: SPF rating. After the mice were purchased, they were raised in a laboratory environment for 1 week, with a 12/12-hour light/dark cycle adjustment, and a temperature of 20°C-25°C; humidity 40%-60%. The acclimatized mice were immunized according to the following scheme. The immunizing antigen was human CD19 (aa20-291)-huFc protein (purchased from ACRO Biosystems, catalog number: CD9-H5251) or CHO-K1 cell line stably transfected with human CD19 protein. For the first immunization of the protein group, the immunogen was emulsified with TiterMax (purchased from Sigma, catalog number: T2684) and injected subcutaneously (SC) and intraperitoneally (IP) at 0.1 ml respectively, that is, each mouse was injected with 50 µg of the immunogen; and for booster immunization, the immunogen was injected subcutaneously at multiple points at 0.1 ml with Imject Alum Adjuvant (purchased from Thermofisher scientific, catalog number: 77161), that is, each mouse was injected with 25 µg of the immunogen. For the first immunization of the cell group, TiterMax and normal saline were emulsified and injected intraperitoneally at 0.1 ml, and after 15 min, 0.1 ml of the cell suspension was injected intraperitoneally, that is, each mouse was injected with 1E7 cells; and for booster immunization, the cell suspension containing 1E7 cells was injected intraperitoneally. The frequency of immunization was once a week, blood was collected on day -3, 19, 34 and 47, and the mouse serum antibody titer was detected by ELISA and FACS methods. The mouse sera in the protein group and the cell group bind to the immunogen to varying degrees, showing antigen-antibody reactions. Tables 5-6 show the detection results of the serum antibody titer of mice numbered 708, 709, 711 and 712 on day 47, and the blank control was 1% (w/v) BSA.

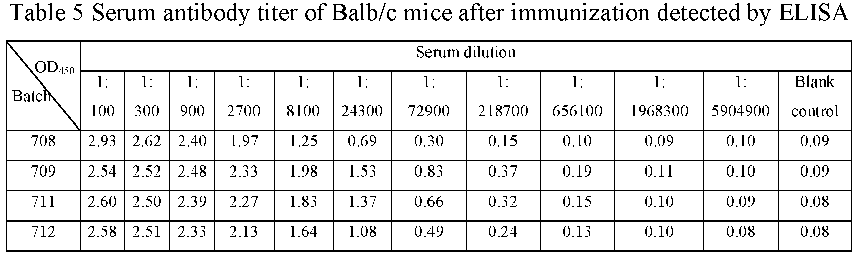

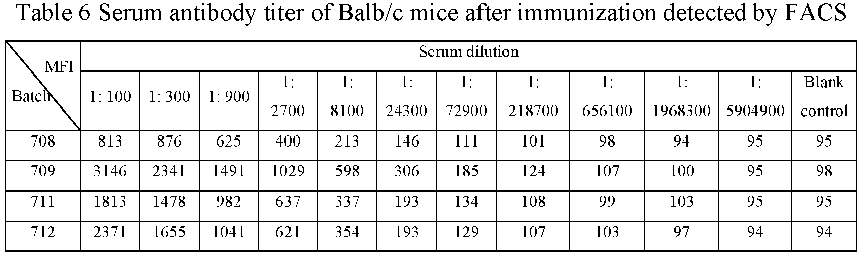

### 3.2 Splenocyte fusion and hybridoma screening

Mice with high antibody titer in serum which tended to plateau were selected for splenocyte fusion. Booster immunization was performed 3 days before splenocyte fusion, an antigen solution prepared with normal saline was injected subcutaneously and intraperitoneally (IP) at 50 µg/mouse.

ACK Lysing Buffer (purchased from Gibco, catalog number: A1049201) was added to lyse the red blood cells doped in the splenocytes to obtain a splenocyte suspension. The cells were centrifugation at a rotating speed of 1500 r/min and washed with DMEM (purchased from Gibco, catalog number: 10566016) basal medium 3 times, and then mixed with mouse myeloma cells SP2/0 (purchased from ATCC, CRL-1581) at a ratio of 2 : 1 in terms of living cells. BTX ECM2001+high-efficiency electrofusion method (see METHODS IN ENZYMOLOGY, VOL. 220) was used for cell fusion. The fused cells were diluted into DMEM medium containing 20% fetal bovine serum (ExCell Bio, catalog number: FSD500) and 1×HAT (purchased from Sigma, catalog number: H0262). The cells were added into a 96-well cell culture plate at 2 × 10⁴/200 µl/well, and cultured at 37°C and 5% CO₂. After 14 days, ELISA was used to screen the supernatant of the fused cells, the positive clones were expanded into a 24-well plate for scale-up culture. The medium was DMEM containing 10% (v/v) fetal bovine serum and 1×HT (purchased from Sigma, catalog number: H0137), and the culture conditions were 37°C and 5% (v/v) CO₂. After culturing for 3 days, the culture liquid of the scale-up culture in the 24-well plate was taken for centrifugation, and the supernatant was collected. The antibody subtype was analyzed for the supernatant, and ELISA and FACS were used to determine the binding activity to human CD19 protein and human CD19 positive cells (see examples 5.1-5.2 for the method for detecting binding activity).

According to the screening results of the 24-well plate, the positive hybridoma cells in both the ELISA and FACS experiments were selected and subcloned using Medium D (purchased from STEMCELL, catalog number: 03810) in a 6-well plate. After about 7 days of subcloning, monoclones were selected and cultured in DMEM medium containing 10% FBS and 1×HT for 2 days at 37°C and 5% CO₂. ELISA was used for preliminary screening, and positive monoclones were selected and expanded into a 24-well plate for further culture. After 2 days, ELISA and FACS were used to determine the antigen binding activity of the positive monoclones, and the optimal clone was selected and scale-up cultured in DMEM medium (purchased from Gibco) containing 10% (v/v) FBS at 37°C and 5% (v/v) CO₂, and cryopreserved in liquid nitrogen, and thus the hybridoma cell of the present disclosure was obtained.

### Example 4 Determination of amino acid sequences of variable regions of light and heavy chains of positive hybridoma clones and preparation of chimeric antibody

### 4.1 Hybridoma antibody sequence information

The hybridoma cells in the logarithmic growth phase were collected, and were fully lysed with Trizol (Invitrogen, Cat No. 15596-018) and stored at -80°C for testing. For the samples, GENEWIZ Suzhou was entrusted to determine the amino acid sequences of the variable regions of the light and heavy chains of the positive hybridoma clones, and 53 clones were obtained. The specific sequences are as shown in Table 7:

**Table 7 Sequences of heavy chain variable region and light chain variable region of positive hybridoma clones**

| Designation of **sequence** | Amino acid sequence |
|---|---|
| F1mab003VH | EVQLQQSGPELVKPGASVKMSCKASGYTFTDYVMHWVRQTPGQGLEWIGYFNPYNDGTNYN EKFKVKATLTSDKSSTTAYMELSSLTSEDSAVYYCARGVYYYGRDFDYWGQGTTLTVSS (SEQ ID NO: 9) |
| F1mab003VL | DVVLTQTPLSLPVSLGDQASISCRSSQSLENSNGNSYLNWYLQKPGQSPQLLIYRVSNRFSGVL DRFSGSGSGTDFTLKISRVEAEDLGVYFCLQITHVPWTFGGGTKLEIK (SEQ ID NO: 10) |
| F2.15.26VH | QVQLKESGPGLVAPSQSLSITCTVSGFSLTDYGVSWIRQSPGKGLEWLGVIWGSGDTYYNSVLE SRLSINKDNSKNQVFLKMNSLQTDDAAMYFCAKHSYYGGSFAMDYWGQGTSVTVSS (SEQ ID NO: 11) |
| F2.15.26VL | DIQMTQTTSSLSASLGDRVTISCRASQDISNYLNWYQQKPDGTVKLLIYYTSRLHSGVPSRFSGS GSGTDYSLTISNLEQEDIATYFCQQGKTLPWTFGGGTKLEIK (SEQ ID NO: 12) |
| F3.3.10VH | DVQLQESGPGLAKLSQSLSLTCSVTGYSITSGYYWNWIRQFPGNKLEWMGHISYDGNNNYNPS LKNRISITRDTSKNQFFLKLNSVTTEDTATYYCARENYSNYYFDYWGQGTTLTVSS (SEQ ID NO: 13) |
| F3.3.10VL | DIVLTQSPASLAVSLGQRATISCRASQSVSKSSYTYIHWYQQKPGQPPKLLIKYASILQSGVPARF SGSGSGTDFTLNFHPVEEEDTATYYCQHSWEIPYTFGGGTKLEIK (SEQ ID NO: 14) |
| F3.5.1VH | QAQLQQSGPELVKPGASVKISCKASGYAFSGSWMNWVQQRPGKGLEWIGRIYPGDGDTYYNG KFKDKAILTADKSSSTAYMQLSSLTSEDSAVYFCAQLRYRYVMDYWGQGTSVTVSS (SEQ ID NO: 15) |
| F3.5.1VL | DIVLTQSPASLAVSLGQRATISCRASESVSIHGSHLMHWYQQKPGQPPRLLIYAASNLESGVPAR FSGSGSETDFTLNIHPVEEEDAATYFCQQGIKDPYTFGGGTKLEIK (SEQ ID NO: 16) |
| F3.13.2VH | DVQLQESGPGLMKPSQSLSLTCSVTGYSITSGYYWNWIRQFPGNKLEWMGYISYDGSNNYNPS LKNRFSITRDTSKNQFFLKLNSVTTEDTATYYCARRKLTGGPYFDYWGQGTTLTVSS (SEQ ID NO: 17) |
| F3.13.2VL | DIVLTQSPASLAVTLGQRATISCRASESVSFHGFHIMHWYQQKPGQPPKLLIYAASHLVSGVPDR FSGSGSETDFTLNIHPVEEEDAAAYFCQQSVEDPWTFGGGTKLEIK (SEQ ID NO: 18) |
| F3.23.10VH | EFQLQQSGPELVKPGASVKISCKASDYSFTDYNMNWVKQSNGASLEWIGIINPNFGSTSYNQKF KGKATLTVDQSSSTAFMQLNSLTSEDSAVYYCARSEYYGSSGFAYWGQGTLVTVSA (SEQ ID NO: 19) |
| F3.23.10VL | DVVVTQTPLSLPVSFGDQVSISCRSSQSLVNSYGNTYLSWYLHKPGQSPQLLIYGISNRFSGVPD RFSGSGSGTDFTLKITTIKPEDLGMYYCLQGTHQPWTFGGGTKLEIK (SEQ ID NO: 20) |
| F3.40.14VH | QAQLQQSGPELVKPGASVKISCKASGYAFSGSWMNWVQQRPGKGLEWIGRIYPGDGDTYYSG KFKDKAILTADTSSSTAYMQLSSLTSEDSAVYFCAQLRSRYVMDYWGQGASVTVSS (SEQ ID NO: 21) |
| F3.40.14VL | DIVLTQSPASLAVSLGQRATISCRASESVSIHGTHLMHWYQQKPGQPPRLLIYAASNLESGVPAR FSGSGSETDFTLNIHPVEEEDAATYFCQQGIDDPYTFGGGTKLEIK (SEQ ID NO: 22) |
| F3.64.4VH | EVQLQQSGPELVKPGASVKISCKASGYTFTDYHINWVKQSHGKSPEWIGDINPNNGGTTYNQK FKDKATLTVDKSSSTAFMELRSLTSEDSAVYYCARFITTVVAWYFDVWGTGTTVTVSS (SEQ ID NO: 23) |
| F3.64.4VL | DIQMTQSPASLSVSVGETVTITCRASENIYSNLAWYQQKQGKSPQLLVYAATNLADGVPSRFSG SGSGTQYSLKINSLQSEDFGNYYCQRFWDTPRTFGGGTKLEIK (SEQ ID NO: 24) |
| F3.74.4VH | QVQLQQSGAELVRPGASVTLSCKASGYTFTDYEMHWVKQTPVHGLEWIGGIAPETGLTTYNQ KFEDKAILTADKSSRTAYMELRSLTSEDSAVYFCNPNNYGLWGTGTTVTVSS (SEQ ID NO: 25) |
| F3.74.4VL | DVVMTQTPLTLSVTIGQPASISCKSSQSLLDSNGKTYLNWLLQRPGQSPKRLIYLVSKLDSGVPD RVTGSGSGTDFTLKISRVEAEDLGVYYCWQGTHFPWTFGGGTKLEIK (SEQ ID NO: 26) |
| F3.77.6VH | EFQLQQSGPELVKPGASVKISCKASGYSFTDYNMNWVKQSNGKSLEWIGTINPNYITSYNQKF KGKATLTVDQSSSTAYMQLNSLTSEDSAVYYCAGDYKGYWGQGTTLTVSS (SEQ ID NO: 27) |
| F3.77.6VL | DVVMTQTPLTLSVTIGQPASISCKSSQNLLDSDGKTYLNWLLQRPGQSPKRLIYLVSKLDSGVP DRFTGSGSGTDFTLKISRVEAEDLGLYYCWQGTHFPWTFGGGTKLEIK (SEQ ID NO: 28) |
| F3.81.2VH | QVQLQQPGAELVMPGASVKLSCKASAYTFTNYWMHWVKQRPGHGLEWIGEIDPSDSYTDYN QNFKAKATLTVDKSSSTAYMQLSSLTSEDSAVYYCARLMGRYFDVWGTGTTVTVSS (SEQ ID NO: 29) |
| F3.81.2VL | DIVMTQSHKFMSTSVGDRVSITCKASQDVSAAVAWYQQKPGQSPKLLINSASYRYTGVPDRFT GSGSGTDFTFTISSVQAEDLAVYYCQQHYSTPLTFGAGTKLELR (SEQ ID NO: 30) |
| F3.83.9VH | DVQLQESGPGLVKPSQSLSLTCSVTGYSITSGYYWNWIRQFPGNKLEWMGYISYDGGNNYNPS LKNRISITRDTSKNQFFLKLNSVTTEDTATYFCAREDYSNWYFDVWGTGTTVTVSS (SEQ ID NO: 31) |
| F3.83.9VL | DIVLTQSPASLAVSLGQRATISCRASQSVSKSSYSYMHWYQQKPGQSPKLLIKYASILKSGVPAR FSGSGSGTDFTLNIHPVEEEDTATYYCQHSWEIPWTFGGGTKLEIK (SEQ ID NO: 32) |
| F3.109.1VH | EVQLQQSGAELVRPGSSVKMSCKTSGYTFTSYGINWVKQRPGQGLEWIGYIYIGNDYTEFNEK FKGKATLTSDTSSSTAYMQLSSLTSEDSAIYFCARFYSNSDPMDYWGHGTSVTVSS (SEQ ID NO: 33) |
| F3.109.1VL | DVVMTQTPLTLSVSIGQPASISCKSSQSLLNSDGKTFLNWLLQRPGQSPKRLIYLVSKLDSGVPD RFTGSGSGTDFTLKISRVEAEDLGVYYCWQGTHFPLTFGAGTKLELK (SEQ ID NO: 34) |
| F3.113.16VH | QVQLQQPGAELVKPGASVKLSCKASGYTFTNYWMQWVKQRPGQGLEWIGEIDPSDSYINYNQ KFKGKATLTADTSSSTAYMQLSSLTYEDSAVYYCARPVTVPWYFDVWGTGTTVTVSS (SEQ ID NO: 35) |
| F3.113.16VL | EIVLTQSPTTMAASPGEKITITCSATSSISSNYLHWYQQKPGFSPKLLIYRTSNLASGVPARFSGSG SGTSYSLTIGTMEAEDVATYYCQQGSAIPRIFTFGSGTKLEIK (SEQ ID NO: 36) |
| F3.114.12VH | DVQLQESGPGLVKPSQSLSLTCSVTGYSITSGYDWNWIRQFPGNKLEWMGYISYDGKNNYNPS LRNRISITRDTSKNQFFLKLNSVTTEDTATYYCARRDLRGYFDVWGTGTTVTVSS (SEQ ID NO: 37) |
| F3.114.12VL | DIVLTQSPATLSVTPGDRVSLSCRASQSISNYLHWYQQKSHESPRLLIKYASLSISGIPSRFSGSGS GTDFTLSINSVETEDFGVYFCQQSNSWPLTFGAGTKLELK (SEQ ID NO: 38) |
| F3.118.14VH | QVQLQQSGAELVRPGASVTLSCKASGYTFPDYEIHWVKQTPVHGLEWIGAIDPETGGIGYNQK FTGKAMLTADKSSSTAYMELRSLTSEDSAVYFCTRNYGSRWGQGSTLTVSS (SEQ ID NO: 39) |
| F3.118.14VL | DVVMTQTPLTLSVTIGQSASISCKSSQSLLESDGKTYLNWLLQRPGQSPKRLIYLVSKLDSGVPD RFTGSASGTDFTLKISRVEAEDLGVYYCWQGTHFPWTFGGGTKLEIK (SEQ ID NO: 40) |
| F3.121.4VH | QVQLQQPGAELVVPGTSVKLSCKASGYTFTNYWMHWVKQRPGQGLEWIGEIDPSDNYANYN QEFQGKATLTVDKSSSTAYMQLSSLTSDDSAVYYCARHDGYFGALDYWGQGTSVTVSS (SEQ ID NO: 41) |
| F3.121.4VL | EIVLTQSPTTMAASPGEKITITCSASSSISSNYLHWYQQKPGFSPKFLIYRTSNLASGVPARFSGSG SGTSYSLTIGTMEAEDVATYYCQQASSIPRMFTFGSGTKLEIK (SEQ ID NO: 42) |
| F3.124.7VH | EVQLQQSGPVLVKPGASVKMSCKASGYTFTDYYMNWVKQRHGKSLEWIGISNPYNGGTSYN QKFKDKATLTVDKSSSTAYMELNSLTSEDSAVYYCAREGGTAQPTSGMDYWGQGTSVTVSS (SEQ ID NO: 43) |
| F3.124.7VL | NIMMTQSPSSLAVSAGEKVTMSCKSSQSVLYSSNQKNYLAWYQQKPGQSPKLLIYWASTRESG VPDRFTGSGSGTDFTLTISSVQPEDPAVYYCHQYRSSYTFGGGTKLEIK (SEQ ID NO: 44) |
| F3.126.15VH | QVQLQQPGAELVMPGASVKLSCKASGYTFTSYWMHWVKQRPGQGLEWIGEIDPSDSYTTYN QNFKGKATLTVDISTSTAYIQLSNLTSEDSAVYYCARLTGNYIHYWGQGTTLTVSS (SEQ ID NO: 45) |
| F3.126.15VL | DIVMTQSHKFMSTSVGDRVSITCKASQDVSPAVAWYQQRPGQCPKLLIFSSSYRSTGVPDRFTG SGSGTDFTFTISSVQAEDLAVYYCQQHYSLPLTFGAGTKLELK (SEQ ID NO: 46) |
| F3.133.14VH | QVQLQQPGAELVMPGASVKLSCKASGYTFTNYWMHWVKQRPGQGLEWIGEIDPSDYYANYN QKFKGKATLTVDKSSSTAYMQLSSLTSEDSAVYYCARSHYYANSYNYWGQGTTLTVSS (SEQ ID NO: 47) |
| F3.133.14VL | EIELTQSPTTMAASPGEKITITCSASSSISSDYLHWYQQKPGFSPKLLIYRTSNLASGLPARFSGSG SGTSYSLTIGTMEAEDVATYYCQQGSSVPRMLTFGAGTKLELK (SEQ ID NO: 48) |
| F3.148.10VH | EVQLQQSGAELVRPGSSVTMSCKTSGYTFTNYGINWVKQRPGQGLEWIGYIFIGNDYTEYNEK FKGKATLTSDTSSSTAYMQLSSLTSEDSAIYFCTRFYSNYYGMDPWGQGTSVTVSS (SEQ ID NO: 49) |
| F3.148.10VL | DVVMTQTPLTLSVTIGQPASISCKSSQSLLHSDGKTYLNWLLQRPGQSPKRLIYLVSKLDSGVPD RFTGSGSGTDFTLKISRVEAEVLGVYYCWQGTHFPPTFGAGTKLEMK (SEQ ID NO: 50) |
| F4.4.6VH | QVQLQQSGAELARPGASVKLSCKASGYTFTSYWMQWVKQRPGQGLEWIGAIYPGDGDSRYT QKFKGKATLTADKSSSTAYMQLSSLASEDSAVYYCARSTTTVLGNNFEYWGQGTTLTVSS (SEQ ID NO: 51) |
| F4.4.6VL | QIVLTQSPAIMSALPGEKVTMTCSASSSVSSMHWYQQKSGTSPKRWIYDTSKLASGVPARFSGS GSGTSYSLTISSMEAEDAATYYCQQWSSNPWTFGGGTKLEIK (SEQ ID NO: 52) |
| F4.5.9VH | QVQLQQSGPELVKPGASVKISCKASGYAFSSSWMNWLKQRPGQGLEWIGRIYPGDGDTNYNG KFKGKATLTADESSSTAYMQLSSLTSVDSAVYFCARGITTIITIAWFASWGQGTLVTVSA (SEQ ID NO: 53) |
| F4.15.9VL | QIVLSQSPTILSASPGEKVTMTCRASSSVSYMHWYQQKPGSSPKPWIYATSNLASGVPPRFSGSG SGTSYSLTISRVGAEDAATYYCQQWSSDPWTFGGGTKLEIK (SEQ ID NO: 54) |
| F4.18.13VH | QVQLQQSGPELVKPGASVKISCKASGYAFSSFWMNWVKQRPGEGLEWIGRIYPGDGDTNYNG KFKGKATLTADDSSSTAYMQLSSLTSVDSAVYFCAREVIAAVVTTDFDYWGQGTTLTVSS (SEQ ID NO: 55) |
| F4.18.13VL | DIQMTQTTSSLSASLGDRVTISCRTSQDISNYLNWYLQKPDGTVKLLIYYTSGLHSGVPSRFSGS GSGTDYSLTISNLEPEDIATYFCQQGKTLPYTFGGGTKLEIK (SEQ ID NO: 56) |
| F4.21.2VH | QVQLKQSGPGLVQPSQSLSITCTVSGFSLSTYGVHWVRQSPGRGLEWLGVIWIGGTTDYNAAFI SRLTISKDNSRGQVFFKMDSLQANDTAIYYCARKGYYKYDGGYYYAMDYWGQGTSVTVSS (SEQ ID NO: 57) |
| F4.21.2VL | DIQMTQSSSYLSVSLGGRVTITCKASDHINNWLAWYQQKPGNAPWLLISGATSLETGVPSRFSG SGSGKDYTLSISSLQTEDVATYYCQQYWSTPLTFGAGTKLELK (SEQ ID NO: 58) |
| F4.22.6VH | QVQLQQSGPELVKPGASVKISCKASGYAFSSFWMNWVKQRPGQGLEWIGRIYPGDGDTNYNG KFKGKATLTADKSSTTAYMHLSSLTSVDSAVYFCTRSVITAVVDWYFDVWGAGTTVTVSS (SEQ ID NO: 59) |
| F4.22.6VL | QIVLSQSPAILSASPGEKVTLTCRASSSVNFMHWYQQKPGSSPKPWIYATSNLASGAPARFSGSG SGTSYSLTISRVEAEDAAAYYCQQWSSYPIFTFGSGTKLEIK (SEQ ID NO: 60) |
| F4.33.12VH | QVQLQQSGAELVRPGSSVKISCKGSGYAFSSYWMNWVKQRPGQGLEWIGQIYPGDGDTNYNG KFKGKATLTADKSSSTAYMQLSSLTSEDSAVYFCARAGFYYGSDSSMDYWGQGTSVTVSS (SEQ ID NO: 61) |
| F4.33.12VL | DIVLTQSPASLAVSLGQRATISCRASESVDNFGISFMNWFQQKPGQPPKLLIYAASNQGSGVPAR FSGSGSGTDFSLNIHPMEADDTAMYFCQQSKEVPYTFGGGTKLEIK (SEQ ID NO: 62) |
| F4.35.5VH | EIQLQQSGPELVKPGASVKVSCKASGYAFTSYNMYWVKQSHGKSLEWIGYIDPYNGDTRYNQ KFKGKATLTVDKSSSTAYMHLNSLTSEDSAVYYCTRSGTGRDYWGQGTTLTVSS (SEQ ID NO: 63) |
| F4.35.SVL | QIVLTQSPAIMSASPGEKVTMTCSASSSISYMFWFQQKPGSSPRLLIYDTSSLASGVPVRFSGSGS GTSYTLTISRMEAEDAATYYCQQWSNYPYTFGGGTKLEMK (SEQ ID NO: 64) |
| F4.37.6VH | QVQLQQSGAELARPGASVKLSCKASGYTFTSFWMQWLKQRPGQGLEWIGTIYPGDGDTRYTQ KFKGKATLTADKSSNTAYMQLSSLASEDSAVYYCARGALSNYGGFAYWGQGTLVTVSA (SEQ ID NO: 65) |
| F4.37.6VL | NIVLTQSPASLAVSLGQRATISCRASESVDSYGHSFMHWYQQKPGQPPKLLIYLASNLESGVPAR FSGSGSRTDFTLTIDPVEADDAATYYCQQNNEDPWTFGGGTKLEIK (SEQ ID NO: 66) |
| F4.41.1VH | QVQLQQSGAELVRPGTSVKVSCKASGYAFTNYLIEWVKQRPGQGLEWIGVINPGSGDTLYNEK FKGKATLTADKSSTTAYMQLSGLTSDVSAIYFCARSPPITTIVADYFDYWGQGTTLTVSS (SEQ ID NO: 67) |
| F4.41.1VL | DIQMTQTTSSLSASLGDRVTISCRASQDISNYLNWYQQKPDGTVKLLIYYTSRLHSGVPSRFSGS GSGADYSLTISNLEQEDIATYFCQQGKTLPYTFGGGTKLEIK (SEQ ID NO: 68) |
| F4.48.12VH | QMQLKESGPGLVAPSQSLSITCTVSGFSLTDYGVSWIRQPPGKGLEWLGVIWGSGSTFYNSALT SRLSISKDNSKSQVFLKMNSLQTADTAMYFCAKHHYYGGSFAMDYWGQGTSVTVSS (SEQ ID NO: 69) |
| F4.48.12VL | DIQMTQASSSLSASLGDRVTISCKTSQDISNYLNWYQQKPDGTFKLLIHYTSRLHSGVPSRFSGS GSGSDYSLTISNLEREDIATYFCQQGNSLPYTFGGGTKLEMK (SEQ ID NO: 70) |
| F4.49.11VH | DVQLRASGPGLVKPSQSLSLTCSVTGYSITSGYYWNWIRQFPGNKLEWMGCISYDGTNNYNPS LKNRISFTRDTSKNQFFLNLNSVTTEDSATYYCARRVYYRYDGVVDYWGQGTSVTVSS (SEQ ID NO: 71) |
| F4.49.11VL | ENVLTQSPAIMSASPGEKVTMTCSASSSVSYIHWYQQKSSTAPKLWIYDTSKLASGVPGRFSGS RSRNSYSLTISSMEAEDVATYYCFQGSGYPLTFGSGTKLEIK (SEQ ID NO: 72) |
| F4.58.6VH | QVQLQQPGAELVKPGASVKLSCKASGYTFTSYWMHWVKQRPGQGLEWIGEIDPSDSYSNYNQ KFKGKATLTVDKSSSTAYMQLSSLTSEDSAVYYCARSNYGSSYGYFDVWGAGTTVTVSS (SEQ ID NO: 73) |
| F4.58.6VL | QIVLTQSPAIMSASPGEKVTMTCSVSSSLSYMHWYQQKPGTSPKRWIYDTSKLASGVPARFSGS GSGTSYSLTISSMEAEDAATYYCHQRSSYPTFGGGTKLEIK (SEQ ID NO: 74) |
| F4.78.1VH | QVQIQQSGAELARPGASVKLSCKASGYTFTNYWMQWIKQRPGQGLEWIGAIYPGDGDTRYTQ KFKGKATLTADKSSSTAYMQLSSLASEDSAVYYCAREEVWSPYGMDYWGQGTSVTVSS (SEQ ID NO: 75) |
| F4.78.IVL | QIVLTQSPAIMSASPGEKVTITCSASSSVSYMGWFQQKPGTSPKLWIYSTSNLASGVPARFSGSG SGTSYSLTISRMEAEDAATYYCQQRSIYPLTFGAGTKLELK (SEQ ID NO: 76) |
| F4.82.11VH | QVTLKESGPGILQASQTLSLTCSFSGFSLSTFGIGVGWVRQPSGKGLEWLAHIWWNDNKFFNTT LKNRLTLSKDPSNNQVFLKIASVDTADTATYYCARSRSALITTGLYAMDYWGQGTSVTVSS (SEQ ID NO: 77) |
| F4.82.11VL | DIQMTQTPSSLSASLGDRVTISCRSSQDISNYVNWYQQKPDGTVKVLINYTSRLHSGVPSRFSGS GSGTDYSLTISNLEQEDIASYFCQQGFTLPYTFGGGTKLEIK (SEQ ID NO: 78) |
| F4.96.6VH | EIQLQQSGPELVKPGASVKVSCKASGYAFSAYNIYWVKQSHGKSLEWIGYIDPYNSGTTYNQK FRGKATLTVDKSSNTAYMHLSSLTSEDSAVYYCTRDGDAMDYWGQGTSVTVSS (SEQ ID NO: 79) |
| F4.96.6VL | EILLTQSPAIIAASPGEKVTITCSASSGVIYMNWYQQKPGSSPKIWIYDVSTLASGVPARFSGSGS GTSFSFTINSMEAEDVATYYCQQRSSYPYTFGGGTKLEIK (SEQ ID NO: 80) |
| F4.107.12VH | EVQLQQSGPELVKPGASVKMSCKASGYTFTSYVLHWVRQKPGQGLEWIGYFNPYNDGTEYNE KFKGKAILTSDKSSSTAYMELNSLTSEDSAVYYCVGGTYYYGSSYPFAYWGQGTLVTVST (SEQ ID NO: 81) |
| F4.107.12VL | DVVMTQISLSLPVSLGDQASISCRSSQSLIHSNGNTYLQWYLQKPGQSPKLLIYKVSNRLSGVP DRFSGSGSGTDFTLKISRVEAEDLGVYFCSQSTHVPYTFGGGTKLEIK (SEQ ID NO: 82) |
| F4.125.12VH | EVKLVESGGGLVQPGGSLKLSCAASGFTFSTYTMSWVRQTPEKRLEWVAFISSGGGHINYPDTV KGRFTISRDNAKNTLYLQMSSLKSEDTAMYYCARHSYWFFDVWGAGTTVTVSS (SEQ ID NO: 83) |
| F4.125.12VL | DVQITQSPSYLAASPGETITINCRASKNIDTYLAWYQEKPGKTNKLLIYSGSTLQSGIPSRFRGGG SGTDFTLTICSLEPEDFAMYYCQQHNEYPLTFGAGTKLELK (SEQ ID NO: 84) |
| F4.145.9VH | EVQLVESGGGLVKPGGSLKLSCAAAGFAFSSYDMSWVRQIPEKRLEWVAYISAGGGNTYFLDT VKGRFTISRDHAKNTLSLQMSSLKSEDTAMYFCVRHGDYYRYFYAMDYWGQGSSVTVSS (SEQ ID NO: 85) |
| F4.145.9VL | DIVMTQSHKFMSTSVGDRVSITCKASQDVGTAVAWSQQKPGQSPKLLIYWASTRHTGVPDRFT GSGSGTDFTLTISNVQSEDLADYFCQQFVTYPYTFGGGTKLEIK (SEQ ID NO: 86) |
| F4.152.7VH | QVQLQQPGAELVKPGASVKLSCKASGYTFTSYWMHWVKQRPGQGLEWIGEIDPSDSYTNYNQ KFKGKATLTADKSSSTAYMQLSGLTSEDSAVYHCARGGDYDGYYVMDYWGQGTSVTVSS (SEQ ID NO: 87) |
| F4.152.7VL | EIVLTQSPTTMAASPGEKITITCSASSSISSNYLHWYQQKPGFSPKLLIYRTSNLASGVPARFSGSG SGTSYSLTIGTMEADDVATYYCQQGSYIPRIFTFGSGTKLEIK (SEQ ID NO: 88) |
| F4.180.1VH | EVQLQQSAPELVKPGASVKMSCKTSGYTFTENIIHWVKQTHGKSLEWIGGINPDNGGTSYHQK FKGRTTLTIDKSSSTAYMELRSLTSDDSAVFFCARQAPLYWYFDIWGAGTTVTVSS (SEQ ID NO: 89) |
| F4.180.1VL | DIVLTQSPASLAVSLGQRATISCRASESVDSYGNSLMHWFQQKPGQPPRLLIYRASNLESGIPARF SGSGSRTDFTLTITPVEADDVATYYCQQSNEDPWTFGGGTKLEIK (SEQ ID NO: 90) |
| F4.191.1VH | QVQLQQPGAELVRPGASVKLSCKASGYTFTSYWINWVKQRPGQGLEWIGNIHPSDSYTNYNQ KFKDKATLTVDKSSSTAYMQFSSPTSEDSAVYYCTREDYYYGSNYDAMDYWGQGTSVTVSS (SEQ ID NO: 91) |
| F4.191.1VL | DIVMTQSHKFMSTSVGDRVNITCKASQDVGSSVAWYQQKPGQSPKLLIYWASTRHAGVPDRFT GSGSGADFTLTISNVQSEDLADYFCQQYSTYPLTFGAGTKLELK (SEQ ID NO: 92) |
| F4.272.13VH | QVQLKESGPGLVAPSQSLSITCTVSGFSLTDYGVSWIRQPPGKGLEWLGVIWGSETTFYNSALKS RLSISKDNSKSQLFLQMNSLQADDTAIYYCAKHHYYGGSFSMDYWGQGTSVTVSS (SEQ ID NO: 93) |
| F4.272.13VL | DIQMTQTTSSLSASLGDRVTISCRASQDINNYLNWYQQKPDGTVKFLIYYTSRLHSGVPSRFSG SGSGTDYSLTISDLEQEDIATYFCQQGNTLPYTFGGGTKLEIK (SEQ ID NO: 94) |
| F4.276.12VH | EIQLQQSGPELVKPGASVKVSCKASGYAFSAYNMYWVKQSHGKSLEWIGYIDPYNGGTNYNQ KFKGKATLTVDKSSSTAYMHLNSLTSEDSAVYYCARDGYEVTYWGQGTLVTVSA (SEQ ID NO: 95) |
| F4.276.12VL | QIVLTQSPALMSASLGEEITLTCSASSSVTYMHWSQQKSGTSPRLLIYSTSNLASGVPSRFSGSGS GTFYSLTISSVEAEDAADYYCHQWSSYPWTFGGGTKLEIK (SEQ ID NO: 96) |
| F5.2.9VH | QVTLKESGPGILQPSQTLSLTCSFSGFSLSTFGLGVGWVRQPSGKGLEWLAHIWWDDDKYYNP ALKSRVTVSKETSKNQVFFKIANVDTADSATYYCARLSRGLRRDVVYAMDHWGQGTSVTVSS (SEQ ID NO: 97) |
| F5.2.9VL | DIQMTQTTSSLSASLGDRVTISCRASQDISNYLNWYQQKPDGSVKLLISYVSRLQSGVPSRFRGS GSGTDYSLTISNLDQEDIATYFCQQGIAFPYTFGGGTKLDIK (SEQ ID NO: 98) |
| F5.3.6VH | DVQLQESGPGLVKPSQSQSLTCSVTGYSITSGYYWNWIRQFPGNKLEWMGYISYDGSNNYNPS LKNRISITRDTSKNQFFLKLNSVTTEDTVTYYCARAPLTGDRYWYFDVWGTGTTVTVSS (SEQ ID NO: 99) |
| F5.3.6VL | DIVLTQSPASLAVSLGQRATISCRASQSVSTSSYSYMHWYQQKPGQTPKLLIKYASNLKSGVPAR FSGSGSGTDFTLNIHPVEEEDTATYYCQHSWEIPPTFGGGTKLEIK (SEQ ID NO: 100) |
| F5.7.1VH | QVTLKESGPGILQPSQTLSLTCSFSGFSLTTFGMGVGWIRQPSGKGLEWLTHIWWDDDKYYNPA LKSRLTISKDTSKNQVFLRIANVDTADTATYYCARVITTGVPYFDYWGQGTTLTVSS (SEQ ID NO: 101) |
| F5.7.1 VL | ENVLTQSPAIMSASPGEKVTMTCRASSSVSSSYLYWYQQKSGASPKLWISSTSNLASGVPARFS GSGSGTSYSLTINSVEAEDAATYYCQQYSGYPWTFGGGTKLEIK (SEQ ID NO: 102) |
| F5.8.6VH | QVQLHQPGAELVMPGASVTLSCKASGYTFTSYWMHWVKQRPGQGLEWIGEIDPSDSYTTYDQ KFKGKATLTVDKFSSTAYMQFSSLTSEDSAVYYCARLTGNHLDYWGQGTTLTVSS (SEQ ID NO: 103) |
| F5.8.6VL | DIVMTQSHKFMSSSVGDRVSITCKASQDVSPAVAWYQQKPGQSPKLLIYSTSYRNTGVPDRFTG SGSGTDFTFTISSVQAEDLAVYYCQQHYSIPLTFGAGTKLELK (SEQ ID NO: 104) |
| F5.10.6VH | DVQLQESGPGLVKPSQSLSLTCSVTDYSITSGYYWTWIRQFPGNKLEWMGYISYDGSTNYNPSL KNRFSITRDTSKNQFFLKLNSVIIEDTATYYCARDRMSTTWYFDVWGTGTTVTVSS (SEQ ID NO: 105) |
| F5.10.6VL | DIVLTQSPASLAVSLGQRATISCRASESVDNYGISFMHWYQQKPGQPPKLLIYRASKTESGIPARF SGSGSRTDFTLTINPVETDDVATYYCQQSNKYPRTFGGGTKVEIK (SEQ ID NO: 106) |
| F5.11.2VH | EVQLVESGGDLVKPGGSLKLSCAASGFTFSSYGMSWVRQTPDKRLEWVATISHGGSYTHYPDS VKGRFTISRDNAKNTLYLQMNSLKSEDTAMYYCVRLPITTVEAPYFFDYWGQGTTLTVSS (SEQ ID NO: 107) |
| F5.11.2VL | DIQMTQTTSSLSASLGDRVTISCRASQDIRNYLNWYQQKPDGTVKLLIYYTSILHSGVPSRFSGS GSGTDYSLTINNLEQEDVATYFCQQGNTLYTFGGGTEVELR (SEQ ID NO: 108) |
| F6.2.9VH | QVQLQQSGAELVKPGASVKISCKASGYAFSSYWMNWVKQRPGKGLEWIGQIYPGDGDTNYN GKFKGKATLTADKSSSTGYMQLSSLTSEDSAVYFCARSRITSVVDWYFDVWGTGTTVTVSS (SEQ ID NO: 109) |
| F6.2.9VL | QIVLSQSPAILSASPGEKVTMTCRASSSVSYMHWYQQKPGSSPKPWIYATSNLASGVPARFSGS GSGTSYSLTISRVEAEDAATYYCQQWSSNPPTFGAGTKLELK (SEQ ID NO: 110) |
| F6.5.20VH | QVQLQQPGAELVKPGASVKLSCKASGYTFTSYWMQWVKQRPGQGLEWIGEIDPSDSYTNYNQ KFKGKASLTVDTSSSTAYMQLSSLTSEDSAVYYCARWGLLRNYYVMDYWGQGTSVTVSS (SEQ ID NO: 111) |
| F6.5.20VL | EIVLTQSPTTMAASPGEKITITCSASSSISSNYLHWYQQKPGFSPKLLIYRTSNLASGVPARFSGSG SGTSYSLTIGTMEAEDVATYYCQQGSSIPRMLTFGAGTKLELK (SEQ ID NO: 112) |
| F6.12.12VH | DVQLQESGPGLVKPSQSLSLTCSVTGYSITSGYYWIWIRQFPGNKLEWMGYISYDASNKYKPSL KNRISITRDTSKNQFFLKLNSVTAEDTATYFCAREDLRGYFDVWGTGTSVTVSS (SEQ ID NO: 113) |
| F6.12.12VL | DIVLTQSPASLAVSLGRRATISCRASQSVSTSGFSYMHWYQQKPGQPPNLLIKYASILQSGVPARF SGSGSGTDFILNIHPVEDVDTATYYCQHSWGIPFTFGSGTKLEIK (SEQ ID NO: 114) |

The Kabat numbering convention was used to analyze the CDRs of the heavy chain variable regions and the light chain variable regions of the aforementioned 53 clones (http://www.abysis.org/abysis/sequence_input/key_annotation/key_annotation.cgi). The specific results are detailed in Table 8.

**Table 8 Antibody heavy chain/light chain variable region CDRs**

| Designation of antibody | CDR1 | CDR2 | CDR3 |
|---|---|---|---|
| F1mab003 VH | DYVMH (SEQ ID NO: 115) | YFNPYNDGTNYNEKFKV (SEQ ID NO: 116) | GVYYYGRDFDY (SEQ ID NO: 117) |
| F1mab003 VL | RSSQSLENSNGNSYLN (SEQ ID NO: 118) | RVSNRFS (SEQ ID NO: 119) | LQITHVPWT (SEQ ID NO: 120) |
| F2.15.26VH | DYGVS (SEQ ID NO: 121) | VIWGSGDTYYNSVLES (SEQ ID NO: 122) | HSYYGGSFAMDY (SEQ ID NO: 123) |
| F2.15.26VL | RASQDISNYLN (SEQ ID NO: 124) | YTSRLHS (SEQ ID NO: 125) | QQGKTLPWT (SEQ ID NO: 126) |
| F3.3.10VH | SGYYWN (SEQ ID NO: 127) | HISYDGNNNYNPSLKN (SEQ ID NO: 128) | ENYSNYYFDY (SEQ ID NO: 129) |
| F3.3.10VL | RASQSVSKSSYTYIH (SEQ ID NO: 130) | YASILQS (SEQ ID NO: 131) | QHSWEIPYT (SEQ ID NO: 132) |
| F3.5.1VH | GSWMN (SEQ ID NO: 133) | RIYPGDGDTYYNGKFKD (SEQ ID NO: 134) | LRYRYVMDY (SEQ ID NO: 135) |
| F3.5.1VL | RASESVSIHGSHLMH (SEQ ID NO: 136) | AASNLES (SEQ ID NO: 137) | QQGIKDPYT (SEQ ID NO: 138) |
| F3.13.2VH | SGYYWN (SEQ ID NO: 139) | YISYDGSNNYNPSLKN (SEQ ID NO: 140) | RKLTGGPYFDY (SEQ ID NO: 141) |
| F3.13.2VL | RASESVSFHGFHIMH (SEQ ID NO: 142) | AASHLVS (SEQ ID NO: 143) | QQSVEDPWT (SEQ ID NO: 144) |
| F3.23.10VH | DYNMN (SEQ ID NO: 145) | IINPNFGSTSYNQKFKG (SEQ ID NO: 146) | SEYYGSSGFAY (SEQ ID NO: 147) |
| F3.23.10VL | RSSQSLVNSYGNTYLS (SEQ ID NO: 148) | GISNRFS (SEQ ID NO: 149) | LQGTHQPWT (SEQ ID NO: 150) |
| F3.40.14VH | GSWMN (SEQ ID NO: 151) | RIYPGDGDTYYSGKFKD (SEQ ID NO: 152) | LRSRYVMDY (SEQ ID NO: 153) |
| F3.40.14VL | RASESVSIHGTHLMH (SEQ ID NO: 154) | AASNLES (SEQ ID NO: 155) | QQGIDDPYT (SEQ ID NO: 156) |
| F3.64.4VH | DYHIN (SEQ ID NO: 157) | DINPNNGGTTYNQKFKD (SEQ ID NO: 158) | FITTWAWYFDV (SEQ ID NO: 159) |
| F3.64.4VL | RASENIYSNLA (SEQ ID NO: 160) | AATNLAD (SEQ ID NO: 161) | QRFWDTPRT (SEQ ID NO: 162) |
| F3.74.4VH | DYEMH (SEQ ID NO: 163) | GIAPETGLTTYNQKFED (SEQ ID NO: 164) | NNYGL (SEQ ID NO: 165) |
| F3.74.4VL | KSSQSLLDSNGKTYLN (SEQ ID NO: 166) | LVSKLDS (SEQ ID NO: 167) | WQGTHFPWT (SEQ ID NO: 168) |
| F3.77.6VH | DYNMN (SEQ ID NO: 169) | TINPNYITSYNQKFKG (SEQ ID NO: 170) | DYKGY (SEQ ID NO: 171) |
| F3.77.6VL | KSSQNLLDSDGKTYLN (SEQ ID NO: 172) | LVSKLDS (SEQ ID NO: 173) | WQGTHFPWT (SEQ ID NO: 174) |
| F3.81.2VH | NYWMH (SEQ ID NO: 175) | EIDPSDSYTDYNQNFKA (SEQ ID NO: 176) | LMGRYFDV (SEQ ID NO: 177) |
| F3.81.2VL | KASQDVSAAVA (SEQ ID NO: 178) | SASYRYT (SEQ ID NO: 179) | QQHYSTPLT (SEQ ID NO: 180) |
| F3.83.9VH | SGYYWN (SEQ ID NO: 181) | YISYDGGNNYNPSLKN (SEQ ID NO: 182) | EDYSNWYFDV (SEQ ID NO: 183) |
| F3.83.9VL | RASQSVSKSSYSYMH (SEQ ID NO: 184) | YASILKS (SEQ ID NO: 185) | QHSWEIPWT (SEQ ID NO: 186) |
| F3.109.1VH | SYGIN (SEQ ID NO: 187) | YIYIGNDYTEFNEKFKG (SEQ ID NO: 188) | FYSNSDPMDY (SEQ ID NO: 189) |
| F3.109.1VL | KSSQSLLNSDGKTFLN (SEQ ID NO: 190) | LVSKLDS (SEQ ID NO: 191) | WQGTHFPLT (SEQ ID NO: 192) |
| F3.113.16VH | NYWMQ (SEQ ID NO: 193) | EIDPSDSYINYNQKFKG (SEQ ID NO: 194) | PVTVPWYFDV (SEQ ID NO: 195) |
| F3.113.16VL | SATSSISSNYLH (SEQ ID NO: 196) | RTSNLAS (SEQ ID NO: 197) | QQGSAIPRIFT (SEQ ID NO: 198) |
| F3.114.12VH | SGYDWN (SEQ ID NO: 199) | YISYDGKNNYNPSLRN (SEQ ID NO: 200) | RDLRGYFDV (SEQ ID NO: 201) |
| F3.114.12VL | RASQSISNYLH (SEQ ID NO: 202) | YASLSIS (SEQ ID NO: 203) | QQSNSWPLT (SEQ ID NO: 204) |
| F3.118.14VH | DYEIH (SEQ ID NO: 205) | AIDPETGGIGYNQKFTG (SEQ ID NO: 206) | NYGSR (SEQ ID NO: 207) |
| F3.118.14VL | KSSQSLLESDGKTYLN (SEQ ID NO: 208) | LVSKLDS (SEQ ID NO: 209) | WQGTHFPWT (SEQ ID NO: 210) |
| F3.121.4VH | NYWMH (SEQ ID NO: 211) | EIDPSDNYANYNQEFQG (SEQ ID NO: 212) | HDGYFGALDY (SEQ ID NO: 213) |
| F3.121.4VL | SASSSISSNYLH (SEQ ID NO: 214) | RTSNLAS (SEQ ID NO: 215) | QQASSIPRMFT (SEQ ID NO: 216) |
| F3.124.7VH | DYYMN (SEQ ID NO: 217) | ISNPYNGGTSYNQKFKD (SEQ ID NO: 218) | EGGTAQPTSGMDY (SEQ ID NO: 219) |
| F3.124.7VL | KSSQSVLYSSNQKNYLA (SEQ ID NO: 220) | WASTRES (SEQ ID NO: 221) | HQYRSSYT (SEQ ID NO: 222) |
| F3.126.15VH | SYWMH (SEQ ID NO: 223) | EIDPSDSYTTYNQNFKG (SEQ ID NO: 224) | LTGNYIHY (SEQ ID NO: 225) |
| F3.126.15VL | KASQDVSPAVA (SEQ ID NO: 226) | SSSYRST (SEQ ID NO: 227) | QQHYSLPLT (SEQ ID NO: 228) |
| F3.133.14VH | NYWMH (SEQ ID NO: 229) | EIDPSDYYANYNQKFKG (SEQ ID NO: 230) | SHYYANSYNY (SEQ ID NO: 231) |
| F3.133.14VL | SASSSISSDYLH (SEQ ID NO: 232) | RTSNLAS (SEQ ID NO: 233) | QQGSSVPRMLT (SEQ ID NO: 234) |
| F3.148.10VH | NYGIN (SEQ ID NO: 235) | YIFIGNDYTEYNEKFKG (SEQ ID NO: 236) | FYSNYYGMDP (SEQ ID NO: 237) |
| F3.148.10VL | KSSQSLLHSDGKTYLN (SEQ ID NO: 238) | LVSKLDS (SEQ ID NO: 239) | WQGTHFPPT (SEQ ID NO: 240) |
| F4.4.6VH | SYWMQ (SEQ ID NO: 241) | AIYPGDGDSRYTQKFKG (SEQ ID NO: 242) | STTTVLGNNFEY (SEQ ID NO: 243) |
| F4.4.6VL | SASSSVSSMH (SEQ ID NO: 244) | DTSKLAS (SEQ ID NO: 245) | QQWSSNPWT (SEQ ID NO: 246) |
| F4.15.9VH | SSWMN (SEQ ID NO: 247) | RIYPGDGDTNYNGKFKG (SEQ ID NO: 248) | GITTIITIAWFAS (SEQ ID NO: 249) |
| F4.15.9VL | RASSSVSYMH (SEQ ID NO: 250) | ATSNLAS (SEQ ID NO: 251) | QQWSSDPWT (SEQ ID NO: 252) |
| F4.18.13VH | SFWMN (SEQ ID NO: 253) | RIYPGDGDTNYNGKFKG (SEQ ID NO: 254) | EVIAA VVTTDFDY (SEQ ID NO: 255) |
| F4.18.13VL | RTSQDISNYLN (SEQ ID NO: 256) | YTSGLHS (SEQ ID NO: 257) | QQGKTLPYT (SEQ ID NO: 258) |
| F4.21.2VH | TYGVH (SEQ ID NO: 259) | VIWIGGTTDYNAAFIS (SEQ ID NO: 260) | KGYYKYDGGYYYAMDY (SEQ ID NO: 261) |
| F4.21.2VL | KASDHINNWLA (SEQ ID NO: 262) | GATSLET (SEQ ID NO: 263) | QQYWSTPLT (SEQ ID NO: 264) |
| F4.22.6VH | SFWMN (SEQ ID NO: 265) | RIYPGDGDTNYNGKFKG (SEQ ID NO: 266) | SVITA VVDWYFDV (SEQ ID NO: 267) |
| F4.22.6VL | RASSSVNFMH (SEQ ID NO: 268) | ATSNLAS (SEQ ID NO: 269) | QQWSSYPIFT (SEQ ID NO: 270) |
| F4.33.12VH | SYWMN (SEQ ID NO: 271) | QIYPGDGDTNYNGKFKG (SEQ ID NO: 272) | AGFYYGSDSSMDY (SEQ ID NO: 273) |
| F4.33.12VL | RASESVDNFGISFMN (SEQ ID NO: 274) | AASNQGS (SEQ ID NO: 275) | QQSKEVPYT (SEQ ID NO: 276) |
| F4.35.5VH | SYNMY (SEQ ID NO: 277) | YIDPYNGDTRYNQKFKG (SEQ ID NO: 278) | SGTGRDY (SEQ ID NO: 279) |
| F4.35.5VL | SASSSISYMF (SEQ ID NO: 280) | DTSSLAS (SEQ ID NO: 281) | QQWSNYPYT (SEQ ID NO: 282) |
| F4.37.6VH | SFWMQ (SEQ ID NO: 283) | TIYPGDGDTRYTQKFKG (SEQ ID NO: 284) | GALSNYGGFAY (SEQ ID NO: 285) |
| F4.37.6VL | RASESVDSYGHSFMH (SEQ ID NO: 286) | LASNLES (SEQ ID NO: 287) | QQNNEDPWT (SEQ ID NO: 288) |
| F4.41.1VH | NYLIE (SEQ ID NO: 289) | VINPGSGDTLYNEKFKG (SEQ ID NO: 290) | SPPITTIVADYFDY (SEQ ID NO: 291) |
| F4.41.1VL | RASQDISNYLN (SEQ ID NO: 292) | YTSRLHS (SEQ ID NO: 293) | QQGKTLPYT (SEQ ID NO: 294) |
| F4.48.2VH | DYGVS (SEQ ID NO: 295) | VIWGSGSTFYNSALTS (SEQ ID NO: 296) | HHYYGGSFAMDY (SEQ ID NO: 297) |
| F4.48.12VL | KTSQDISNYLN (SEQ ID NO: 298) | YTSRLHS (SEQ ID NO: 299) | QQGNSLPYT (SEQ ID NO: 300) |
| F4.49.11VH | SGYYWN (SEQ ID NO: 301) | CISYDGTNNYNPSLKN (SEQ ID NO: 302) | RVYYRYDGWDY (SEQ ID NO: 303) |
| F4.49.11VL | SASSSVSYIH (SEQ ID NO: 304) | DTSKLAS (SEQ ID NO: 305) | FQGSGYPLT (SEQ ID NO: 306) |
| F4.58.6VH | SYWMH (SEQ ID NO: 307) | EIDPSDSYSNYNQKFKG (SEQ ID NO: 308) | SNYGSSYGYFDV (SEQ ID NO: 309) |
| F4.58.6VL | SVSSSLSYMH (SEQ ID NO: 310) | DTSKLAS (SEQ ID NO: 311) | HQRSSYPT (SEQ ID NO: 312) |
| F4.78.1VH | NYWMQ (SEQ ID NO: 313) | AIYPGDGDTRYTQKFKG (SEQ ID NO: 314) | EEVWSPYGMDY (SEQ ID NO: 315) |
| F4.78.IVL | SASSSVSYMG (SEQ ID NO: 316) | STSNLAS (SEQ ID NO: 317) | QQRSIYPLT (SEQ ID NO: 318) |
| F4.82.11VH | TFGIGVG (SEQ ID NO: 319) | HIWWNDNKFFNTTLKN (SEQ ID NO: 320) | SRSALITTGLYAMDY (SEQ ID NO: 321) |
| F4.82.11VL | RSSQDISNYVN (SEQ ID NO: 322) | YTSRLHS (SEQ ID NO: 323) | QQGFTLPYT (SEQ ID NO: 324) |
| F4.96.6VH | AYNIY (SEQ ID NO: 325) | YIDPYNSGTTYNQKFRG (SEQ ID NO: 326) | DGDAMDY (SEQ ID NO: 327) |
| F4.96.6VL | SASSGVIYMN (SEQ ID NO: 328) | DVSTLAS (SEQ ID NO: 329) | QQRSSYPYT (SEQ ID NO: 330) |
| F4.107.12VH | SYVLH (SEQ ID NO: 331) | YFNPYNDGTEYNEKFKG (SEQ ID NO: 332) | GTYYYGSSYPFAY (SEQ ID NO: 333) |
| F4.107.12VL | RSSQSLIHSNGNTYLQ (SEQ ID NO: 334) | KVSNRLS (SEQ ID NO: 335) | SQSTHVPYT (SEQ ID NO: 336) |
| F4.125.12VH | TYTMS (SEQ ID NO: 337) | FISSGGGHINYPDTVKG (SEQ ID NO: 338) | HSYWFFDV (SEQ ID NO: 339) |
| F4.125.12VL | RASKNIDTYLA (SEQ ID NO: 340) | SGSTLQS (SEQ ID NO: 341) | QQHNEYPLT (SEQ ID NO: 342) |
| F4.145.9VH | SYDMS (SEQ ID NO: 343) | YISAGGGNTYFLDTVKG (SEQ ID NO: 344) | HGDYYRYFYAMDY (SEQ ID NO: 345) |
| F4.145.9VL | KASQDVGTAVA (SEQ ID NO: 346) | WASTRHT (SEQ ID NO: 347) | QQFVTYPYT (SEQ ID NO: 348) |
| F4.152.7VH | SYWMH (SEQ ID NO: 349) | EIDPSDSYTNYNQKFKG (SEQ ID NO: 350) | GGDYDGYYVMDY (SEQ ID NO: 351) |
| F4.152.7VL | SASSSISSNYLH (SEQ ID NO: 352) | RTSNLAS (SEQ ID NO: 353) | QQGSYIPRIFT (SEQ ID NO: 354) |
| F4.180.1VH | ENIIH (SEQ ID NO: 355) | GINPDNGGTSYHQKFKG (SEQ ID NO: 356) | QAPLYWYFDI (SEQ ID NO: 357) |
| F4.180.1VL | RASESVDSYGNSLMH (SEQ ID NO: 358) | RASNLES (SEQ ID NO: 359) | QQSNEDPWT (SEQ ID NO: 360) |
| F4.191.1VH | SYWIN (SEQ ID NO: 361) | NIHPSDSYTNYNQKFKD (SEQ ID NO: 362) | EDYYYGSNYDAMDY (SEQ ID NO: 363) |
| F4.191.1VL | KASQDVGSSVA (SEQ ID NO: 364) | WASTRHA (SEQ ID NO: 365) | QQYSTYPLT (SEQ ID NO: 366) |
| F4.272.13VH | DYGVS (SEQ ID NO: 367) | VIWGSETTFYNSALKS (SEQ ID NO: 368) | HHYYGGSFSMDY (SEQ ID NO: 369) |
| F4.272.13VL | RASQDINNYLN (SEQ ID NO: 370) | YTSRLHS (SEQ ID NO: 371) | QQGNTLPYT (SEQ ID NO: 372) |
| F4.276.12VH | AYNMY (SEQ ID NO: 373) | YIDPYNGGTNYNQKFKG (SEQ ID NO: 374) | DGYEVTY (SEQ ID NO: 375) |
| F4.276.12VL | SASSSVTYMH (SEQ ID NO: 376) | STSNLAS (SEQ ID NO: 377) | HQWSSYPWT (SEQ ID NO: 378) |
| F5.2.9VH | TFGLGVG (SEQ ID NO: 379) | HIWWDDDKYYNPALKS (SEQ ID NO: 380) | LSRGLRRDWYAMDH (SEQ ID NO: 381) |
| F5.2.9VL | RASQDISNYLN (SEQ ID NO: 382) | YVSRLQS (SEQ ID NO: 383) | QQGIAFPYT (SEQ ID NO: 384) |
| F5.3.6VH | SGYYWN (SEQ ID NO: 385) | YISYDGSNNYNPSLKN (SEQ ID NO: 386) | APLTGDRYWYFDV (SEQ ID NO: 387) |
| F5.3.6VL | RASQSVSTSSYSYMH (SEQ ID NO: 388) | YASNLKS (SEQ ID NO: 389) | QHSWEIPPT (SEQ ID NO: 390) |
| F5.7.1VH | TFGMGVG (SEQ ID NO: 391) | HIWWDDDKYYNPALKS (SEQ ID NO: 392) | VITTGVPYFDY (SEQ ID NO: 393) |
| F5.7.1VL | RASSSVSSSYLY (SEQ ID NO: 394) | STSNLAS (SEQ ID NO: 395) | QQYSGYPWT (SEQ ID NO: 396) |
| F5.8.6VH | SYWMH (SEQ ID NO: 397) | EIDPSDSYTTYDQKFKG (SEQ ID NO: 398) | LTGNHLDY (SEQ ID NO: 399) |
| F5.8.6VL | KASQDVSPAVA (SEQ ID NO: 400) | STSYRNT (SEQ ID NO: 401) | QQHYSIPLT (SEQ ID NO: 402) |
| F5.10.6VH | SGYYWT (SEQ ID NO: 403) | YISYDGSTNYNPSLKN (SEQ ID NO: 404) | DRMSTTWYFDV (SEQ ID NO: 405) |
| F5.10.6VL | RASESVDNYGISFMH (SEQ ID NO: 406) | RASKTES (SEQ ID NO: 407) | QQSNKYPRT (SEQ ID NO: 408) |
| F5.11.2VH | SYGMS (SEQ ID NO: 409) | TISHGGSYTHYPDSVKG (SEQ ID NO: 410) | LPITTVEAPYFFDY (SEQ ID NO: 411) |
| F5.11.2VL | RASQDIRNYLN (SEQ ID NO: 412) | YTSILHS (SEQ ID NO: 413) | QQGNTLYT (SEQ ID NO: 414) |
| F6.2.9VH | SYWMN (SEQ ID NO: 415) | QIYPGDGDTNYNGKFKG (SEQ ID NO: 416) | SRITSVVDWYFDV (SEQ ID NO: 417) |
| F6.2.9VL | RASSSVSYMH (SEQ ID NO: 418) | ATSNLAS (SEQ ID NO: 419) | QQWSSNPPT (SEQ ID NO: 420) |
| F6.5.20VH | SYWMQ (SEQ ID NO: 421) | EIDPSDSYTNYNQKFKG (SEQ ID NO: 422) | WGLLRNYYVMDY (SEQ ID NO: 423) |
| F6.5.20VL | SASSSISSNYLH (SEQ ID NO: 424) | RTSNLAS (SEQ ID NO: 425) | QQGSSIPRMLT (SEQ ID NO: 426) |
| F6.12.12VH | SGYYWI (SEQ ID NO: 427) | YISYDASNKYKPSLKN (SEQ ID NO: 428) | EDLRGYFDV (SEQ ID NO: 429) |
| F6.12.12VL | RASQSVSTSGFSYMH (SEQ ID NO: 430) | YASILQS (SEQ ID NO: 431) | QHSWGIPFT (SEQ ID NO: 432) |

### 4.2 Preparation of chimeric antibody

Biointron (Jiangsu) Biological Inc. was entrusted to clone the heavy chain variable region sequences of the aforementioned 53 clones into expression vector pcDNA3.4-B1HH1 containing a signal peptide and the heavy chain constant region of human antibody IgG1 (the sequence of the heavy chain constant region is as shown in SEQ ID NO: 433), respectively, clone the light chain variable region sequence of the clone in which the light chain is a Kappa chain into expression vector pcDNA3.4-B1HLK containing a signal peptide and the Kappa light chain constant region of human antibody IgG1 (the sequence of the light chain constant region is as shown in SEQ ID NO: 434), and clone the light chain variable region sequence of the clone in which the light chain is a Lambda chain into expression vector pcDNA3.4-BIHL5 containing a signal peptide and the Lambda light chain constant region of human antibody IgG1 (the sequence of the light chain constant region is as shown in SEQ ID NO: 435), and thus the expression vector of the human-murine chimeric antibody was obtained. The antibody was prepared according to the method in example 1.2. The heavy chain and light chain constant region sequence information is as follows:

ASTKGPSVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPENNYKTTPPVLDSDGSFF
LYSKLTVDKSRWQQGNVFSCSVMHEALHNHYTQKSLSLSPGK (SEQ ID NO: 433).


 (SEQ ID NO: 434).

GQPKANPTVTLFPPSSEELQANKATLVCLISDFYPGAVTVAWKADGSPVKAGVETTKPSKQSNNKYAASS
YLSLTPEQWKSHRSYSCQVTHEGSTVEKTVAPTECS (SEQ ID NO: 435).

### Example 5 Identification of CD19 chimeric antibodies

### 5.1 The binding of chimeric antibodies to human CD19 protein detected by enzyme-linked immunosorbent assay (ELISA)

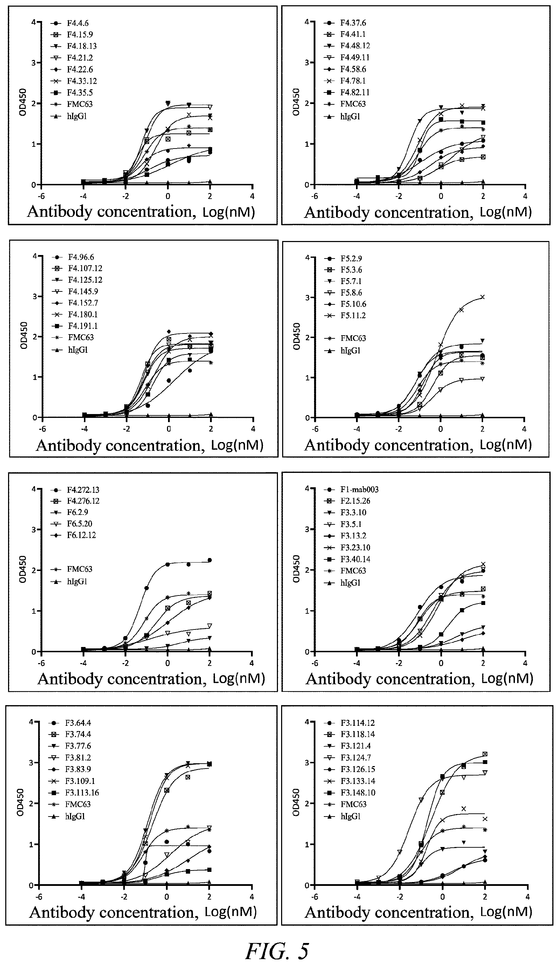

Human CD19-his protein (purchased from ACROBiosystems, catalog number: CD9-H52H2) was diluted with PBS to a final concentration of 4 µg/mL, and then added to a 96-well ELISA plate at 100 µl/well. The plate was sealed with a plastic film and incubated overnight at 4°C, the plate was washed 2 times with PBS the next day, and then a blocking solution [PBS+2% (w/w) BSA] was added for blocking at room temperature for 2 h. The blocking solution was poured off, and 100 nM gradiently diluted CD19 chimeric antibody or control antibody was added at 50 µl/well. After incubation at 37°C for 2 h, the plate was washed 3 times with PBS. HRP (horseradish peroxidase)-labeled secondary antibody (purchased from Jackson, catalog number: 109-035-088) was added, and incubated at 37°C for 1 h, and the plate was washed 5 times with PBS. TMB substrate was added at 50 µl/well, and incubated at room temperature for 10 min, and then a stop solution (1.0 N HCl) was added at 50 µl/well. The OD450nm values were read by an ELISA microplate reader (Multimode Plate Reader, EnSight, purchased from Perkin Elmer). The binding results of the chimeric antibodies to human CD19 detected by ELISA are as shown in FIG. 5 and Tables 9-10. The results show that the chimeric antibodies all can bind to human CD19 at ELISA level. Unless particularly stated, the control antibody hIgG1 used in the identification or detection of CD19 chimeric antibodies in example 5, example 6, example 7 and example 8 was antibody anti-hel-hIgG1 (purchased from Biointron, catalog number: B11790 1) against hen egg lysozyme, the FMC63 antibody was FMC63-hIgG1 prepared in example 1.2, and the 9G8 was 9G8-hIgG1 prepared in example 1.2.

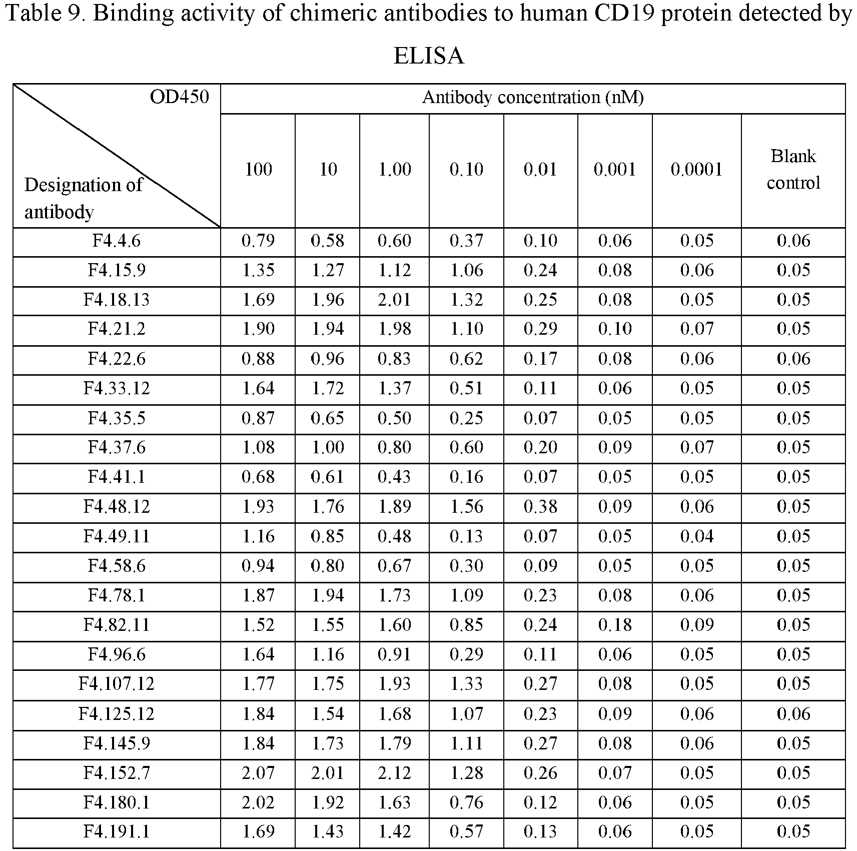

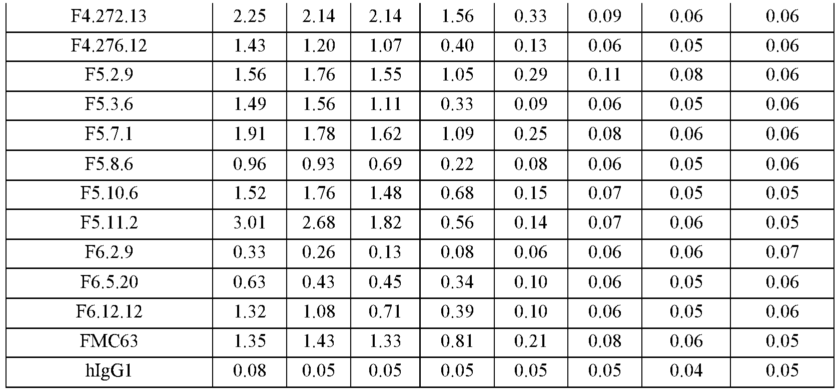

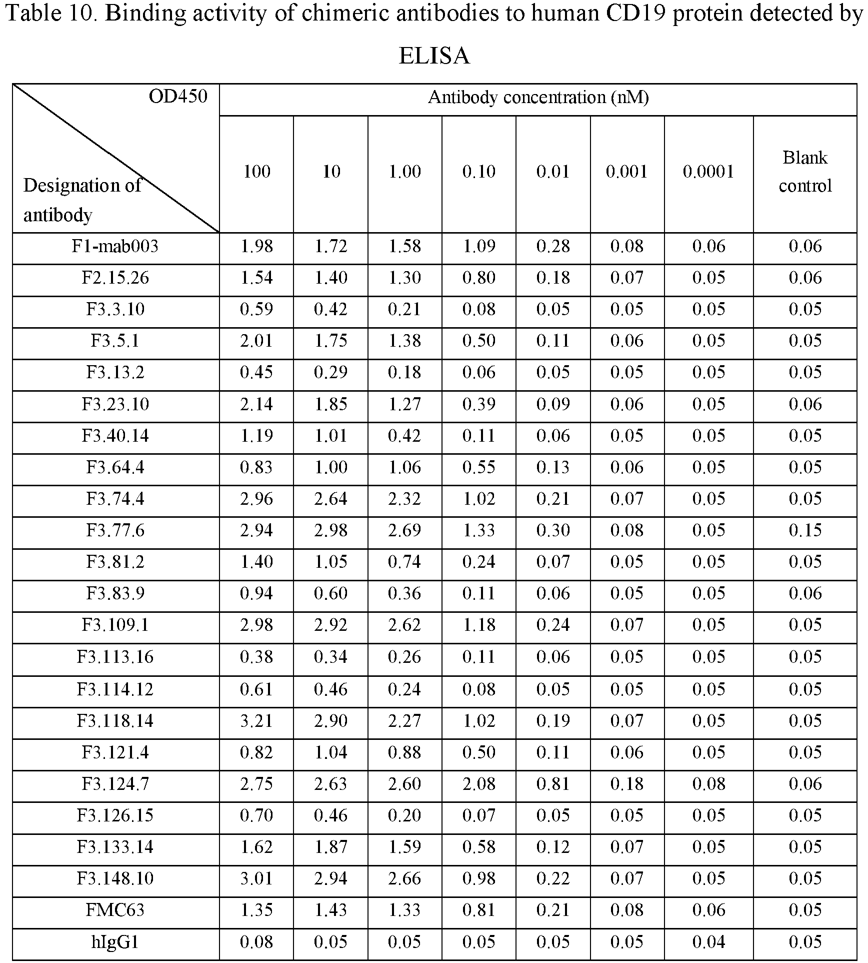

### 5.2 The binding of chimeric antibodies to different CD19 expressing cells detected by fluorescence-activated cell sorting (FACS)

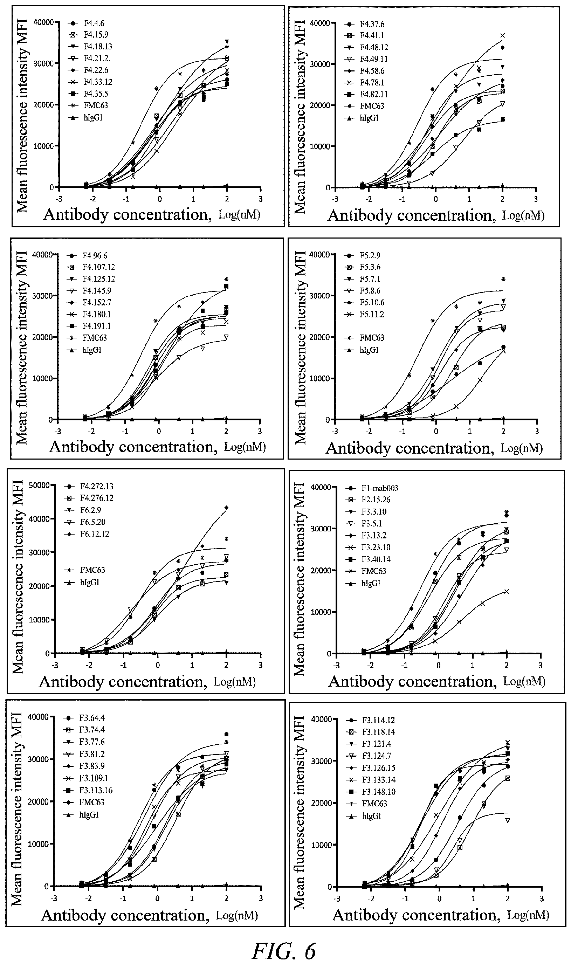

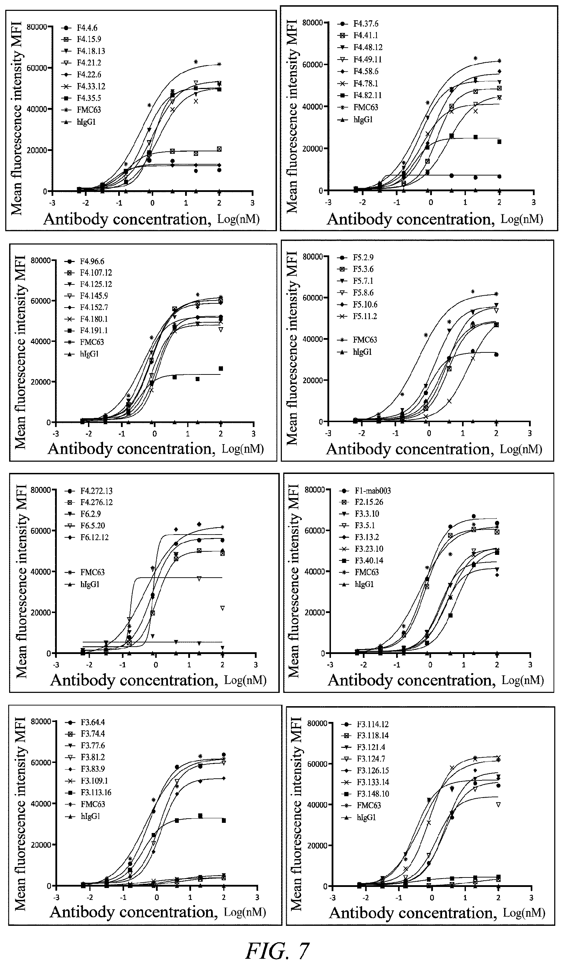

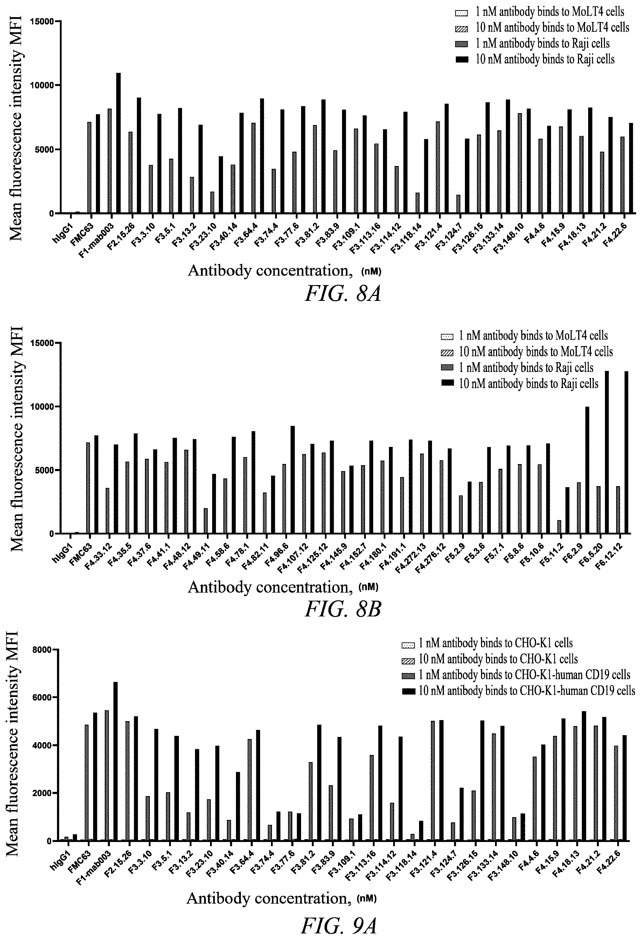

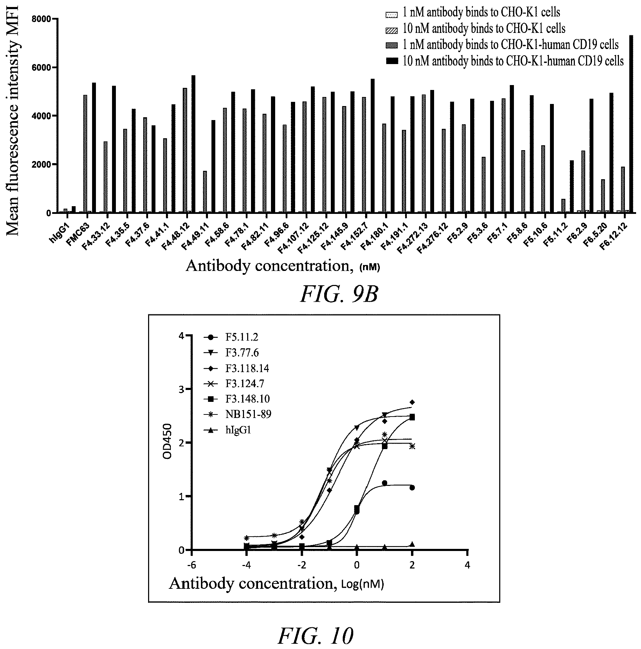

The desired cells were scale-up cultured in a T-75 cell culture flask to the logarithmic growth phase. For the adherent cell CHO-K1, the medium was aspirated, the cells were washed 2 times with a PBS buffer, and then digested with trypsin. After the digestion was terminated, the cells were washed 2 times with a PBS buffer; and for suspension cell Raji, the supernatant of the medium was discarded by directly centrifugation, and the cell pellet was washed 2 times with PBS. After counting the cells in the previous step, the cell pellet was resuspended with [PBS + 2% (w/w) BSA] blocking solution to 2 × 10⁶ cells/ml, and added to a 96-well FACS reaction plate at 50 µl/well, and then the sample to be tested was added at 50 µl/well, and incubated on ice for 2 h. The obtained mixture was centrifuged and washed 3 times with a PBS buffer. Alexa Fluor 647-labeled secondary antibody (purchased from Jackson Immuno, catalog number: 109-605-088) was added at 50 µl/well, and the cells were incubated on ice for 1 h. The obtained mixture was centrifuged and washed 5 times with a PBS buffer, and FACS (FACS CantoTM, purchased from BD Company) was used for detection and result analysis. Data analysis was performed by software (FlowJo) to obtain the mean fluorescence intensity (MFI) of the cells. And then software (GraphPad Prism8) was used for analysis, data fitting, and EC50 value calculation. The analysis results are as shown in Table 11 and FIGs. 6-7, indicating that the chimeric antibodies all can bind to human CD19 protein on the surface of Raji cells and CHO-K1-human CD19 cells. The same method was used to detect the binding of the chimeric antibodies to endogenous CD19-negative cells, MoLT4 cells (purchased from ATCC, CRL-1582), and CHO-K1 cells. The results are as shown in FIGs. 8A-8B and 9A-9B, indicating that all chimeric antibodies do not bind to MoLT4 cells and CHO-K1 cells, and have good specificity.

**Table 11. Binding activity of chimeric antibodies to Raji cells and CHO-K1-human CD19 cells detected by FACS**

| Designation of antibody | Raji | | CHO-K1-human CD19 | |
|---|---|---|---|---|
| | Maximum mean fluorescence intensity | Ec50 (nM) | Maximum mean fluorescence intensity | Ec50 (nM) |
| F1-mab003 | 33119 | 0.57 | 66886 | 0.69 |
| F2.15.26 | 29188 | 0.53 | 60386 | 0.71 |
| F3.3.10 | 29731 | 2.90 | 48331 | 1.90 |
| F3.5.1 | 24846 | 1.47 | 49924 | 2.49 |
| F3.13.2 | 27124 | 5.24 | 43895 | 2.46 |
| F3.23.10 | 14876 | 4.77 | 50411 | 3.59 |
| F3.40.14 | 27003 | 2.59 | 49094 | 6.00 |
| F3.64.4 | 35797 | 0.43 | 63727 | 0.61 |
| F3.74.4 | 30072 | 3.41 | 3848 | 4.55 |
| F3.77.6 | 27416 | 1.62 | 4993 | 3.13 |
| F3.81.2 | 31265 | 0.68 | 59550 | 1.27 |
| F3.83.9 | 29681 | 1.75 | 52231 | 1.34 |
| F3.109.1 | 29926 | 0.44 | 3977 | 0.57 |
| F3.113.16 | 28820 | 0.96 | 34114 | 0.39 |
| F3.114.12 | 28655 | 3.34 | 50448 | 2.32 |
| F3.118.14 | 25898 | 8.38 | 3382 | 18.02 |
| F3.121.4 | 32899 | 0.31 | 53703 | 0.30 |
| F3.124.7 | 19120 | 2.46 | 47489 | 1.35 |
| F3.126.15 | 30253 | 1.18 | 56743 | 2.47 |
| F3.133.14 | 34425 | 0.93 | 63055 | 0.82 |
| F3.148.10 | 31796 | 0.28 | 4699 | 0.26 |
| F4.4.6 | 26023 | 0.56 | 14826 | 0.08 |
| F4.15.9 | 31255 | 0.71 | 20531 | 0.17 |
| F4.18.13 | 35189 | 1.31 | 51550 | 0.60 |
| F4.21.2 | 30806 | 2.69 | 52607 | 1.02 |
| F4.22.6 | 27259 | 0.67 | 14690 | 0.10 |
| F4.33.12 | 28242 | 2.60 | 52468 | 1.47 |
| F4.35.5 | 25022 | 0.69 | 50040 | 1.08 |
| F4.37.6 | 24606 | 0.51 | 8487 | ~0.035 |
| F4.41.1 | 23428 | 1.02 | 48631 | 1.49 |
| F4.48.12 | 29304 | 0.55 | 52723 | 0.48 |
| F4.49.11 | 20285 | 6.21 | 44151 | 3.10 |
| F4.58.6 | 26054 | 1.31 | 56739 | 1.09 |
| F4.78.1 | 36926 | 1.52 | 44268 | 0.51 |
| F4.82.11 | 16604 | 0.85 | 25999 | 0.32 |
| F4.96.6 | 25703 | 0.74 | 52038 | 1.16 |
| F4.107.12 | 26659 | 0.60 | 59936 | 0.77 |
| F4.125.12 | 27141 | 0.51 | 51838 | 0.48 |
| F4.145.9 | 19978 | 0.78 | 48541 | 0.84 |
| F4.152.7 | 26038 | 0.76 | 58864 | 0.78 |
| F4.180.1 | 23666 | 0.96 | 49608 | 1.24 |
| F4.191.1 | 32270 | 1.89 | 26491 | 0.34 |
| F4.272.13 | 27578 | 0.81 | 55315 | 0.77 |
| F4.276.12 | 23464 | 0.78 | 50192 | 1.05 |
| F5.2.9 | 17598 | 2.79 | 33972 | 1.02 |
| F5.3.6 | 22385 | 3.19 | 46881 | 3.62 |
| F5.7.1 | 28747 | 1.13 | 56215 | 1.54 |
| F5.8.6 | 27270 | 1.26 | 53607 | 3.50 |
| F5.10.6 | 22107 | 1.34 | 47670 | 2.60 |
| F5.11.2 | 16600 | 23.43 | 46606 | 14.67 |
| F6.2.9 | 21477 | 0.98 | 10293 | Weak binding |
| F6.5.20 | 28636 | 0.17 | 48296 | ∼ 0.17 |
| F6.12.12 | 43284 | 5.25 | 63253 | 0.84 |
| FMC63 | 33996 | 0.29 | 62943 | 0.49 |
| hIgG1 | 435 | Negative | 99 | Negative |

### Example 6 Detection of species cross-binding activity of chimeric antibodies

### 6.1 The binding of chimeric antibodies to CD19 proteins of different species detected by ELISA

In order to detect the species cross-binding activity of the chimeric antibodies, an ELISA plate was coated with commercial murine CD19 (ACROBiosystems, catalog number: 50510-M08H) and monkey CD19 (ACROBiosystems, catalog number: 90051-C08H), respectively, and the ELISA detection was performed according to the method in example 5.1. None of the chimeric antibodies bound to murine CD19 protein at ELISA level.

The binding results of the chimeric antibodies to monkey CD19 detected by ELISA are as shown in FIG. 10 and Table 12, indicating that F5.11.2, F3.77.6, F3.118.14, F3.124.7, F3.148.10 have binding activity to monkey CD19 protein. hIgG1 is negative control and NB151-89 is a positive serum capable of binding to monkey CD19-His protein.

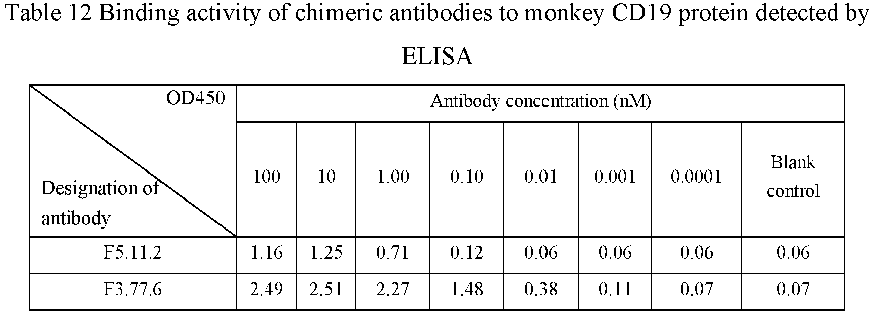

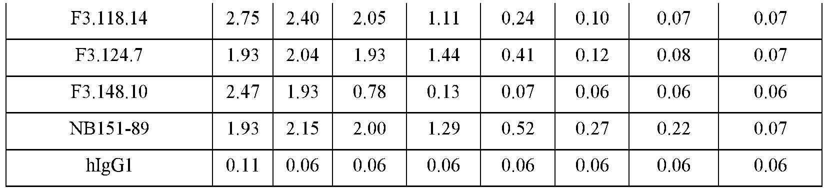

### 6.2 Binding of chimeric antibodies to monkey CD19 expressing cells detected by FACS

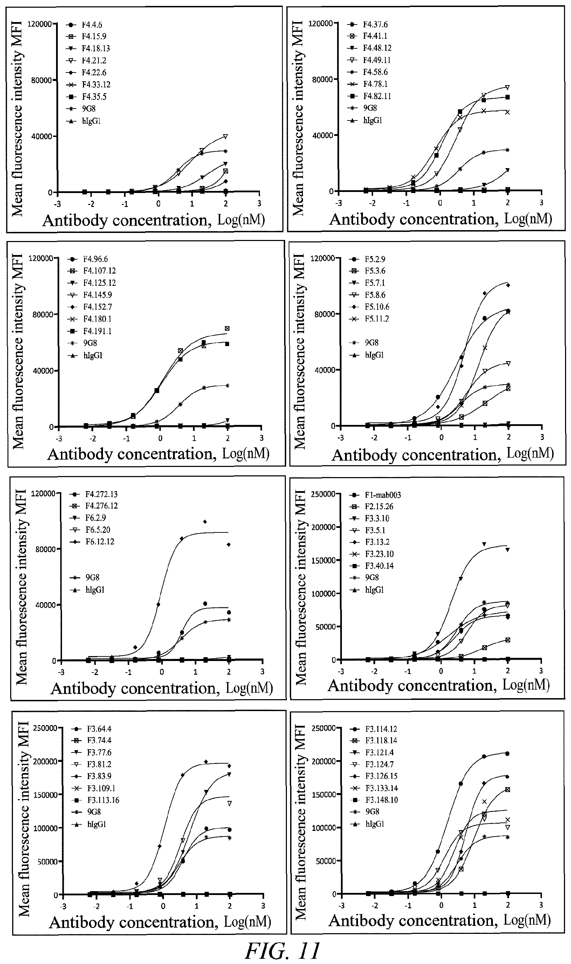

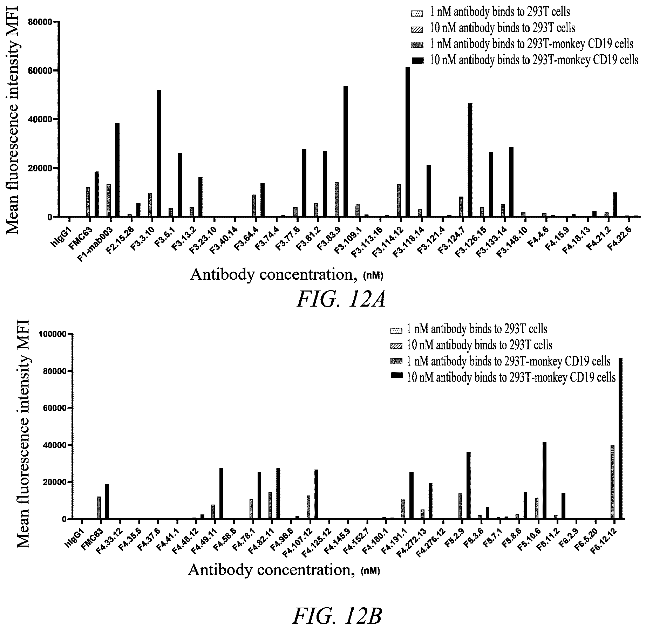

HEK293T-monkey CD19 cells were subjected to FACS detection and data analysis according to the method in example 5.2. The analysis results are as shown in Tables 13-14 and FIG. 11, indicating that except for F3.23.10, F3.40.14, F3.74.4, F3.109.1, F3.113.16, F3.121.4, F3.148.10, F4.4.6, F4.33.12, F4.35.5, F4.37.6, F4.41.1, F4.58.6, F4.96.6, F4.145.9, F4.152.7, F4.180.1, F4.276.12, F5.7.1 and F6.5.20, all the other chimeric antibodies have binding activity to 293T cells overexpressing monkey CD19, and the EC50 shows that the highest binding activity is up to 0.73 nM. The same method was used to detect binding of the chimeric antibodies to HEK293T cells. The results are as shown in FIGs. 12A-12B, indicating that none of the chimeric antibodies bind to HEK293T cells.

**Table 13 Binding activity of CD19 antibodies to 293T-monkey CD19 cells detected by FACS**

| Designation of antibody | 293T-monkey CD19 | |
|---|---|---|
| | Maximum mean fluorescence intensity | Ec50 (nM) |
| F1-mab003 | 75050 | 1.76 |
| F2.15.26 | 28708 | 16.68 |
| F3.3.10 | 173329 | 2.08 |
| F3.5.1 | 79963 | 6.11 |
| F3.13.2 | 66411 | 2.64 |
| F3.23.10 | 77 | No binding |
| F3.40.14 | 281 | No binding |
| F3.64.4 | 98360 | 3.99 |
| F3.74.4 | 121 | No binding |
| F3.77.6 | 178868 | 6.45 |
| F3.81.2 | 152647 | 3.44 |
| F3.83.9 | 198489 | 1.12 |
| F3.109.1 | 148 | No binding |
| F3.113.16 | 176 | No binding |
| F3.114.12 | 210030 | 1.56 |
| F3.118.14 | 156008 | 9.64 |
| F3.121.4 | 80 | No binding |
| F3.124.7 | 112207 | 1.27 |
| F3.126.15 | 175195 | 5.57 |
| F3.133.14 | 138298 | 2.47 |
| F3.148.10 | 177 | No binding |
| 9G8 | 85742 | 3.00 |
| hIgG1 | 90 | Negative |

**Table 14 Binding activity of CD19 antibodies to 293T-monkey CD19 cells detected by FACS**

| Designation of antibody | 293T-monkey CD19 | |
|---|---|---|
| | Maximum mean fluorescence intensity | Ec50 (nM) |
| F4.4.6 | 662 | No binding |
| F4.15.9 | 14750 | Weak binding |
| F4.18.13 | 19701 | 26.17 |
| F4.21.2 | 39235 | 9.10 |
| F4.22.6 | 7380 | -129.2 |
| F4.33.12 | 50 | No binding |
| F4.35.5 | 70 | No binding |
| F4.37.6 | 58 | No binding |
| F4.41.1 | 545 | No binding |
| F4.48.12 | 14334 | 183.90 |
| F4.49.11 | 73507 | 3.08 |
| F4.58.6 | 81 | No binding |
| F4.78.1 | 57041 | 0.73 |
| F4.82.11 | 66592 | 1.18 |
| F4.96.6 | 73 | No binding |
| F4.107.12 | 69545 | 1.24 |
| F4.125.12 | 4080 | Weak binding |
| F4.145.9 | 108 | No binding |
| F4.152.7 | 58 | No binding |
| F4.180.1 | 94 | No binding |
| F4.191.1 | 59560 | 1.14 |
| F4.272.13 | 40481 | 3.86 |
| F5.2.9 | 81524 | 2.82 |
| F5.3.6 | 26083 | 20.67 |
| F5.7.1 | 938 | No binding |
| F5.8.6 | 43844 | 5.70 |
| F5.10.6 | 99874 | 4.98 |
| F5.11.2 | 80550 | 13.00 |
| F4.276.12 | 597 | No binding |
| F6.2.9 | 2133 | 85.98 |
| F6.5.20 | 531 | No binding |
| F6.12.12 | 99105 | 0.94 |
| 9G8 | 28850 | 3.64 |
| hIgG1 | 90 | Negative |

### 6.3 The binding of chimeric antibodies to peripheral blood B cells of cynomolgus monkey (Latin name: Macaca fascicularis) detected by FACS

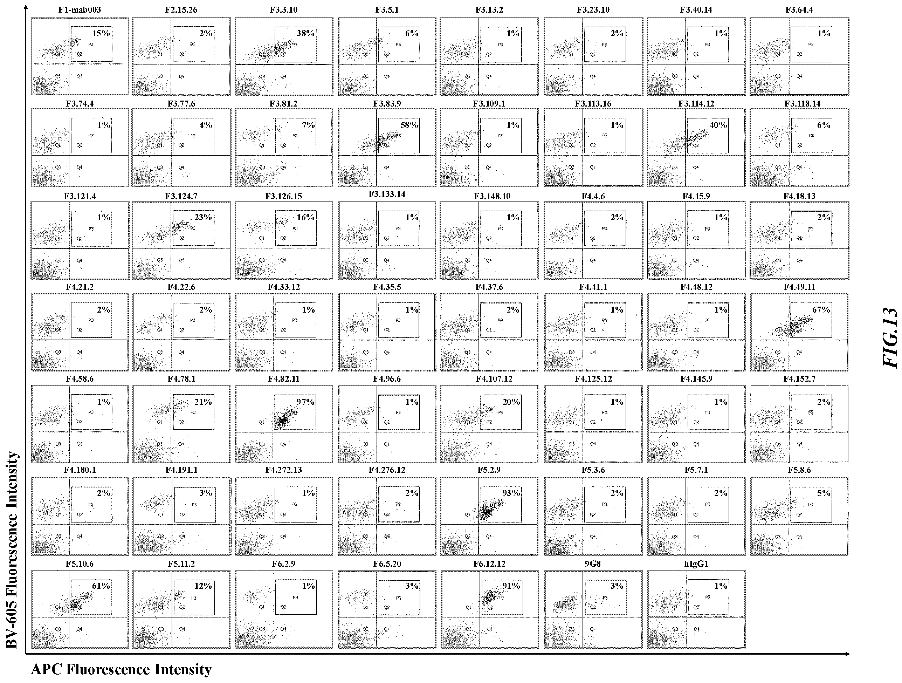

The monkey peripheral blood mononuclear cells were extracted from fresh cynomolgus monkey peripheral blood (purchased from Shanghai Medicilon Inc.) according to the instructions of *Ficoll-Paque Plus* (purchased from GE Healthcae, catalog number: 171440-02). After the cell suspension was centrifuged, the cells were resuspended in PBS containing 1% BSA, and then the cells were counted. At the same time, murine antibody Brilliant Violet 605 anti-human CD20 (catalog number: 302334, purchased from Biolegend) having monkey CD20 cross-binding activity and the chimeric antibodies to be tested (1 nM, 10 nM and 100 nM) were added. The mixture was incubated for 1 h at room temperature. After washing the cells three times, APC-labeled secondary antibody anti-human IgG Fc (catalog number: 409306, purchased from Biolegend) was added. After incubation at room temperature in the dark for 30 minutes, the cells were washed 5 times, gently resuspended with PBS containing 1% BSA, and detected and analyzed by FACS (FACS CantoTM, purchased from BD Company), in which CD20 was used as a marker of B cells to gate the CD20-positive B cell population, the proportion of the chimeric antibody-labeled positive cells was analyzed, and the proportion of the chimeric antibody positive cell population to B cell population was calculated after treatments with the chimeric antibodies at the concentrations of 100 nM, 10 nM and 1 nM, respectively. The results are as shown in Table 15 and FIG. 13. It can be seen from the results that F1-mab003, F3.3.10, F3.83.9, F3.114.12, F3.124.7, F3.126.15, F4.49.11, F4.78.1, F4.82.11, F4.107.12, F5.2.9, F5.10.6, F5.11.2 and F6.12.12 still bind to B cells of cynomolgus monkeys in a high proportion even under the condition of low concentration of 1 nM, and have equivalent or better binding activity than positive antibody 9G8; and other antibodies have no binding or relatively weak binding to cynomolgus monkey CD19.

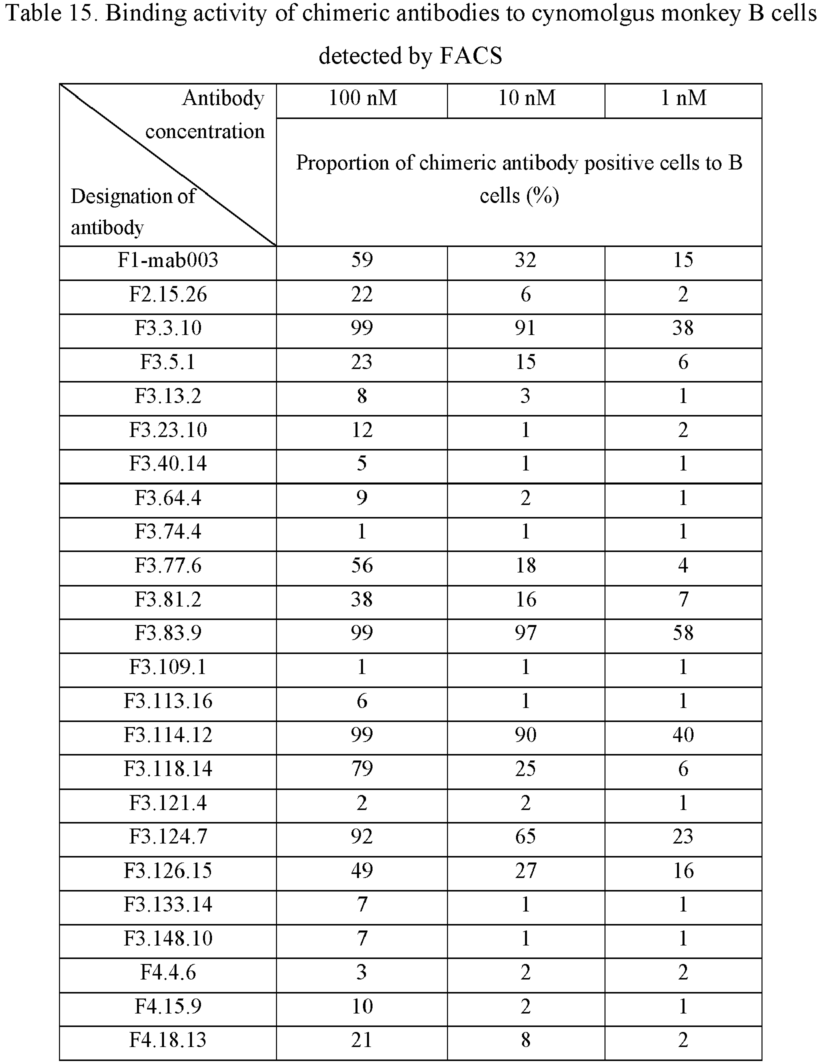

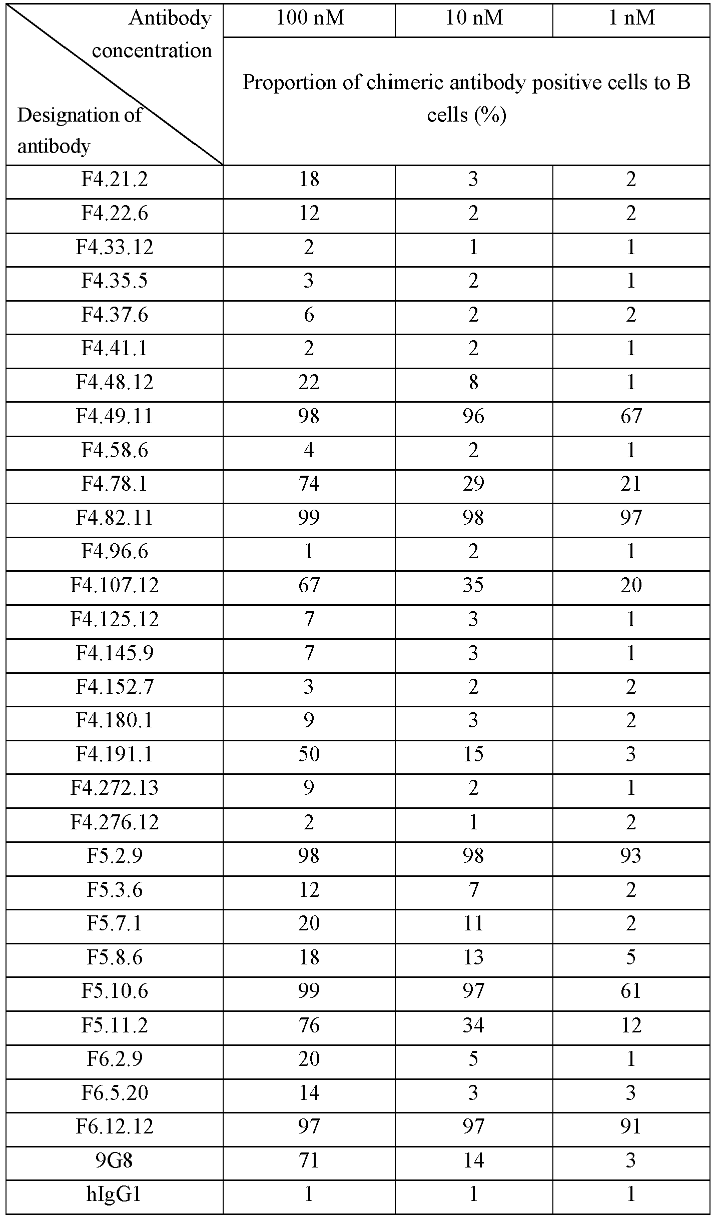

### Example 7 Affinity assay of anti-CD19 antibody

### 7.1 Assay of affinity of chimeric antibodies to human CD19-His protein

Anti-human CD19 antibodies were captured using Protein A chip (GE Helthcare; 29-127-558). A sample buffer and a running buffer were HBS-EP+(10 mM HEPES, 150 mM NaCl, 3 mM EDTA and 0.05% surfactant P20) (GE Healthcare; BR-1006-69). The flow-through cell was set to 25°C. The sample block was set to 16°C. Both were pretreated with the running buffer. In each cycle, an antibody to be detected was first captured with a Protein A chip, and then a single concentration of CD19 antigen protein was injected. The binding and dissociation processes of the antibody and the antigen protein were recorded, and finally Glycine pH 1.5 (GE Healthcare; BR-1003-54) was used to complete chip regeneration. The binding was measured by injecting different concentrations of recombinant human CD19-His proteins in a solution for 240 s with a flow rate of 30 µl/min. The concentration started from 200 nM (see the detailed results for the actual concentration in the test) and was diluted at 1 : 1, making a total of 5 concentrations. The dissociation phase was monitored for up to 600 s and was triggered by switching from sample solution to running buffer. The surface was regenerated by washing with a 10 mM glycine solution (pH 1.5) for 30 s at a flow rate of 30 µl/min. Bulk refractive index difference was corrected by subtracting the response obtained from the goat anti-human Fc surface. Blank injection was also subtracted (= double reference). For calculation of apparent KD and other kinetic parameters, Langmuir 1 : 1 model was used. The binding rate (Ka), dissociation rate (Kd) and binding affinity (KD) of the chimeric antibodies to human CD19-His protein are as shown in Table 16, with antibody FMC63 as a control.

**Table 16. Affinity of chimeric antibodies to human CD19 detected by SPR (biacore)**

| Designation of antibody | ka (1/Ms) | kd (1/s) | KD (M) |
|---|---|---|---|
| F1-mab003 | 4.50E+05 | 1.11E-04 | 2.47E-10 |
| F2.15.26 | 2.73E+05 | 2.29E-04 | 8.39E-10 |
| F3.3.10 | 7.34E+04 | 6.21E-04 | 8.47E-09 |
| F3.5.1 | 1.56E+05 | 8.47E-05 | 5.43E-10 |
| F3.13.2 | 6.47E+04 | 5.26E-04 | 8.13E-09 |
| F3.23.10 | 3.54E+04 | 1.86E-04 | 5.24E-09 |
| F3.40.14 | 8.43E+04 | 1.43E-04 | 1.70E-09 |
| F3.64.4 | 1.91E+05 | 3.13E-04 | 1.64E-09 |
| F3.74.4 | 4.71E+04 | 8.79E-04 | 1.87E-08 |
| F3.77.6 | 8.44E+04 | 1.69E-04 | 2.00E-09 |
| F3.81.2 | 1.35E+05 | 1.04E-04 | 7.66E-10 |
| F3.83.9 | 8.51E+04 | 3.12E-04 | 3.67E-09 |
| F3.109.1 | 1.90E+05 | 1.08E-03 | 5.67E-09 |
| F3.113.16 | 8.48E+04 | 1.05E-03 | 1.24E-08 |
| F3.114.12 | 7.70E+04 | 4.65E-04 | 6.03E-09 |
| F3.118.14 | 5.39E+04 | 3.26E-04 | 6.05E-09 |
| F3.121.4 | 3.27E+05 | 3.75E-04 | 1.15E-09 |
| F3.124.7 | 6.93E+04 | 1.38E-04 | 1.99E-09 |
| F3.126.15 | 6.13E+04 | 7.99E-04 | 1.30E-08 |
| F3.133.14 | 9.08E+04 | 8.84E-05 | 9.74E-10 |
| F3.148.10 | 2.24E+05 | 1.19E-03 | 5.32E-09 |
| F4.4.6 | 2.01E+05 | 1.90E-03 | 9.47E-09 |
| F4.15.9 | 3.48E+05 | 9.49E-04 | 2.72E-09 |
| F4.18.13 | 4.72E+05 | 1.71E-04 | 3.62E-10 |
| F4.21.2 | 1.85E+05 | 1.67E-04 | 9.04E-10 |
| F4.22.6 | 2.48E+05 | 1.47E-03 | 5.94E-09 |
| F4.33.12 | 1.31E+05 | 4.52E-04 | 3.45E-09 |
| F4.35.5 | 2.29E+05 | 3.51E-03 | 1.53E-08 |
| F4.37.6 | 3.59E+05 | 3.27E-03 | 9.11E-09 |
| F4.41.1 | 1.79E+05 | 2.39E-03 | 1.34E-08 |
| F4.48.12 | 2.64E+05 | 1.29E-04 | 4.87E-10 |
| F4.49.11 | 7.30E+04 | 1.28E-03 | 1.76E-08 |
| F4.58.6 | 1.43E+05 | 2.00E-03 | 1.39E-08 |
| F4.78.1 | 1.84E+05 | 1.70E-04 | 9.26E-10 |
| F4.82.11 | 3.41E+04 | 7.74E-04 | 2.27E-08 |
| F4.96.6 | 2.47E+05 | 9.18E-04 | 3.71E-09 |
| F4.107.12 | 2.00E+05 | 2.17E-04 | 1.09E-09 |
| F4.125.12 | 2.78E+05 | 2.04E-04 | 7.34E-10 |
| F4.145.9 | 1.61E+05 | 5.91E-04 | 3.68E-09 |
| F4.152.7 | 2.64E+05 | 8.90E-05 | 3.37E-10 |
| F4.180.1 | 2.37E+05 | 8.52E-05 | 3.59E-10 |
| F4.191.1 | 2.05E+05 | 3.88E-04 | 1.90E-09 |
| F4.272.13 | 2.21E+05 | 6.51E-05 | 2.94E-10 |
| F4.276.12 | 2.43E+05 | 8.10E-04 | 3.33E-09 |
| F5.2.9 | 2.57E+04 | 4.02E-04 | 1.57E-08 |
| F5.3.6 | 3.51E+04 | 1.22E-04 | 3.49E-09 |
| F5.7.1 | 1.92E+05 | 2.25E-04 | 1.18E-09 |
| F5.8.6 | 1.81E+05 | 1.08E-03 | 5.98E-09 |
| F5.10.6 | 8.21E+04 | 1.51E-04 | 1.84E-09 |
| F5.11.2 | 3.66E+04 | 6.15E-05 | 1.68E-09 |
| F6.2.9 | 1.52E+05 | 3.30E-03 | 2.17E-08 |
| F6.5.20 | 3.55E+05 | 1.87E-03 | 5.28E-09 |
| F6.12.12 | 1.67E+05 | 2.45E-04 | 1.46E-09 |
| FMC63 | 1.82E+05 | 3.33E-04 | 1.83E-09 |

### 7.2 Assay of affinity of chimeric antibodies to monkey CD19-His protein

According to the method in example 7.1, the affinity of the chimeric antibodies to monkey CD19 (ACROBiosystems, catalog number: 90051-C08H) protein was determined. The results are as shown in Table 17.

**Table 17. Affinity of chimeric antibodies to monkey CD19 detected by SPR (biacore)**

| Designation of antibody | ka (1/Ms) | kd (1/s) | KD (M) |
|---|---|---|---|
| F3.77.6 | 1.84E+04 | 1.40E-07 | 7.59E-12 |
| F3.118.14 | 1.67E+04 | 9.72E-08 | 5.83E-12 |
| F3.124.7 | 7.00E+04 | 5.62E-05 | 8.03E-10 |
| NB151-89 | 2.82E+04 | 6.55E-06 | 2.33E-10 |

### Example 8 Identification of antigen binding regions of chimeric antibodies

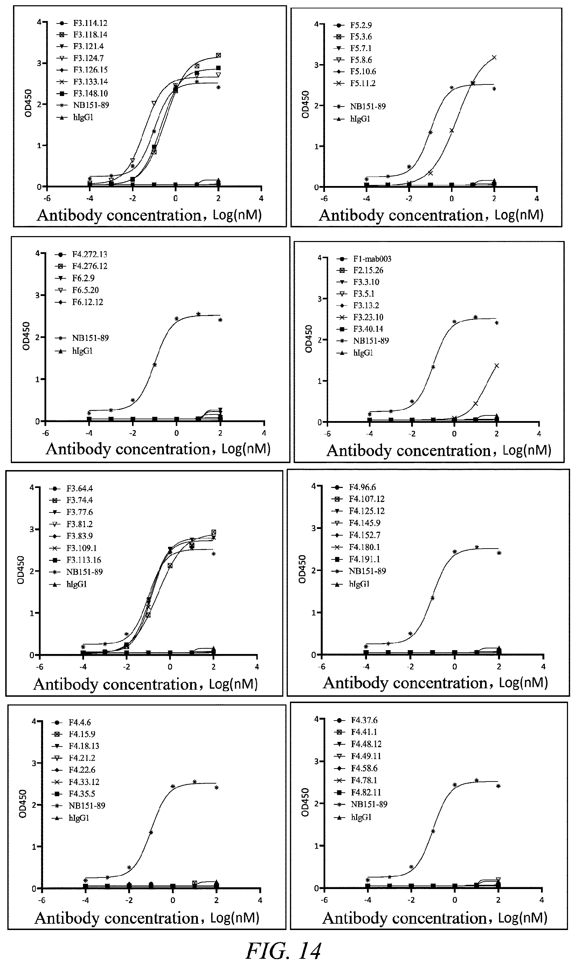

CD19 protein contains 4 exons in the extracellular region, in which exons 1-3 are located at the membrane-distal end and exon 4 is located at the membrane-proximal end. The antigen binding epitope of FMC63 is the membrane-proximal end (exon 4) of the extracellular region of CD19 protein. To identify the distribution of antigen binding epitopes of the chimeric antibodies, human CD19 exon 1-3-His (membrane-distal end) was used to coat at 2 µg/mL according to the ELISA method in example 5.1. The results are as shown in FIG. 14 and Table 18, indicating that except for F3.23.10, F3.74.4, F3.77.6, F3.109.1, F3.118.14, F3.124.7, F3.148.10 and F5.11.2, other antibodies have no binding activity to human CD19 exon 1-3-His. As shown in example 5.1, except for F3.3.10, F3.13.2, F3.113.16 and F6.2.9 that have weak binding to human CD19-His, other chimeric antibodies have good binding activity to human CD19-His. The antibodies can be divided into two categories: the first category does not bind to human CD19 exon 1-3-His, but has binding activity to human CD19-His, and the binding epitope is located at the non-membrane-distal end, such as F1-mab003; and the second category has binding activity to human CD19 exon 1-3-His, and has good binding activity to human CD19-His, and the binding epitope is located at the membrane-distal end, such as F3.23.10.

**Table 18. Classification of chimeric antibodies by ELISA method according to epitopes**

| Designation of antibody | Binding region | |
|---|---|---|
| | CD19 exon 1-3 | CD19 full-length protein |
| F1-mab003 | - | + |
| F2.15.26 | - | + |
| F3.3.10 | - | Weak binding |
| F3.5.1 | - | + |
| F3.13.2 | - | Weak binding |
| F3.23.10 | + | + |
| F3.40.14 | - | + |
| F3.64.4 | - | + |
| F3.74.4 | + | + |
| F3.77.6 | + | + |
| F3.81.2 | - | + |
| F3.83.9 | - | + |
| F3.109.1 | + | + |
| F3.113.16 | - | Weak binding |
| F3.114.12 | - | + |
| F3.118.14 | + | + |
| F3.121.4 | - | + |
| F3.124.7 | + | + |
| F3.126.15 | - | + |
| F3.133.14 | - | + |
| F3.148.10 | + | + |
| F4.4.6 | - | + |
| F4.15.9 | - | + |
| F4.18.13 | - | + |
| F4.21.2 | - | + |
| F4.22.6 | - | + |
| F4.33.12 | - | + |
| F4.35.5 | - | + |
| F4.37.6 | - | + |
| F4.41.1 | - | + |
| F4.48.12 | - | + |
| F4.49.11 | - | + |
| F4.58.6 | - | + |
| F4.78.1 | - | + |
| F4.82.11 | - | + |
| F4.96.6 | - | + |
| F4.107.12 | - | + |
| F4.125.12 | - | + |
| F4.145.9 | - | + |
| F4.152.7 | - | + |
| F4.180.1 | - | + |
| F4.191.1 | - | + |
| F4.272.13 | - | + |
| F4.276.12 | - | + |
| F5.2.9 | - | + |
| F5.3.6 | - | + |
| F5.7.1 | - | + |
| F5.8.6 | - | + |
| F5.10.6 | - | + |
| F5.11.2 | + | + |
| F6.2.9 | - | Weak binding |
| F6.5.20 | - | + |
| F6.12.12 | - | + |
| FMC63 | - | + |
| NB151-89 | + | + |
| hIgG1 | - | - |

### Example 9 Humanization of anti-human CD19 chimeric antibodies

### 9.1 Humanized design of chimeric antibodies

By aligning the IMGT (http://imgt.cines.fr) human antibody heavy and light chain variable region germline gene database, heavy chain and light chain variable region germline genes having high homology to the murine antibody were selected as templates, respectively, and the CDRs of the murine antibody were grafted into the corresponding human template, to form a variable region sequence in the order of FR1-CDR1-FR2-CDR2-FR3-CDR3-FR4. According to needs, the key amino acids in the framework sequence were back-mutated to the corresponding amino acids of the murine antibody to ensure the original affinity, and thus the humanized monoclonal antibody was obtained. The CDR amino acid residues of the antibody were determined and annotated by the Kabat numbering system.

### 9.1.1 Humanization of F3.121.4

The humanization light chain templates of the murine antibody F3.121.4 were IGKV6-21*01/IGKV3-11*01 and IGKJ2*01, and the humanization heavy chain templates were IGHV1-3*01 and IGHJ6*01. The CDRs of the murine antibody F3.121.4 were grafted into the human templates of the murine antibody respectively, and thus the corresponding humanized versions were obtained. According to needs, the key amino acids in the FR region sequence of the humanized antibody of F3.121.4 were back-mutated to the corresponding amino acids of the murine antibody to ensure the original affinity. If there are sites prone to chemical modification in the antibody, these sites are mutated to eliminate the risk of modification. See Table 19 for specific back mutation design.

**Table 19 Back mutation design of humanized antibody of F3.121.4**

| **VL** | | **VH** | |
|---|---|---|---|
| L1 | Graft (IGKV3-11*01) + L46F | H1 | Graft (IGHV1-3 *01) + R71V,T73K |
| L2 | Graft (IGKV6-21*01) | H2 | Graft (IGHV1-3*01) + R44G,R71V,T73K |
| L3 | Graft (IGKV6-21*01) + L46F,K49Y | H3 | Graft (IGHV1-3*01) + A40R,R44G,R71V,T73K |
| | | H4 | Graft (IGHV1-3 *01) + V5Q,A40R,R44G,R71V,T73K |

Notes: Graft represents the grafting of the CDRs of the murine antibody into the FR region sequence of the human germline template; L46F represents the mutation of L at position 47 of Graft into F, and so on. The numbering of back-mutated amino acids is according to the Kabat numbering.

The specific sequence of the variable region of the humanized antibody of F3.121.4 is as follows, where the underlined line represents a CDR region determined by the Kabat numbering system and the boxed character is a back mutation:

EIVLTQSPATLSLSPGERATLSCSASSSISSNYLHWYQQKPGQAPRFLIYRTSNLASGIPARFSGSGSGTDFT
LTISSLEPEDFAVYYCQQASSIPRMFTFGQGTKLEIK (F3.121.4.VL1, SEQ ID NO: 436).

EIVLTQSPDFQSVTPKEKVTITCSASSSISSNYLHWYQQKPDQSPKLLIKRTSNLASGVPSRFSGSGSGTDF
TLTINSLEAEDAATYYCQQASSIPRMFTFGQGTKLEIK (F3.121.4.VL2, SEQ ID NO: 437).

EIVLTQSPDFQSVTPKEKVTITCSASSSISSNYLHWYQQKPDQSPKFLIYRTSNLASGVPSRFSGSGSGTDF
TLTINSLEAEDAATYYCQQASSIPRMFTFGQGTKLEIK (F3.121.4.VL3, SEQ ID NO: 438).

EVQLVQSGAEVKKPGASVKVSCKASGYTFTNYWMHWVRQAPGQRLEWMGEIDPSDNYANYNQEFQG
RVTITVDKSASTAYMELSSLRSEDTAVYYCARHDGYFGALDYWGQGTTVTVSS (F3.121.4.VH1, SEQ ID
NO: 439).

EVQLVQSGAEVKKPGASVKVSCKASGYTFTNYWMHWVRQAPGQGLEWMGEIDPSDNYANYNQEFQG
RVTITVDKSASTAYMELSSLRSEDTAVYYCARHDGYFGALDYWGQGTTVTVSS (F3.121.4.VH2, SEQ ID
NO: 440).

EVQLVQSGAEVKKPGASVKVSCKASGYTFTNYWMHWVRQRPGQGLEWMGEIDPSDNYANYNQEFQG
RVTITVDKSASTAYMELSSLRSEDTAVYYCARHDGYFGALDYWGQGTTVTVSS (F3.121.4.VH3, SEQ ID
NO: 441).

EVQLQQSGAEVKKPGASVKVSCKASGYTFTNYWMHWVRQRPGQGLEWMGEIDPSDNYANYNQEFQ
GRVTITVDKSASTAYMELSSLRSEDTAVYYCARHDGYFGALDYWGQGTTVTVSS (F3.121.4.VH4, SEQ ID
NO: 442).

The sequences of the humanization templates are as follows: 
EIVLTQSPDFQSVTPKEKVTITCRASQSIGSSLHWYQQKPDQSPKLLIKYASQSFSGVPSRFSGSGSGTDFT
LTINSLEAEDAATYYCHQSSSLP (IGKV6-21*01, SEQ ID NO: 443).
 
EIVLTQSPATLSLSPGERATLSCRASQSVSSYLAWYQQKPGQAPRLLIYDASNRATGIPARFSGSGSGTDFT
LTISSLEPEDFAVYYCQQRSNWP (IGKV3-11*01, SEQ ID NO: 444).
 FGQGTKLEIK (IGKJ2*01, SEQ ID NO: 445). 
QVQLVQSGAEVKKPGASVKVSCKASGYTFTSYAMHWVRQAPGQRLEWMGWINAGNGNTKYSQKFQG
RVTITRDTSASTAYMELSSLRSEDTAVYYCAR (IGHV1-3*01, SEQ ID NO: 446).
 WGQGTTVTVSS (IGHJ6*01, SEQ ID NO: 447).

In the present disclosure, different light chain and heavy chain sequences were selected from the above-mentioned back mutation design of the light chain and heavy chain variable regions of the humanized antibody of F3.121.4 for cross combination, respectively, and finally a plurality of humanized antibodies of F3.121.4 were obtained, which were detailed in Table 20.

**Table 20 Combination of variable regions of humanized antibody of F3.121.4**

| Fv | F3.121.4.VH1 | F3.121.4.VH2 | F3.121.4.VH3 | F3.121.4.VH4 |
|---|---|---|---|---|
| F3.121.4.VL1 | F3.121.4-L1H1 | F3.121.4-L1H2 | F3.121.4-L1H3 | F3.121.4-L1H4 |
| F3.121.4.VL2 | F3.121.4-L2H1 | F3.121.4-L2H2 | F3.121.4-L2H3 | F3.121.4-L2H4 |
| F3.121.4.VL3 | F3.121.4-L3H1 | F3.121.4-L3H2 | F3.121.4-L3H3 | F3.121.4-L3H4 |

### 9.2 Preparation of humanized antibodies of F3.121.4

Biointron (Jiangsu) Biological Inc. was entrusted to clone the heavy chain variable region sequence of the humanized antibody of F3.121.4 into expression vector pcDNA3.4-B1HH1 containing a signal peptide and the heavy chain constant region of murine antibody IgG1 (the sequence of the heavy chain constant region is as shown in SEQ ID NO: 448), and clone the light chain variable region sequence into expression vector pcDNA3.4-B1BLK containing a signal peptide and the light chain constant region of the murine antibody IgG1 (the sequence of the light chain constant region is as shown in SEQ ID NO: 449), and thus the expression vector of the humanized antibody of F3.121.4 was obtained. The humanized antibody was prepared according to the method as shown in example 1.2. The sequences of the heavy chain and light chain constant regions of the antibody are as follows:

AKTTPPSVYPLAPGSAAQTNSMVTLGCLVKGYFPEPVTVTWNSGSLSSGVHTFPAVLQSDLYTLSSSVT
VPSSTWPSETVTCNVAHPASSTKVDKKIVPRDCGCKPCICTVPEVSSVFIFPPKPKDVLTITLTPKVTCVVVDIS
KDDPEVQFSWFVDDVEVHTAQTQPREEQFNSTFRSVSELPIMHQDWLNGKEFKCRVNSAAFPAPIEKTISKTK
GRPKAPQVYTIPPPKEQMAKDKVSLTCMITDFFPEDITVEWQWNGQPAENYKNTQPIMDTDGSYFVYSKLN
VQKSNWEAGNTFTCSVLHEGLHNHHTEKSLSHSPGK (SEQ ID NO: 448).

RADAAPTVSIFPPSSEQLTSGGASVVCFLNNFYPKDINVKWKIDGSERQNGVLNSWTDQDSKDSTYSMS
STLTLTKDEYERHNSYTCEATHKTSTSPIVKSFNRNEC (SEQ ID NO: 449).

### Example 10 Identification of humanized antibodies of F3.121.4

### 10.1 The binding of humanized antibodies to CD19 protein detected by enzyme-linked immunosorbent assay (ELISA)

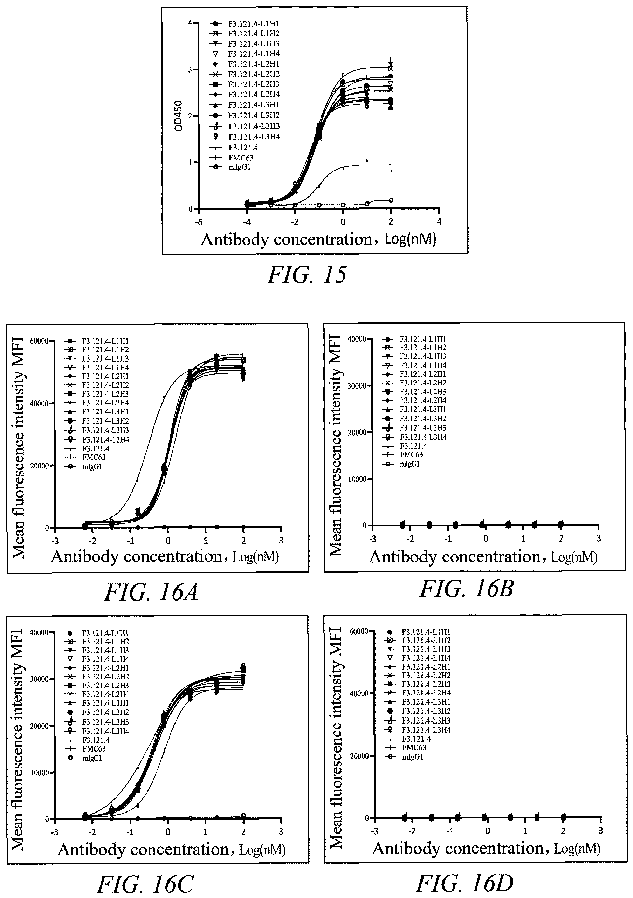

ELISA detection and data analysis were performed according to the method in example 5.1 (the secondary antibody used was purchased from Jackson Immuno, catalog number: 115-035-003). The analysis results are as shown in Table 21 and FIG. 15, indicating that the humanized antibodies of F3.121.4 all can bind to human CD19 at ELISA level. Unless particularly stated, the control antibody mIgG1 used in the identification or detection of humanized antibodies in examples 10-13 was antibody anti-hel-mIgG1 (purchased from Biointron, catalog number: B118301) against hen egg lysozyme, the FMC63 antibody was FMC63-mIgG1 prepared in example 1.2, and the 9G8 was 9G8-mIgG1 prepared in example 1.2.

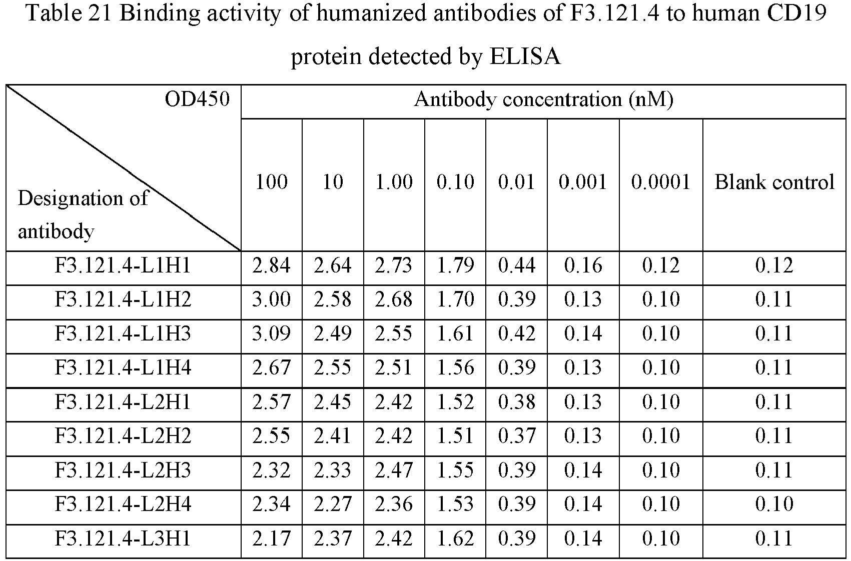

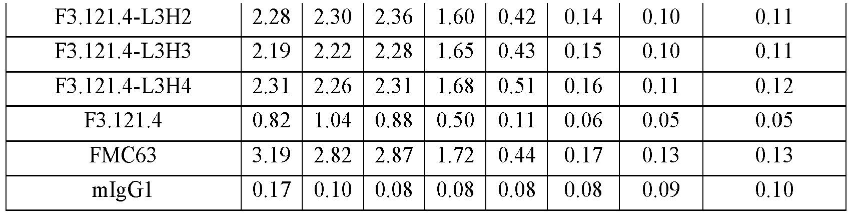

### 10.2 The binding of humanized antibodies to different CD19 expressing cells detected by fluorescence-activated cell sorting (FACS)

FACS detection and data analysis were performed according to the method in example 5.2 (the secondary antibody used was purchased from Jackson Immuno, catalog number: 115-605-003). The analysis results are as shown in Table 22 and FIGs. 16A-16D. The results in Table 22 indicate that the humanized antibodies of F3.121.4 all can bind to human CD19 protein on the surface ofRaji cells and CHO-K1-human CD19 cells (2C8) (Table 22, and FIGs. 16A and 16C). The same method was used to detect the binding of the humanized antibodies of F3.121.4 to CHO-K1 cells and endogenous CD19-negative cells, MOLT-4 cells (purchased from ATCC, CRL-1582). The results are as shown in FIGs. 16B and 16D, indicating that all humanized antibodies of F3.121.4 do not bind to MoLT4 cells and CHO-K1 cells, and have good specificity.

**Table 22 Binding activity of humanized antibodies of F3.121.4 to Raji cells and CHO-K1-human CD19 cells detected by FACS**

| Designation of antibody | Raji | | CHO-K1-human CD19 | |
|---|---|---|---|---|
| | Maximum mean fluorescence intensity | Ec50 (nM) | Maximum mean fluorescence intensity | Ec50 (nM) |
| F3.121.4-L1H1 | 30482 | 0.43 | 51573 | 1.18 |
| F3.121.4-L1H2 | 31691 | 0.43 | 54215 | 1.23 |
| F3.121.4-L1H3 | 31994 | 0.46 | 54364 | 1.10 |
| F3.121.4-L1H4 | 31574 | 0.44 | 54655 | 1.19 |
| F3.121.4-L2H1 | 29125 | 0.38 | 53162 | 1.08 |
| F3.121.4-L2H2 | 30122 | 0.41 | 52206 | 1.14 |
| F3.121.4-L2H3 | 29557 | 0.45 | 52161 | 1.17 |
| F3.121.4-L2H4 | 29242 | 0.37 | 51985 | 1.13 |
| F3.121.4-L3H1 | 32010 | 0.38 | 53467 | 1.13 |
| F3.121.4-L3H2 | 32206 | 0.42 | 52103 | 1.08 |
| F3.121.4-L3H3 | 32225 | 0.45 | 51804 | 1.16 |
| F3.121.4-L3H4 | 32465 | 0.43 | 50473 | 1.05 |
| F3.121.4 | 32899 | 0.31 | 53703 | 0.30 |
| FMC63 | 28813 | 0.78 | 55117 | 1.60 |
| mIgG1 | 651 | Negative | 82 | Negative |

### Example 11 Detection of species cross-binding activity

ELISA detection and data analysis were performed according to the method in example 6.1. The analysis results show that the humanized antibodies of F3.121.4 do not bind to murine CD 19 protein and monkey CD 19 protein at ELISA level.

FACS detection and data analysis were performed according to the method in example 6.2. The analysis results show that the humanized antibodies of F3.121.4 do not bind to 293T-monkey CD19 cells.

### Example 12 Affinity assay of humanized antibodies of F3.121.4

SPR (biacore) detection and data analysis were performed according to the method in example 7.1. The results are as shown in Table 23, indicating that the affinities of the humanized antibodies of F3.121.4 to human CD19 are all above 1E-08M.

**Table 23. Affinity of humanized antibodies of F3.121.4 to human CD19 detected by SPR (biacore)**

| Designation of antibody | ka (1/Ms) | kd (1/s) | KD (M) |
|---|---|---|---|
| F3.121.4-L1H1 | 1.21E+05 | 3.45E-06 | 2.84E-11 |
| F3.121.4-L1H2 | 1.18E+05 | 6.84E-08 | 5.80E-13 |
| F3.121.4-L1H3 | 1.23E+05 | 6.17E-07 | 5.02E-12 |
| F3.121.4-L1H4 | 9.11E+04 | 1.86E-08 | 2.05E-13 |
| F3.121.4-L2H1 | 1.30E+05 | 1.86E-04 | 1.43E-09 |
| F3.121.4-L2H2 | 1.34E+05 | 1.95E-04 | 1.46E-09 |
| F3.121.4-L2H3 | 1.37E+05 | 1.88E-04 | 1.38E-09 |
| F3.121.4-L2H4 | 1.22E+05 | 1.86E-04 | 1.53E-09 |
| F3.121.4-L3H1 | 1.30E+05 | 3.18E-05 | 2.44E-10 |
| F3.121.4-L3H2 | 1.13E+05 | 5.65E-09 | 4.98E-14 |
| F3.121.4-L3H3 | 1.18E+05 | 1.04E-06 | 8.80E-12 |
| F3.121.4-L3H4 | 1.12E+05 | 2.51E-05 | 2.25E-10 |
| F3.121.4 | 3.27E+05 | 3.75E-04 | 1.15E-09 |
| FMC63 | 1.82E+05 | 3.33E-04 | 1.83E-09 |

### Example 13 Identification of antigen binding regions of humanized antibodies of F3.121.4

To identify the distribution of antigen binding epitopes of the humanized antibodies of F3.121.4, human CD19 exon 1-3-His (membrane-distal end) was used to coat at 2 µg/mL according to the ELISA method in example 5.1. The results are as shown in Table 24, indicating that none of the humanized antibodies of F3.121.4 has binding activity to human CD19 exon 1-3-His protein. Therefore, it can be judged that the binding epitopes of the humanized antibody of F3.121.4 and the chimeric antibody F3.121.4 are both located at the non-membrane-distal end.

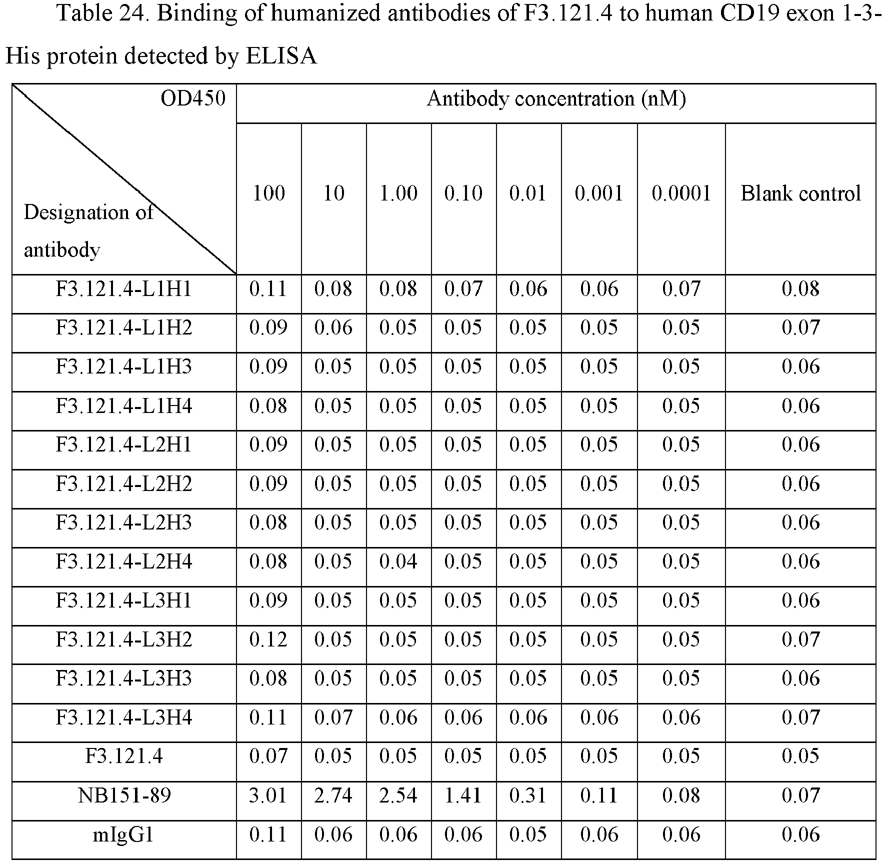

1. An antibody or antigen binding fragment specifically binding to human CD19, wherein the antibody or antigen binding fragment comprises a heavy chain variable region and/or a light chain variable region, the heavy chain variable region comprises HCDR1-3, and the light chain variable region comprises LCDR1-3,
   the HCDR1 comprises a sequence as shown in any one of SEQ ID NOs: 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421 or 427, or a sequence having at least 70% identity or at most 3 mutations compared thereto;
   the HCDR2 comprises a sequence as shown in any one of SEQ ID NOs: 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422 or 428, or a sequence having at least 70% identity or at most 3 mutations compared thereto;
   the HCDR3 comprises a sequence as shown in any one of SEQ ID NOs: 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423 or 429, or a sequence having at least 70% identity or at most 3 mutations compared thereto;
   the LCDR1 comprises a sequence as shown in any one of SEQ ID NOs: 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424 or 430, or a sequence having at least 70% identity or at most 3 mutations compared thereto;
   the LCDR2 comprises a sequence as shown in any one of SEQ ID NOs: 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425 or 431, or a sequence having at least 70% identity or at most 3 mutations compared thereto;
   and the LCDR3 comprises a sequence as shown in any one of SEQ ID NOs: 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426 or 432, or a sequence having at least 70% identity or at most 3 mutations compared thereto.
2. The antibody or antigen binding fragment according to claim 1, wherein the HCDR1-3 comprise sequences as shown in SEQ ID NOs: 115-117, 121-123, 127-129, 133-135, 139-141, 145-147, 151-153, 157-159, 163-165, 169-171, 175-177, 181-183, 187-189, 193-195, 199-201, 205-207, 211-213, 217-219, 223-225, 229-231, 235-237, 241-243, 247-249, 253-255, 259-261, 265-267, 271-273, 277-279, 283-285, 289-291, 295-297, 301-303, 307-309, 313-315, 319-321, 325-327, 331-333, 337-339, 343-345, 349-351, 355-357, 361-363, 367-369, 373-375, 379-381, 385-387, 391-393, 397-399, 403-405, 409-411, 415-417, 421-423 or 427-429, or sequences having at least 70% identity or at most 3 mutations compared thereto; and/or
   the LCDR1-3 comprise sequences as shown in SEQ ID NOs: 118-120, 124-126, 130-132, 136-138, 142-144, 148-150, 154-156, 160-162, 166-168, 172-174, 178-180, 184-186, 190-192, 196-198, 202-204, 208-210, 214-216, 220-222, 226-228, 232-234, 238-240, 244-246, 250-252, 256-258, 262-264, 268-270, 274-276, 280-282, 286-288, 292-294, 298-300, 304-306, 310-312, 316-318, 322-324, 328-330, 334-336, 340-342, 346-348, 352-354, 358-360, 364-366, 370-372, 376-378, 382-384, 388-390, 394-396, 400-402, 406-408, 412-414, 418-420, 424-426 or 430-432, or sequences having at least 70% identity or at most 3 mutations compared thereto.
3. The antibody or antigen binding fragment according to claim 2, wherein the antibody or antigen binding fragment comprises the heavy chain variable region and the light chain variable region, and the HCDR1-3 and the LCDR1-3 comprise sequences selected from the group consisting of:
   (1) sequences of SEQ ID NOs: 115-120, or sequences having at least 70% identity or at most 3 mutations compared thereto;
   (2) sequences of SEQ ID NOs: 121-126, or sequences having at least 70% identity or at most 3 mutations compared thereto;
   (3) sequences of SEQ ID NOs: 127-132, or sequences having at least 70% identity or at most 3 mutations compared thereto;
   (4) sequences of SEQ ID NOs: 133-138, or sequences having at least 70% identity or at most 3 mutations compared thereto;
   (5) sequences of SEQ ID NOs: 139-144, or sequences having at least 70% identity or at most 3 mutations compared thereto;
   (6) sequences of SEQ ID NOs: 145-150, or sequences having at least 70% identity or at most 3 mutations compared thereto;
   (7) sequences of SEQ ID NOs: 151-156, or sequences having at least 70% identity or at most 3 mutations compared thereto;
   (8) sequences of SEQ ID NOs: 157-162, or sequences having at least 70% identity or at most 3 mutations compared thereto;
   (9) sequences of SEQ ID NOs: 163-168, or sequences having at least 70% identity or at most 3 mutations compared thereto;
   (10) sequences of SEQ ID NOs: 169-174, or sequences having at least 70% identity or at most 3 mutations compared thereto;
   (11) sequences of SEQ ID NOs: 175-180, or sequences having at least 70% identity or at most 3 mutations compared thereto;
   (12) sequences of SEQ ID NOs: 181-186, or sequences having at least 70% identity or at most 3 mutations compared thereto;
   (13) sequences of SEQ ID NOs: 187-192, or sequences having at least 70% identity or at most 3 mutations compared thereto;
   (14) sequences of SEQ ID NOs: 193-198, or sequences having at least 70% identity or at most 3 mutations compared thereto;
   (15) sequences of SEQ ID NOs: 199-204, or sequences having at least 70% identity or at most 3 mutations compared thereto;
   (16) sequences of SEQ ID NOs: 205-210, or sequences having at least 70% identity or at most 3 mutations compared thereto;
   (17) sequences of SEQ ID NOs: 211-216, or sequences having at least 70% identity or at most 3 mutations compared thereto;
   (18) sequences of SEQ ID NOs: 217-222, or sequences having at least 70% identity or at most 3 mutations compared thereto;
   (19) sequences of SEQ ID NOs: 223-228, or sequences having at least 70% identity or at most 3 mutations compared thereto;
   (20) sequences of SEQ ID NOs: 229-234, or sequences having at least 70% identity or at most 3 mutations compared thereto;
   (21) sequences of SEQ ID NOs: 235-240, or sequences having at least 70% identity or at most 3 mutations compared thereto;
   (22) sequences of SEQ ID NOs: 241-246, or sequences having at least 70% identity or at most 3 mutations compared thereto;
   (23) sequences of SEQ ID NOs: 247-252, or sequences having at least 70% identity or at most 3 mutations compared thereto;
   (24) sequences of SEQ ID NOs: 253-258, or sequences having at least 70% identity or at most 3 mutations compared thereto;
   (25) sequences of SEQ ID NOs: 259-264, or sequences having at least 70% identity or at most 3 mutations compared thereto;
   (26) sequences of SEQ ID NOs: 265-270, or sequences having at least 70% identity or at most 3 mutations compared thereto;
   (27) sequences of SEQ ID NOs: 271-276, or sequences having at least 70% identity or at most 3 mutations compared thereto;
   (28) sequences of SEQ ID NOs: 277-282, or sequences having at least 70% identity or at most 3 mutations compared thereto;
   (29) sequences of SEQ ID NOs: 283-288, or sequences having at least 70% identity or at most 3 mutations compared thereto;
   (30) sequences of SEQ ID NOs: 289-294, or sequences having at least 70% identity or at most 3 mutations compared thereto;
   (31) sequences of SEQ ID NOs: 295-300, or sequences having at least 70% identity or at most 3 mutations compared thereto;
   (32) sequences of SEQ ID NOs: 301-306, or sequences having at least 70% identity or at most 3 mutations compared thereto;
   (33) sequences of SEQ ID NOs: 307-312, or sequences having at least 70% identity or at most 3 mutations compared thereto;
   (34) sequences of SEQ ID NOs: 313-318, or sequences having at least 70% identity or at most 3 mutations compared thereto;
   (35) sequences of SEQ ID NOs: 319-324, or sequences having at least 70% identity or at most 3 mutations compared thereto;
   (36) sequences of SEQ ID NOs: 325-330, or sequences having at least 70% identity or at most 3 mutations compared thereto;
   (37) sequences of SEQ ID NOs: 331-336, or sequences having at least 70% identity or at most 3 mutations compared thereto;
   (38) sequences of SEQ ID NOs: 337-342, or sequences having at least 70% identity or at most 3 mutations compared thereto;
   (39) sequences of SEQ ID NOs: 343-348, or sequences having at least 70% identity or at most 3 mutations compared thereto;
   (40) sequences of SEQ ID NOs: 349-354, or sequences having at least 70% identity or at most 3 mutations compared thereto;
   (41) sequences of SEQ ID NOs: 355-360, or sequences having at least 70% identity or at most 3 mutations compared thereto;
   (42) sequences of SEQ ID NOs: 361-366, or sequences having at least 70% identity or at most 3 mutations compared thereto;
   (43) sequences of SEQ ID NOs: 367-372, or sequences having at least 70% identity or at most 3 mutations compared thereto;
   (44) sequences of SEQ ID NOs: 373-378, or sequences having at least 70% identity or at most 3 mutations compared thereto;
   (45) sequences of SEQ ID NOs: 379-384, or sequences having at least 70% identity or at most 3 mutations compared thereto;
   (46) sequences of SEQ ID NOs: 385-390, or sequences having at least 70% identity or at most 3 mutations compared thereto;
   (47) sequences of SEQ ID NOs: 391-396, or sequences having at least 70% identity or at most 3 mutations compared thereto;
   (48) sequences of SEQ ID NOs: 397-402, or sequences having at least 70% identity or at most 3 mutations compared thereto;
   (49) sequences of SEQ ID NOs: 403-408, or sequences having at least 70% identity or at most 3 mutations compared thereto;
   (50) sequences of SEQ ID NOs: 409-414, or sequences having at least 70% identity or at most 3 mutations compared thereto;
   (51) sequences of SEQ ID NOs: 415-420, or sequences having at least 70% identity or at most 3 mutations compared thereto;
   (52) sequences of SEQ ID NOs: 421-426, or sequences having at least 70% identity or at most 3 mutations compared thereto;
   (53) sequences of SEQ ID NOs: 427-432, or sequences having at least 70% identity or at most 3 mutations compared thereto.
4. The antibody or antigen binding fragment according to any one of claims 1-3, wherein the heavy chain variable region comprises a sequence as shown in any one of SEQ ID NOs: 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 439-442, or a sequence having at least 70% identity or at most 15 amino acid differences compared thereto;
   and the light chain variable region comprises a sequence as shown in any one of SEQ ID NOs: 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 436, 437 or 438, or a sequence having at least 70% identity or at most 15 amino acid differences compared thereto.
5. The antibody or antigen binding fragment according to any one of claims 1-4, wherein the at least 70% identity is preferably at least 75%, 80%, 85%, 90%, 91%, 92%, 93%, 94%, 95%, 96%, 97%, 98%, 99% or 100% identity; the at most 3 mutations are preferably at most 3, 2, 1 or 0 mutations; the at most 15 mutations are preferably at most 15, 14, 13, 12, 11, 10, 9, 8, 7, 6, 5, 4, 3, 2, 1 or 0 mutations; and preferably, the mutation is an insertion, a deletion or a substitution, the substitution is preferably a conservative amino acid substitution, and the mutation is preferably a back mutation or a hotspot mutation.
6. The antibody or antigen binding fragment according to any one of claims 1-5, wherein the antibody or antigen binding fragment further comprises a heavy chain constant region and/or a light chain constant region;
   preferably, the heavy chain constant region is selected from IgG, such as IgG1, IgG2, IgG3 or IgG4; the IgG can be selected from human IgG, such as human IgG1 or human IgG4; the light chain constant region is selected from a κ chain or a λ chain, preferably a κ chain;
   preferably, the heavy chain constant region can be selected from SEQ ID NO: 433 or 448;
   and preferably, the light chain constant region can be selected from SEQ ID NOs: 434-435 or 449.
7. The antibody or antigen binding fragment according to any one of claims 1-6, wherein the antibody or antigen binding fragment is selected from a monoclonal antibody, a polyclonal antibody, a natural antibody, an engineered antibody, a monospecific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, a fully human antibody, Fab, Fab', Fab'-SH, F(ab')₂, Fd, Fv, scFv, a diabody or a single domain antibody.
8. The antibody or antigen binding fragment according to any one of claims 1-7, wherein the antibody or antigen binding fragment further comprise a conjugate; and the conjugate can be selected from a therapeutic agent or a tracer, the therapeutic agent can be selected from a radioisotope, a chemotherapeutic drug or an immunomodulator, and the tracer can be selected from a radiological contrast agent, a paramagnetic ion, a metal, a fluorescent tag, a chemiluminescence tag, an ultrasonic contrast agent and a photosensitizer.
9. The antibody or antigen binding fragment according to any one of claims 1-8, wherein the antibody or antigen binding fragment binds to human CD19 with a KD value of less than 1 E-08M, 1E-09M, 1E-10M or 1E-11M; preferably, the antibody or antigen binding fragment also binds to monkey CD19, more preferably, the antibody or antigen binding fragment binds to monkey CD19 with a KD value of less than 1E-8M, 1E-9M, 1E-10M, 1E-11M or 1E-12M.
10. A multi-specific antigen binding molecule, wherein the multi-specific antigen binding molecule comprises at least a first antigen binding module and a second antigen binding module, the first antigen binding module comprises the antibody or antigen binding fragment according to any one of claims 1-10, and the second antigen binding module binds to other targets different from the first antigen binding module or binds to different epitopes of the same target; preferably, the second antigen binding module is an antibody or an antigen binding fragment;
   and preferably, the other targets are selected from the group of: (1) a tumor specific antigen (TSA) or a tumor associated antigen (TAA); (2) an immune checkpoint; and (3) a target that recruits and/or activates immune cells.
11. A chimeric antigen receptor (CAR), wherein the chimeric antigen receptor comprises at least an extracellular antigen binding domain, a transmembrane domain and an intracellular signaling domain, and the extracellular antigen binding domain comprises the antibody or antigen binding fragment according to any one of claims 1-9 or the multi-specific antigen binding molecule according to claim 10.
12. An immune effector cell, wherein the immune effector cell expresses the chimeric antigen receptor according to claim 11 and/or a nucleic acid molecule encoding the chimeric antigen receptor according to claim 11;
   preferably, the immune effector cell is selected from a T cell, a natural killer cell (a NK cell), a natural killer T cell (an NKT cell), a monocyte, a macrophage, a dendritic cell or a mast cell, more preferably the T cell is selected from a cytotoxic T cell, a regulatory T cell or a helper T cell;
   and preferably, the immune effector cell is an autologous immune effector cell or an allogeneic immune effector cell.
13. An isolated nucleic acid molecule, wherein the nucleic acid molecule encodes the antibody or antigen binding fragment according to claims 1-9, the multi-specific antigen binding molecule according to claim 10 or the chimeric antigen receptor according to claim 11.
14. A vector, wherein the vector comprises the nucleic acid molecule according to claim 13.
15. A cell, wherein the cell comprises the vector according to claim 14.
16. A method for preparing the antibody or antigen binding fragment according to claims 1-9, or the multi-specific antigen binding molecule according to claim 10, wherein the method comprises: (1) culturing the cell according to claim 15, and/or (2) isolating the antibody or antigen binding fragment, or the multi-specific antigen binding molecule expressed by the cell.
17. A method for preparing the immune effector cell according to claim 12, wherein the method comprises introducing a nucleic acid molecule encoding the chimeric antigen receptor according to claim 11 into the immune effector cell, and/or initiating the immune effector cell to express the chimeric antigen receptor.
18. A pharmaceutical composition, wherein the pharmaceutical composition comprises the antibody or antigen binding molecule according to any one of claims 1-9, or the multi-specific antigen binding molecule according to claim 10, or the immune effector cell according to claim 12, or the nucleic acid molecule according to claim 13, or the vector according to claim 14, or the cell according to claim 15, or a product prepared according to the method according to claim 16 or 17; preferably, the composition further comprises a pharmaceutically acceptable carrier, diluent or auxiliary agent.
19. Use of the antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17 in the preparation of a drug for treating a CD19-related disease; preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.
20. A method for treating a CD19-related disease, wherein the method comprises administering to a subject an effective amount of a drug, and the drug comprises the antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.
21. The antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17, for use in a drug for treating a CD19-related disease, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

## Claims

1. An antibody or antigen binding fragment specifically binding to human CD19, wherein the antibody or antigen binding fragment comprises a heavy chain variable region and/or a light chain variable region, the heavy chain variable region comprises HCDR1-3, and the light chain variable region comprises LCDR1-3,
the HCDR1 comprises a sequence as shown in any one of SEQ ID NOs: 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421 or 427, or a sequence having at least 70% identity or at most 3 mutations compared thereto;
the HCDR2 comprises a sequence as shown in any one of SEQ ID NOs: 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422 or 428, or a sequence having at least 70% identity or at most 3 mutations compared thereto;
the HCDR3 comprises a sequence as shown in any one of SEQ ID NOs: 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423 or 429, or a sequence having at least 70% identity or at most 3 mutations compared thereto;
the LCDR1 comprises a sequence as shown in any one of SEQ ID NOs: 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424 or 430, or a sequence having at least 70% identity or at most 3 mutations compared thereto;
the LCDR2 comprises a sequence as shown in any one of SEQ ID NOs: 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425 or 431, or a sequence having at least 70% identity or at most 3 mutations compared thereto;
and the LCDR3 comprises a sequence as shown in any one of SEQ ID NOs: 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426 or 432, or a sequence having at least 70% identity or at most 3 mutations compared thereto.

2. The antibody or antigen binding fragment according to claim 1, wherein the HCDR1-3 comprise sequences as shown in SEQ ID NOs: 115-117, 121-123, 127-129, 133-135, 139-141, 145-147, 151-153, 157-159, 163-165, 169-171, 175-177, 181-183, 187-189, 193-195, 199-201, 205-207, 211-213, 217-219, 223-225, 229-231, 235-237, 241-243, 247-249, 253-255, 259-261, 265-267, 271-273, 277-279, 283-285, 289-291, 295-297, 301-303, 307-309, 313-315, 319-321, 325-327, 331-333, 337-339, 343-345, 349-351, 355-357, 361-363, 367-369, 373-375, 379-381, 385-387, 391-393, 397-399, 403-405, 409-411, 415-417, 421-423 or 427-429, or sequences having at least 70% identity or at most 3 mutations compared thereto; and/or
the LCDR1-3 comprise sequences as shown in SEQ ID NOs: 118-120, 124-126, 130-132, 136-138, 142-144, 148-150, 154-156, 160-162, 166-168, 172-174, 178-180, 184-186, 190-192, 196-198, 202-204, 208-210, 214-216, 220-222, 226-228, 232-234, 238-240, 244-246, 250-252, 256-258, 262-264, 268-270, 274-276, 280-282, 286-288, 292-294, 298-300, 304-306, 310-312, 316-318, 322-324, 328-330, 334-336, 340-342, 346-348, 352-354, 358-360, 364-366, 370-372, 376-378, 382-384, 388-390, 394-396, 400-402, 406-408, 412-414, 418-420, 424-426 or 430-432, or sequences having at least 70% identity or at most 3 mutations compared thereto.

3. The antibody or antigen binding fragment according to claim 2, wherein the antibody or antigen binding fragment comprises the heavy chain variable region and the light chain variable region, and the HCDR1-3 and the LCDR1-3 comprise sequences selected from the group consisting of:
(1) sequences of SEQ ID NOs: 115-120, or sequences having at least 70% identity or at most 3 mutations compared thereto;
(2) sequences of SEQ ID NOs: 121-126, or sequences having at least 70% identity or at most 3 mutations compared thereto;
(3) sequences of SEQ ID NOs: 127-132, or sequences having at least 70% identity or at most 3 mutations compared thereto;
(4) sequences of SEQ ID NOs: 133-138, or sequences having at least 70% identity or at most 3 mutations compared thereto;
(5) sequences of SEQ ID NOs: 139-144, or sequences having at least 70% identity or at most 3 mutations compared thereto;
(6) sequences of SEQ ID NOs: 145-150, or sequences having at least 70% identity or at most 3 mutations compared thereto;
(7) sequences of SEQ ID NOs: 151-156, or sequences having at least 70% identity or at most 3 mutations compared thereto;
(8) sequences of SEQ ID NOs: 157-162, or sequences having at least 70% identity or at most 3 mutations compared thereto;
(9) sequences of SEQ ID NOs: 163-168, or sequences having at least 70% identity or at most 3 mutations compared thereto;
(10) sequences of SEQ ID NOs: 169-174, or sequences having at least 70% identity or at most 3 mutations compared thereto;
(11) sequences of SEQ ID NOs: 175-180, or sequences having at least 70% identity or at most 3 mutations compared thereto;
(12) sequences of SEQ ID NOs: 181-186, or sequences having at least 70% identity or at most 3 mutations compared thereto;
(13) sequences of SEQ ID NOs: 187-192, or sequences having at least 70% identity or at most 3 mutations compared thereto;
(14) sequences of SEQ ID NOs: 193-198, or sequences having at least 70% identity or at most 3 mutations compared thereto;
(15) sequences of SEQ ID NOs: 199-204, or sequences having at least 70% identity or at most 3 mutations compared thereto;
(16) sequences of SEQ ID NOs: 205-210, or sequences having at least 70% identity or at most 3 mutations compared thereto;
(17) sequences of SEQ ID NOs: 211-216, or sequences having at least 70% identity or at most 3 mutations compared thereto;
(18) sequences of SEQ ID NOs: 217-222, or sequences having at least 70% identity or at most 3 mutations compared thereto;
(19) sequences of SEQ ID NOs: 223-228, or sequences having at least 70% identity or at most 3 mutations compared thereto;
(20) sequences of SEQ ID NOs: 229-234, or sequences having at least 70% identity or at most 3 mutations compared thereto;
(21) sequences of SEQ ID NOs: 235-240, or sequences having at least 70% identity or at most 3 mutations compared thereto;
(22) sequences of SEQ ID NOs: 241-246, or sequences having at least 70% identity or at most 3 mutations compared thereto;
(23) sequences of SEQ ID NOs: 247-252, or sequences having at least 70% identity or at most 3 mutations compared thereto;
(24) sequences of SEQ ID NOs: 253-258, or sequences having at least 70% identity or at most 3 mutations compared thereto;
(25) sequences of SEQ ID NOs: 259-264, or sequences having at least 70% identity or at most 3 mutations compared thereto;
(26) sequences of SEQ ID NOs: 265-270, or sequences having at least 70% identity or at most 3 mutations compared thereto;
(27) sequences of SEQ ID NOs: 271-276, or sequences having at least 70% identity or at most 3 mutations compared thereto;
(28) sequences of SEQ ID NOs: 277-282, or sequences having at least 70% identity or at most 3 mutations compared thereto;
(29) sequences of SEQ ID NOs: 283-288, or sequences having at least 70% identity or at most 3 mutations compared thereto;
(30) sequences of SEQ ID NOs: 289-294, or sequences having at least 70% identity or at most 3 mutations compared thereto;
(31) sequences of SEQ ID NOs: 295-300, or sequences having at least 70% identity or at most 3 mutations compared thereto;
(32) sequences of SEQ ID NOs: 301-306, or sequences having at least 70% identity or at most 3 mutations compared thereto;
(33) sequences of SEQ ID NOs: 307-312, or sequences having at least 70% identity or at most 3 mutations compared thereto;
(34) sequences of SEQ ID NOs: 313-318, or sequences having at least 70% identity or at most 3 mutations compared thereto;
(35) sequences of SEQ ID NOs: 319-324, or sequences having at least 70% identity or at most 3 mutations compared thereto;
(36) sequences of SEQ ID NOs: 325-330, or sequences having at least 70% identity or at most 3 mutations compared thereto;
(37) sequences of SEQ ID NOs: 331-336, or sequences having at least 70% identity or at most 3 mutations compared thereto;
(38) sequences of SEQ ID NOs: 337-342, or sequences having at least 70% identity or at most 3 mutations compared thereto;
(39) sequences of SEQ ID NOs: 343-348, or sequences having at least 70% identity or at most 3 mutations compared thereto;
(40) sequences of SEQ ID NOs: 349-354, or sequences having at least 70% identity or at most 3 mutations compared thereto;
(41) sequences of SEQ ID NOs: 355-360, or sequences having at least 70% identity or at most 3 mutations compared thereto;
(42) sequences of SEQ ID NOs: 361-366, or sequences having at least 70% identity or at most 3 mutations compared thereto;
(43) sequences of SEQ ID NOs: 367-372, or sequences having at least 70% identity or at most 3 mutations compared thereto;
(44) sequences of SEQ ID NOs: 373-378, or sequences having at least 70% identity or at most 3 mutations compared thereto;
(45) sequences of SEQ ID NOs: 379-384, or sequences having at least 70% identity or at most 3 mutations compared thereto;
(46) sequences of SEQ ID NOs: 385-390, or sequences having at least 70% identity or at most 3 mutations compared thereto;
(47) sequences of SEQ ID NOs: 391-396, or sequences having at least 70% identity or at most 3 mutations compared thereto;
(48) sequences of SEQ ID NOs: 397-402, or sequences having at least 70% identity or at most 3 mutations compared thereto;
(49) sequences of SEQ ID NOs: 403-408, or sequences having at least 70% identity or at most 3 mutations compared thereto;
(50) sequences of SEQ ID NOs: 409-414, or sequences having at least 70% identity or at most 3 mutations compared thereto;
(51) sequences of SEQ ID NOs: 415-420, or sequences having at least 70% identity or at most 3 mutations compared thereto;
(52) sequences of SEQ ID NOs: 421-426, or sequences having at least 70% identity or at most 3 mutations compared thereto;
(53) sequences of SEQ ID NOs: 427-432, or sequences having at least 70% identity or at most 3 mutations compared thereto.

4. The antibody or antigen binding fragment according to any one of claims 1-3, wherein the heavy chain variable region comprises a sequence as shown in any one of SEQ ID NOs: 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 439-442, or a sequence having at least 70% identity or at most 15 amino acid differences compared thereto;
and the light chain variable region comprises a sequence as shown in any one of SEQ ID NOs: 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 436, 437 or 438, or a sequence having at least 70% identity or at most 15 amino acid differences compared thereto.

5. The antibody or antigen binding fragment according to any one of claims 1-4, wherein the at least 70% identity is preferably at least 75%, 80%, 85%, 90%, 91%, 92%, 93%, 94%, 95%, 96%, 97%, 98%, 99% or 100% identity; the at most 3 mutations are preferably at most 3, 2, 1 or 0 mutations; the at most 15 mutations are preferably at most 15, 14, 13, 12, 11, 10, 9, 8, 7, 6, 5, 4, 3, 2, 1 or 0 mutations; and preferably, the mutation is an insertion, a deletion or a substitution, the substitution is preferably a conservative amino acid substitution, and the mutation is preferably a back mutation or a hotspot mutation.

6. The antibody or antigen binding fragment according to any one of claims 1-5, wherein the antibody or antigen binding fragment further comprises a heavy chain constant region and/or a light chain constant region;
preferably, the heavy chain constant region is selected from IgG, such as IgG1, IgG2, IgG3 or IgG4; the IgG can be selected from human IgG, such as human IgG1 or human IgG4; the light chain constant region is selected from a κ chain or a λ chain, preferably a κ chain;
preferably, the heavy chain constant region can be selected from SEQ ID NO: 433 or 448;
and preferably, the light chain constant region can be selected from SEQ ID NOs: 434-435 or 449.

7. The antibody or antigen binding fragment according to any one of claims 1-6, wherein the antibody or antigen binding fragment is selected from a monoclonal antibody, a polyclonal antibody, a natural antibody, an engineered antibody, a monospecific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, a fully human antibody, Fab, Fab', Fab'-SH, F(ab')₂, Fd, Fv, scFv, a diabody or a single domain antibody.

8. The antibody or antigen binding fragment according to any one of claims 1-7, wherein the antibody or antigen binding fragment further comprise a conjugate; and the conjugate can be selected from a therapeutic agent or a tracer, the therapeutic agent can be selected from a radioisotope, a chemotherapeutic drug or an immunomodulator, and the tracer can be selected from a radiological contrast agent, a paramagnetic ion, a metal, a fluorescent tag, a chemiluminescence tag, an ultrasonic contrast agent and a photosensitizer.

9. The antibody or antigen binding fragment according to any one of claims 1-8, wherein the antibody or antigen binding fragment binds to human CD19 with a KD value of less than 1 E-08M, 1E-09M, 1E-10M or 1E-11M; preferably, the antibody or antigen binding fragment also binds to monkey CD19, more preferably, the antibody or antigen binding fragment binds to monkey CD19 with a KD value of less than 1E-8M, 1E-9M, 1E-10M, 1E-11M or 1E-12M.

10. A multi-specific antigen binding molecule, wherein the multi-specific antigen binding molecule comprises at least a first antigen binding module and a second antigen binding module, the first antigen binding module comprises the antibody or antigen binding fragment according to any one of claims 1-10, and the second antigen binding module binds to other targets different from the first antigen binding module or binds to different epitopes of the same target; preferably, the second antigen binding module is an antibody or an antigen binding fragment;
and preferably, the other targets are selected from the group of: (1) a tumor specific antigen (TSA) or a tumor associated antigen (TAA); (2) an immune checkpoint; and (3) a target that recruits and/or activates immune cells.

11. A chimeric antigen receptor (CAR), wherein the chimeric antigen receptor comprises at least an extracellular antigen binding domain, a transmembrane domain and an intracellular signaling domain, and the extracellular antigen binding domain comprises the antibody or antigen binding fragment according to any one of claims 1-9 or the multi-specific antigen binding molecule according to claim 10.

12. An immune effector cell, wherein the immune effector cell expresses the chimeric antigen receptor according to claim 11 and/or a nucleic acid molecule encoding the chimeric antigen receptor according to claim 11;
preferably, the immune effector cell is selected from a T cell, a natural killer cell (a NK cell), a natural killer T cell (an NKT cell), a monocyte, a macrophage, a dendritic cell or a mast cell, more preferably the T cell is selected from a cytotoxic T cell, a regulatory T cell or a helper T cell;
and preferably, the immune effector cell is an autologous immune effector cell or an allogeneic immune effector cell.

13. An isolated nucleic acid molecule, wherein the nucleic acid molecule encodes the antibody or antigen binding fragment according to claims 1-9, the multi-specific antigen binding molecule according to claim 10 or the chimeric antigen receptor according to claim 11.

14. A vector, wherein the vector comprises the nucleic acid molecule according to claim 13.

15. A cell, wherein the cell comprises the vector according to claim 14.

16. A method for preparing the antibody or antigen binding fragment according to claims 1-9, or the multi-specific antigen binding molecule according to claim 10, wherein the method comprises: (1) culturing the cell according to claim 15, and/or (2) isolating the antibody or antigen binding fragment, or the multi-specific antigen binding molecule expressed by the cell.

17. A method for preparing the immune effector cell according to claim 12, wherein the method comprises introducing a nucleic acid molecule encoding the chimeric antigen receptor according to claim 11 into the immune effector cell, and/or initiating the immune effector cell to express the chimeric antigen receptor.

18. A pharmaceutical composition, wherein the pharmaceutical composition comprises the antibody or antigen binding molecule according to any one of claims 1-9, or the multi-specific antigen binding molecule according to claim 10, or the immune effector cell according to claim 12, or the nucleic acid molecule according to claim 13, or the vector according to claim 14, or the cell according to claim 15, or a product prepared according to the method according to claim 16 or 17; preferably, the composition further comprises a pharmaceutically acceptable carrier, diluent or auxiliary agent.

19. Use of the antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17 in the preparation of a drug for treating a CD19-related disease; preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

20. A method for treating a CD19-related disease, wherein the method comprises administering to a subject an effective amount of a drug, and the drug comprises the antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.

21. The antibody or antigen binding fragment according to any one of claims 1-9, the multi-specific antigen binding molecule according to claim 10, the immune effector cell according to claim 12, the nucleic acid molecule according to claim 13, the vector according to claim 14, the cell according to claim 15, the pharmaceutical composition according to claim 18, or a product prepared according to the method according to claim 16 or 17, for use in a drug for treating a CD19-related disease, preferably, the CD19-related disease is selected from a tumor or an autoimmune disease; and more preferably, the tumor is selected from lymphoma or leukemia, such as B cell lymphoma, acute lymphocytic leukemia, chronic lymphocytic leukemia, non-Hodgkin lymphoma, mantle cell lymphoma, follicular lymphoma, diffuse large B cell lymphoma or multiple myeloma, and the autoimmune disease is selected from an autoimmune disease of the nervous system, a rheumatoid disease, systemic lupus erythematosus, an IgG4 related disease, multiple sclerosis or a neuromyelitis optica spectrum disorder.
